# FASCICULE DE BREVET EUROPEEN

(11) **EP 1 171 405 B1**
(45) Date de publication et mention de la délivrance du brevet: **03.01.2007**
(21) Numéro de dépôt: 00920852.1
(22) Date de dépôt: 20.04.2000
(51) Int. Cl.: C07B 41/06, C07B 61/00, C07K 1/107, G01N 33/547, C12Q 1/68

(54) **SUPPORT SOLIDE FONCTIONNALISE POUR LA SYNTHESE D'ALPHA-OXOALDEHYDES**
FUNKTIONALISIERTE FESTTRÄGER ZUR HERSTELLUNG VON ALPHA-OXOALDEHYDEN
FUNCTIONALISED SOLID SUPPORT FOR ALPHA-OXOALDEHYDE SYNTHESIS

(30) Priorité: 21.04.1999 FR 9905024
(43) Date de publication de la demande: 16.01.2002
(73) Titulaire: INSTITUT PASTEUR DE LILLE, 59019 Lille Cédex (FR); CENTRE NATIONAL DE LA RECHERCHE SCIENTIFIQUE, 75016 Paris Cédex (FR)
(72) Inventeur: MELNYK, Oleg, F-59370 Mons-en-Baroeul (FR); FRUCHART, Jean-Sébastien, F-62800 Liévin (FR); BOUREL, Line, F-59000 Lille (FR); GRAS-MASSE, Hélène, F-59710 Merignies (FR)
(74) Mandataire: Lepeudry-Gautherat, Thérèse
(86) Numéro de dépôt international: PCT/FR2000/001035
(87) Numéro de publication internationale: WO 2000/064843

(56) Documents cités:
- WO-A-94/25071
- US-A- 5 362 852
- J. F. LYNAS: "Inhibitors of the chymotrypsin-like activity of proteasome based on di- and tri-peptidyl alpha-keto aldehydes (glyoxals)" BIOORGANIC & MEDICINAL CHEMISTRY LETTERS, vol. 8, no. 4, 1998, pages 373-378, XP004136915
- K. F. GEOGHEGAN: "Site-directed conjugation of nonpeptide groups to peptides and proteins via periodate oxidation of a 2-amino alcohol. Application to modification at N-terminal serine" BIOCONJUGATE CHEMISTRY., vol. 3, 1992, pages 138-146, XP002133027 AMERICAN CHEMICAL SOCIETY, WASHINGTON., US ISSN: 1043-1802 cité dans la demande
- L. ZHANG: "Preparation of functionally active cell-permeable peptides by single step ligation of two peptide modules" PROCEEDINGS OF THE NATIONAL ACADEMY OF SCIENCES OF USA, vol. 95, no. 16, 4 août 1998 (1998-08-04), pages 9184-9189, XP002133028 WASHINGTON US cité dans la demande
- J.-S. FRUCHART: "A new linker for the synthesis of C-terminal peptide alpha-oxo aldehydes" TETRAHEDRON LETTERS, vol. 40, 20 août 1999 (1999-08-20), pages 6225-6228, XP004174018 OXFORD GB

## Description

La présente invention est relative à des supports solides fonctionnalisés pour des synthèses organiques en phase solide, à leur procédé de préparation et à leurs applications notamment pour la synthèse de peptides α-oxoaldéhydes ou d'autres molécules organiques, telles que les dérivés non peptidiques de l'acide glyoxylique.

La présente invention est également relative à la création de banques combinatoires, de plaques de microtitration ou de biopuces, telles que des puces à ADN.

La synthèse automatisée de molécules organiques et notamment de macromolécules peptidiques en phase solide a été mise au point, au départ par Merrifield. Le schéma général de la réaction est le suivant : liaison d'un premier acide aminé N-protégé à un support solide insoluble fonctionnalisé, déprotection de la fonction amine terminale, extension de la chaîne (couplages successifs de différents acides aminés), éventuellement déprotection de l'acide aminé N-terminal puis, simultanément, clivage du polypeptide de la résine et déprotection des chaînes latérales des acides aminés.

Différents types de supports solides insolubilisés ont été proposés : polystyrènes, polyméthacrylates, polysaccharides, résines phénoliques, silice, verre poreux et polyacrylamides ; les supports les plus utilisés sont les polyacrylamides et les polystyrènes, plus particulièrement les poly(styrènes-co-divinylbenzènes).

Une synthèse sur support solide dépend de plusieurs facteurs pour être efficace et faire preuve d'un rendement convenable : le choix de la résine fonctionnalisée, le choix des groupes protecteurs des acides aminés, le choix des conditions de réaction et le choix des différents peptides qui pourront être combinés pour obtenir le produit final souhaité.

Toutefois, les résines utilisées actuellement ne permettent pas de générer directement le produit final, par exemple un produit portant une fonction α-oxoaldéhyde, à partir de la fonction d'ancrage au support solide. Dans le cas de la synthèse de peptides comportant une fonction α-oxoaldéhyde, les résines habituellement utilisées ne permettent pas d'obtenir le peptide α-oxoaldéhyde autrement que sous la forme d'un précurseur qui devra subir une transformation ultérieure en phase homogène pour générer la fonction attendue.

A l'heure actuelle, des structures peptidiques de grande taille peuvent être obtenues par ligation chimique, en milieu aqueux ou partiellement aqueux, de fragments peptidiques partiellement protégés ou totalement déprotégés, préalablement obtenus par synthèse en phase solide à l'aide de résines conventionnelles.

Cette méthode fait intervenir une liaison covalente de type amide, thioester, thioéther, thiazolidine, oxime ou encore hydrazone entre deux fragments peptidiques convenablement fonctionnalisés. En particulier, les liaisons thiazolidine, oxime et hydrazone nécessitent la préparation de peptides portant des fonctions aldéhydes.

Geoghegan, K.F. *et al.* (Bioconjugate Chem., 1992, 3, 138-146) ont décrit des peptides portant une fonction aldéhyde en position N-terminale, obtenus par oxydation périodique, en phase homogène, d'une sérine ou d'une thréonine en position N-terminale (présence d'un β-amino-alcool). La fonction α-oxoaldéhyde N-terminale obtenue est hautement réactive vis-à-vis de fonctions 1,2-aminothiol, hydroxylamine ou hydrazine portées par d'autres fragments peptidiques, permettant leur couplage.

Cette méthode nécessite la préparation du β-aminoalcool, sa purification, son oxydation et enfin la séparation du produit des réactifs d'oxydation.

L'utilisation de β-amino-alcools est également décrite dans le Brevet US 5 362 852 : une fonction α-oxoaldéhyde est générée, dans un peptide, par oxydation périodique en phase homogène d'un groupement 2-hydroxylamine présent dans la chaîne peptidique, ce qui est le cas soit dans des peptides possédant une sérine ou une thréonine en position N-terminale, soit dans des peptides dans lesquels un groupe hydroxylysine a été inséré.

De façon similaire, la Demande Internationale PCT WO 94/25071 décrit la formation d'une fonction α-oxoaldéhyde au niveau de l'extrémité N-terminale d'un peptide, par oxydation periodique en phase homogène d'un peptide portant une sérine ou une thréonine au niveau de ladite extrémité N-terminale. Ledit peptide est synthétisé de façon automatisée à l'aide d'une résine Sasrin^{®}, fournie par Bachem.

En ce qui concerne la création d'une fonction aldéhyde en position C-terminale, il n'existe que quelques méthodes ; certaines d'entre elles nécessitent la formation de fonctions α-aminoaldéhydes en position C-terminale. Les peptides résultant présentent l'inconvénient d'être instables, entre autres d'être sujets à des réactions d'épimérisation.

J.P. TAM *et al.* (Proc. Natl. Acad. Sci., Août 1998, 95, 9184-9189) ont proposé la synthèse de peptides portant un groupe glycoaldéhyde (-NH-CH₂-CHO) en position C-terminale. La synthèse décrite est peu pratique : elle nécessite la préparation d'un bras espaceur ainsi que la formation de la fonction aldéhyde en phase homogène. La phase solide n'est utilisée que pour la synthèse peptidique récurrente solide. Cette synthèse n'est pas automatisable puisque l'étape finale s'effectue en phase homogène.

L'introduction d'une fonction α-oxoaldéhyde en position C-terminale est décrite dans la Demande Internationale PCT WO 94/25071 sus-mentionnée, ladite fonction n'étant toutefois pas directement liée au squelette peptidique, comme cela ressort du procédé utilisé : une chaîne peptidique est synthétisée en phase solide, à l'aide d'une résine de type polystyrène, une séquence de cinq lysines étant insérée en position C-terminale ; des sérines sont greffées sur les fonctions amines libres α (extrémités N-terminale du peptide) ou ε (chaînes latérales des lysines) ; le peptide ainsi modifié est séparé du support solide ; une oxydation périodique en phase homogène permet la formation de fonctions α-oxoaldéhydes en position N-terminale et au niveau des chaînes latérales des lysines présentes au niveau de l'extrémité C-terminale.

J.P. TAM *et al.* (Proc. Natl. Acad. Sci, Août 1998, 95, 9184-9189) ont également décrit, de façon similaire, l'insertion d'un résidu lysine en position C-terminale d'un peptide obtenu en phase solide, le greffage d'un résidu sérine sur la fonction amine de la chaîne latérale de lysine, le clivage du peptide du support et l'oxydation périodique, en phase homogène, du β-aminoalcool présent au niveau de ladite sérine, pour obtenir une fonction α-oxoaldéhyde.

Dans toutes ces méthodes, la fonctionnalisation des peptides par une fonction aldéhyde s'effectue en phase homogène, après clivage du peptide du support solide.

Le besoin d'une méthode simple et automatisable de synthèse de produits organiques présentant une fonction α-oxoaldéhyde, tels que des peptides portant une fonction α-oxoaldéhyde, en position C-terminale par exemple, pour leur utilisation par exemple en tant qu'inhibiteurs d'enzymes ou de produits d'intérêt pharmaceutique ou pharmacologique, ou dans le cadre de la ligation chimique pour l'élaboration de structures plus complexes, ainsi que d'un support adapté à la mise en oeuvre de cette méthode se fait donc ressentir.

Les Inventeurs se sont donc donné pour but de pourvoir à un support solide adapté à la modification chimique de molécules organiques par des fonctions α-oxoaldéhydes, ainsi qu'à un procédé de synthèse de composés comportant au moins une fonction α-oxoaldéhyde, ledit procédé répondant aux impératifs suivants :
- il doit permettre la formation de la fonction α-oxoaldéhyde en une seule étape, au cours de la séparation du produit du support solide,
- l'étape de clivage du produit du support solide doit générer directement le produit final, c'est-à-dire, dans le cas d'une synthèse peptidique, le peptide α-oxoaldéhyde sous forme déprotégée,
- le procédé doit mettre en oeuvre des réactifs peu coûteux, être efficace (bon rendement), simple et automatisable.

La présente invention a ainsi pour objet un support solide fonctionnalisé pour la synthèse de composés comportant au moins une fonction α-oxoaldéhyde, lequel support est caractérisé en ce qu'il répond à la formule I suivante : 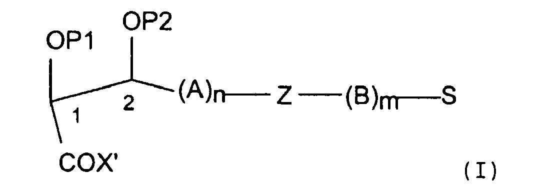 dans laquelle :
- S représente un support solide dont au moins la surface a la propriété d'être bien solvatée en milieu aqueux ou partiellement aqueux ;
- n et m, qui peuvent être identiques ou différents, représentent 0 ou 1 ;
- Z représente un groupement sélectionné dans le groupe constitué par les groupements éther, thioéther, ester, amine, amide, sulfonamide, hydroxylamine, hydrazine, hydrazone, thiazolidine, oxime, carbonate, carbamate, thiocarbamate, urée, thiourée et leurs dérivés lorsque n est égal à 1 ;
- Z représente un groupement sélectionné dans le groupe constitué par les groupements ester, amide, N-acylhydroxylamine, hydrazide, hydrazone, oxime, thiazolidine et leurs dérivés lorsque n est égal à 0 ;
- P1 et P2, qui peuvent être identiques ou différents ou former ensemble un cycle avec les atomes d'oxygène auxquels ils sont attachés, représentent des atomes d'hydrogène ou des groupements protecteurs des fonctions hydroxyles en positions 1,2, P1 et P2 étant dans ce cas sélectionnés dans le groupe constitué par les groupements -R₁,
- (CO)R₁, -(CO)OR₁, -(SO₂)OR₁, -SiR'R''R''', acétals et cétals, R₁, R', R" et R''' représentant des groupes alkyles comprenant de 1 à 18 atomes de carbone, linéaires, ramifiés ou cycliques, saturés ou insaturés, ou bien des groupes aryles ou hétéroaryles, lesdits groupes alkyles, aryles ou hétéroaryles étant éventuellement partiellement ou intégralement substitués par un ou plusieurs atomes d'halogènes, un ou plusieurs groupes amino, hydroxy, alcoxy, aryloxy, alkylthio ou arylthio ;
- A et B, qui peuvent être identiques ou différents, représentent une chaîne carbonée linéaire, ramifiée ou cyclique, saturée ou insaturée, comportant de 1 à 18 atomes de carbone et éventuellement de 1 à 7 groupements sélectionnés dans le groupe constitué par les groupements carbonyles, les cycles carbonés, les hétérocycles, les aryles et les hétéroaryles, A et/ou B comportant éventuellement en outre de 1 à 16 hétéroatomes, de préférence de 1 à 16 atomes d'azote ou d'oxygène, et pouvant être substitués par 1 à 16 groupements hydroxyles ou amino éventuellement convenablement protégés,
   au moins l'un des carbones de A et/ou de B étant éventuellement substitué par un groupement : 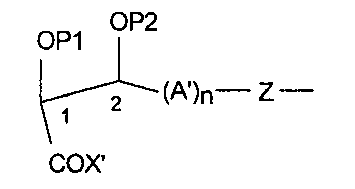 dans lequel A' est identique ou différent de A ; dans ce dernier cas, A' est sélectionné parmi les autres groupements A définis ci-dessus ; et
- X' est sélectionné dans le groupe constitué par les groupes -OR₁ et -NR'R'', R₁, R' et R'' représentant un atome d'hydrogène ou étant tels que définis ci-dessus.

A titre d'exemple et de façon non limitative :
- des groupes alkyles convenables sont des groupes méthyle, éthyle, propyle, isopropyle, tertiobutyle, vinyle, allyle ou benzyle ;
- des groupes aryles (cycles aromatiques) convenables sont des groupes phényle ou crésyle ;
- des hétéroaryles (hétérocycles aromatiques) convenables sont des groupes pyridine, pyrimidine ou pyrazine ;
- des cycles carbonés convenables sont des groupes cyclopentyle ou cyclohexyle ;
- un hétérocycle convenable est un groupe pipérazine.

Des atomes d'halogène pouvant substituer lesdits groupes alkyles, aryles ou hétéroaryles sont par exemple le fluor, le chlore ou le brome. Des groupes alcoxy convenables sont par exemple des groupes méthoxy, éthoxy, propoxy, isopropoxy, vinyloxy et alloxy ; des groupes aryloxy convenables sont par exemple des groupes phénoxy ou crésyloxy ; des groupes alkylthio convenables sont par exemple des groupes méthylthio, éthylthio, propylthio ou vinylthio ; des groupes arylthio convenables sont par exemple des groupes phénylthio ou crésylthio.

A titre d'exemple et de façon non limitative, les groupes protecteurs P1 et P2 peuvent être les suivants :
- lorsque P1 et P2 représentent un groupement -R₁, ce dernier peut être un méthyle, un tertiobutyle, un paraméthoxybenzyle, un méthoxyméthyle, un benzyloxyméthyle, un paraméthoxybenzyloxyméthyle, un paraméthoxyphénoxyméthyle, un tertiobutoxyméthyle, un 4-pentènyloxyméthyle, un silyloxyméthyle, un 2-méthoxyéthoxyméthyle, un 2-(triméthylsilyl)-éthoxyméthyle, un triphénylméthyle, un tétrahydropyranyle, un guaiacolméthyle ... ;
- lorsque P1 et P2 représentent un groupement
- (CO)R₁, ce dernier peut être un acétyle, un benzoyle, un paraméthylbenzoyle, un pivaloyle ... ;
- lorsque P1 et P2 représentent un groupement -(CO)OR₁, ce dernier peut être un groupement -COOCH₃, -COOC₂H₅, -COOCH₂C₆H₅, 9-fluorènyl-méthoxycarbonyle ... ;
- lorsque P1 et P2 représentent un groupement -(SO₂)OR₁, ce dernier peut être un groupement -(SO₂)OCH₃, -(SO₂)OC₂H₅ ... ;
- lorsque P1 et P2 représentent un groupement -SiR'R''R''', ce dernier peut être un groupement triméthylsilyle, triéthylsilyle, triisopropylsilyle, tertiobutyldiméthylsilyle, tertiobutyldiphénylsilyle ... ;
- lorsque P1 et P2 représentent un groupement acétal ou un cétal, ces derniers peuvent être un benzylidène, un isopropylidène ...

En variante, lorsque P1 et P2 forment ensemble un cycle avec les atomes d'oxygène auxquels ils sont attachés, ce cycle peut être un 1,3,2-dioxathiolane.

Il est bien entendu toutefois que tout groupe protecteur d'une fonction hydroxyle peut être utilisé en tant que groupe P1 ou P2, tel que décrit par exemple dans *" Protective Groups in Organic Synthesis",* Theodora W. Greene et Peter G.M. Wuts, 1991, John Wiley and Sons Inc.

De manière surprenante, de tels supports fonctionnalisés selon la présente invention sont particulièrement bien adaptés à la préparation de composés comportant au moins une fonction α-oxoaldéhyde, et ce sans synthèse préalable d'un aldéhyde.

En outre, ils présentent de nombreux avantages :
- spécificité de la coupure du composé synthétisé à partir du support, dans des conditions douces,
- compatibilité avec beaucoup de molécule organiques,
- possibilité de recyclage du support initial, comme cela sera détaillé ci-après.

Selon un mode de réalisation préféré dudit support S, il est sélectionné dans le groupe constitué par :
- les particules en verre borosilicaté poreux,
- les surfaces en verre ou en silicium,
- les résines sélectionnées dans le groupe constitué par les résines polyacrylamides, les résines polyacrylamide-polyéthylèneglycol, les résines polyéthylèneglycol-polystyrène, les résines réticulées éthoxylate-acrylate et les résines de type polyéthylène, polystyrène ou polypropylène greffé par des molécules hydrophiles.
   De telles résines sont disponibles commercialement sous les dénominations Spar^{®} (résine de type polyacrylamide), PEGA^{®} (résine de type polyacrylamide-polyéthylèneglycol), TentaGel^{®}, ArgoGel^{®}, NovaSyn^{®}, NovaGel^{®} (résines de type polyéthylèneglycol-polystyrène), Clear^{®} (résine réticulée éthoxylate-acrylate), Chiron^{®}, SynPhase-MD^{®} (couronne de polyéthylène greffée par un copolymère acide méthacrylique/diméthylacrylamide), SynPhase-HM^{®} (couronne de polyéthylène greffée par de l'hydroxyéthyl-méthacrylate), etc.
   Une surface en verre convenable est par exemple une lame de microscope.
   Un support de formule I préféré selon la présente invention est par exemple tel que S est une résine de type polyacrylamide-polyéthylèneglycol, m et n représentent 1, B est une chaîne -(CH₂)₂-CO-NH-, Z est un groupement amide, A est une chaîne -CO-NH-(CH₂)₃-, P1 et P2 forment ensemble un groupement protecteur isopropylidène et X' représente une chaîne -NH- (CH₂)₃-NH₂.
   Un autre support de formule I préféré selon la présente invention est par exemple tel que S est une résine de type polyacrylamide-polyéthylèneglycol ou polyéthylèneglycol-polystyrène, m et n représentent 0, Z est un groupement amide, P1 et P2 forment ensemble un groupe protecteur isopropylidène et X' représente un groupe méthoxy.
   Un autre support de formule I préféré selon la présente invention répond par exemple à la formule I, dans laquelle S est tel que défini précédemment, m représente 1, n représente 0, Z est un groupement amide, P1 et P2 forment ensemble un groupe protecteur isopropylidène, X' représente un groupe méthoxy et B représente le groupe suivant : 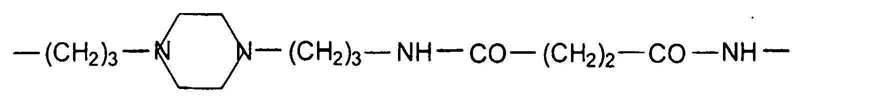
   Un autre support de formule I préféré selon la présente invention répond par exemple à la formule I, dans laquelle S est tel que défini précédemment, m et n représentent 1, Z est un groupe amide, P1 et P2 forment ensemble un groupe protecteur isopropylidène, B est un groupe -(CH₂)₂-CO-NH-, A représente le groupe suivant : 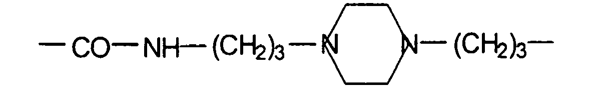 et x' représente le groupe suivant : 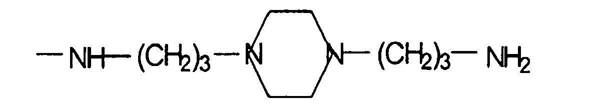
   Un autre support de formule I préféré selon la présente invention répond par exemple à la formule I, dans laquelle S est tel que défini précédemment, m représente 0, n représente 1, Z est un groupe carbamate, P1 et P2 forment ensemble un groupe protecteur isopropylidène, X' représente un groupe méthoxy et A représente le groupe suivant : 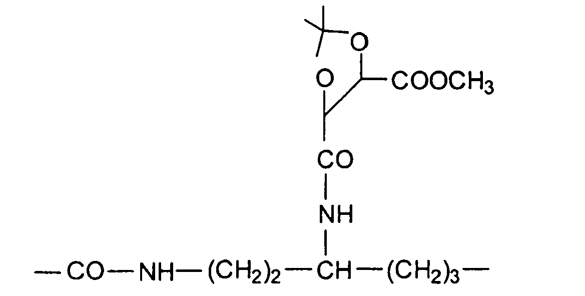
   Un autre support de formule I préféré selon la présente invention répond par exemple à la formule suivante : 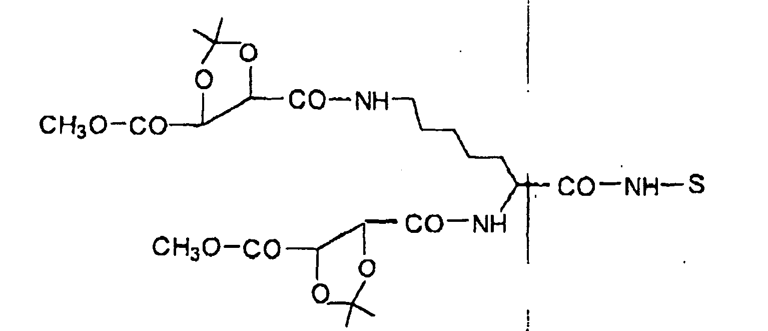
dans laquelle S est tel que défini ci-dessus, n = 0, m = 1, Z est un groupement amide X' représente un groupe méthoxy, P1 et P2 forment ensemble un groupe isopropylidène et B représente un groupe :

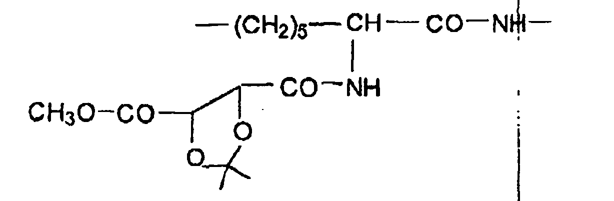

Un autre support de formule 1 préféré selon la présente invention répond par exemple à la formule suivante : 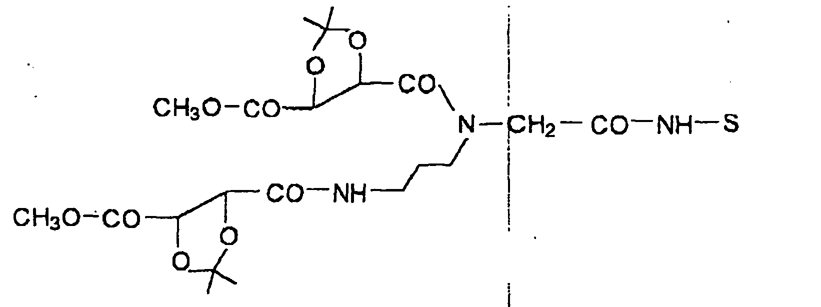 dans laquelle S est tel que défini ci-dessus, n = 0, m = 1, Z est un groupement amide, X' représente un groupe méthoxy, P1 et P2 forment ensemble un groupe iso-propylidène et B représente un groupe :

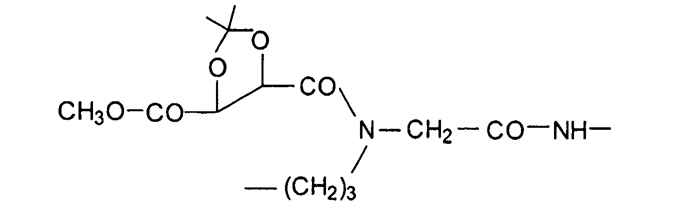

Les supports de formule I selon l'invention peuvent avantageusement être utilisés en tant que supports solides pour la synthèse de peptides α-oxoaldéhydes ou d'autres molécules organiques fonctionnalisées par au moins un α-oxoaldéhyde, telles que les dérivés de l'acide glyoxylique.

Les supports de formule I selon la présente invention peuvent également être utilisés pour la synthèse d'oligonucléotides, notamment d'oligodésoxynucléotides, ou de lipides fonctionnalisés par au moins un α-oxoaldéhyde, par des techniques bien connues, décrites par exemple dans *" Oligonucleotide synthesis : a practical approach",* 1984, M. J. GAIT Ed., IRL Press, Washington D.C. Les supports de formule I selon la présente invention peuvent également être utilisés pour la synthèse de peptides ou de protéines en phase solide de façon automatisée, comme décrit par exemple par R.C. Sheppard dans Comp. Org. Chem., 1979, 5, 352-355 et par R.B. Merrifield dans Science, 18 avril 1986, 232, 341-347. Au cours d'une telle synthèse, les chaînes latérales des acides aminés sont convenablement protégées, comme cela est connu en soi.

La présente invention a également pour objet un procédé de préparation du support de formule I décrit ci-dessus, ledit procédé étant caractérisé en ce qu'il consiste à faire réagir :
- un support solide fonctionnalisé de formule S-(B)ₘ-Y, dans laquelle B, S et m sont tels que décrits ci-dessus et Y représente un groupe nucléophile, B pouvant être substitué par au moins un autre groupement Y, identique ou différent de celui représenté dans la formule S-(B)ₘ-Y, en plus de celui représenté dans la formule S-(B)ₘ-Y, auquel cas ledit support solide est polyfonctionnalisé,
- avec un composé sélectionné dans le groupe constitué par les anhydrides cycliques de l'acide tartrique ou d'un de ses dérivés et les composés de formule II : 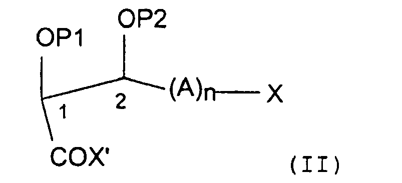
dans laquelle n, A, P1, P2 et X' sont tels que définis ci-dessus et X représente un groupe électrophile.

Au sens de la présente invention, on entend par " nucléophile" un groupe possédant une paire d'électrons libres, c'est-à-dire un groupe donneur d'électrons. On entend par " électrophile " un groupe possédant une orbitale moléculaire vacante de faible énergie capable d'interagir avec la paire d'électrons libres du groupe nucléophile, c'est-à-dire un groupe accepteur d'électrons.

En variante, dans le cas où n = 1, Y peut représenter un groupe électrophile et X peut représenter un groupe nucléophile.

Selon un mode de mise en oeuvre avantageux dudit procédé selon la présente invention, un groupe électrophile convenable (c'est-à-dire le groupe X dans le cas ou n = 0, et le groupe X ou le groupe Y dans le cas où n = 1) est de préférence sélectionné dans le groupe constitué par les groupements suivants :
- lorsque n = 1 : -CR₂R₃Cl, -CR₂R₃Br, -CR₂R₃I, -CR₂R₃OSO₂R₄, -CHO, -COOR₂, -COF, -COCl, -COBr, -SO₂OR₂, -SOOCl, ester succinimidylique, ester sulfosuccinimidylique, -COO⁻ transformé par un agent d'activation électrophile, -N=C=O (isocyanate), -N=C=S (isothiocyanate), -O-CO-OR₂, -O-CO-Im, -O-COCl, -NR₂-COCl et -NR₂-CO-Im,
   où Im représente un groupe imidazolyle éventuellement substitué de formule : 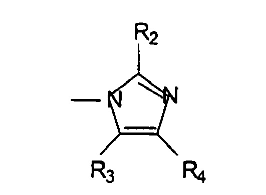 et où R₂, R₃ et R₄, qui peuvent être identiques ou différents, représentent des atomes d'hydrogène ou des groupes alkyles comprenant de 1 à 18 atomes de carbone, linéaires, ramifiés ou cycliques, saturés ou insaturés, ou bien des groupes aryles ou hétéroaryles, lesdits groupes alkyles, aryles et hétéroaryles étant éventuellement partiellement ou intégralement substitués par un ou plusieurs atomes d'halogènes, un ou plusieurs groupes amino, hydroxy, alcoxy, aryloxy, alkylthio ou arylthio ;
- lorsque n = 0 : -CHO, -COOR₂, -COF, -COCl, -COBr, -COO⁻ transformé par un agent d'activation électrophile, R₂ étant tel que décrit ci-dessus pour n = 1.

Un agent d'activation électrophile est un agent qui, réagissant avec le carboxylate -COO⁻, lui confère un caractère électrophile. Un tel agent est bien connu de l'Homme de l'Art et est par exemple décrit dans " *Comprehensive organic synthesis",* B. M. Trost et I. Fleming Ed., Pergamon Press, 1991, volumes 1-9.

Selon un autre mode de mise en oeuvre avantageux dudit procédé, un groupe nucléophile convenable (c'est-à-dire le groupe Y dans le cas où n = 0, et le groupe X ou le groupe Y dans le cas où n = 1) est de préférence sélectionné dans le groupe constitué par les groupements suivants lorsque n = 0 ou lorsque n = 1 :
-OH, -SH, -COO⁻, -NHR₅, -CONR₅NH₂, -N[R₅(CO)]NH₂, -N[R₅O (CO)]NH₂, -N[R₅NH (CO)]NH₂, -SO₂NH₂, -SO₂NR₅NH₂, -ONH₂, -(CO)ONH₂ et -C(NHR₅)-CR₅R₆-SH, R₅ et R₆ ayant la même signification que les groupes R₂ à R₄ définis ci-dessus pour les groupements électrophiles.

Selon un mode de mise en oeuvre avantageux du procédé de préparation du support de formule I, dans le cas où n = 0 et où X = -COOR₂, avec R₂ = H, ce groupement peut jouer aussi bien le rôle de nucléophile que d'électrophile. Dans le cas où n = 0 et où X = -COOH, X peut donc représenter un groupe nucléophile et Y un groupe électrophile. Par exemple, si X = -COOH et Y = -NHR₅, X joue le rôle d'électrophile. En revanche, si X = -COOH et Y = -CR₂R₃Cl, -CR₂R₃Br, -CR₂R₃I ou -CR₂R₃OSO₂R₄, alors X joue le rôle de nucléophile.

Ainsi, la réaction du support solide S-(B)ₘ-Y avec un anhydride cyclique de l'acide tartrique, ou d'un de ses dérivés, ou avec le composé de formule II permet d'obtenir le support de formule I selon l'invention. Selon les groupements X et Y choisis, différents groupements Z peuvent être formés. A titre d'exemple et de façon non limitative :
- un groupement Z = éther (respectivement, thioéther ou amine) peut être formé par réaction de X = -OH (respectivement, -SH ou -NHR₅) avec Y = -CR₂R₃Cl, -CR₂R₃Br, -CR₂R₃I ou -CR₂R₃OSO₂R₄ (par exemple un tosylate ou un mésylate) ;
- un groupement Z = ester (respectivement, amide) peut être formé par réaction de X = -OH (respectivement, -NHR₅) avec Y = -COOR₂, -COF, -COCl ou -COBr ;
- un groupement Z = sulfonamide peut être formé par réaction de X = -NHR₅ avec Y = -SO₂OR₂ ou -SOOCl ;
- un groupement Z = hydroxylamine ou dérivé d'hydroxylamine, tel qu'une liaison N-acylhydroxylamine ou N,O-acylhydroxylamine, peut être formé à partir de X = -ONH₂ ou -(CO)ONH₂ avec Y = -COOR₂, -COF, -COC1, -COBr, -SO₂OR₂ ou -SOOCl ;
- un groupement Z = hydrazine ou dérivé d'hydrazine peut être formé par réaction de X = -CONR₅NH₂, -N[R₅(CO)]NH₂, -N[R₅O(CO)]NH₂, -N[R₅NH(CO)]NH₂ ou -SO₂NR₅NH₂ avec Y = -COOR₂, -COF, -COCl, -COBr, -SO₂OR₂ ou -SOOC1 ;
- un groupement Z = hydrazone ou ses dérivés (par exemple sulfonylhydrazone) peut être formé par réaction de X = -CONR₅NH₂ avec Y = -CHO ;
- un groupement Z = thiazolidine peut être formé entre X = -C(NHR₅)-CR₅R₆-SH et Y = -CHO ;
- un groupement Z = oxime peut être formé par réaction entre X = -ONH₂ et Y = -CHO ;
- un groupement Z = carbonate (-O-CO-O-) peut être formé par réaction entre X = -O-CO-OR₂, -O-CO-Im ou -O-COCl et Y = -OH ;
- un groupement Z = carbamate (-NR₂-CO-O-) peut être formé entre X = -NR₂-COCl, -NR₂-CO-Im ou isocyanate et Y = -OH, ou encore entre X = -O-CO-OR₂, -O-CO-Im ou -O-COCl et Y = -NHR₅ ;
- un groupement Z = thiocarbamate (-NR₂-CS-O-) peut être formé entre X = isothiocyanate et Y = -OH ;
- un groupement Z = urée peut être formé entre X = -NR₂-CO-Im, -NR₂-CO-Im ou isocyanate et Y = -NHR₅ ;
- un groupement Z = thiourée peut être formé entre X = isocyanate et Y = -NHR₅.

Selon un mode de mise en oeuvre avantageux du procédé de préparation du support de formule I selon l'invention, ledit anhydride cyclique de l'acide tartrique ou de ses dérivés est l'anhydride di-O-benzoyl-tartrique ou l'anhydride diacétyl-tartrique.

Selon un autre mode de mise en oeuvre avantageux du procédé de préparation du support de formule I selon l'invention, lorsque P1 et P2 représentent un atome d'hydrogène lors de la réaction du support solide de formule S-(B)ₘ-Y avec le composé de formule II, des groupes P1 et P2 autres que des atomes d'hydrogène, à savoir des groupements protecteurs des fonctions hydroxyles en positions 1,2, peuvent éventuellement être introduits après la réaction du support solide de formule S-(B)ₘ-Y avec le composé de formule II et avant que le support de formulé I selon l'invention ne soit utilisé pour la synthèse de composés comportant au moins une fonction α-oxoaldéhyde.

Selon encore un autre mode de mise en oeuvre avantageux du procédé selon l'invention, le composé de formule II représente l'acide tartrique ou un dérivé de l'acide tartrique. Auquel cas, la réaction entre le support solide S-(B)ₘ-Y et le composé de formule II correspond, quand le groupe A du composé de formule II est tel qu'il comporte une fonction carbonyle en alpha du groupe -OP2, au greffage de l'acide tartrique ou d'un dérivé de l'acide tartrique sur le support solide S-(B)ₘ-Y.

Un tel dérivé de l'acide tartrique est par exemple, et de façon non limitative :
- un sel de l'acide tartrique (sel de sodium, de lithium),
- l'acide dibenzoyl-tartrique,
- l'acide di-paratoluyl-tartrique,
- l'acide di-isopropyl-tartrique,
- l'acide di-pivaloyl-tartrique,
- l'acide di-acétyl-tartrique,
- l'acide métatartrique,
- l'acide cichorinic,
- l'acide tartralinique,
- l'acide 4-fluoro-tartranilique,
- l'acide 4-chloro-tartranilique,
- l'acide 4-méthyl-tartranilique,
- l'acide 4-nitro-tartranilique,
- l'acide mono-n-octylamide tartrique,
- l'acide N-(2,4-dichlorophényl)-2,3-dihydroxy-succinamique,
- l'acide 2,3-diacétoxy-N-[1-(1-naphtyl)éthyl]succinamique,
- l'acétate de 4-acétoxy-2,5-dioxo-tétrahydrofurane-3-yle,
- le di-paratoluate de 3,4-dihydroxy-2,5-dioxotétrahydrofurane,
- le tartrate de diméthyle,
- le tartrate de diéthyle,
- le tartrate de dibutyle,
- le tartrate de ditertiobutyle,
- le tartrate de diisopropyle,
- le tartrate de disuccinimidyle,
- le tartrate de bis(sulfosuccinimidyle),
- le tartrate de diisoamyle,
- le tartrate de dibenzyle,
- le tartrate de 3-pyridylméthyle,
- le tartrate de diméthyl-2,3-O-isopropylidene,
- le tartrate de diméthyl-2,3-O-benzylidène,
- le tartrate de diméthyl-2,3-O-(1-phényléthylidène),
- le tartrate de diéthyl-2,3-O-isopropylidène,
- le tartrate de diéthyl-2,3-O-benzylidèrie,
- le tartrate de diisopropyl-O,O-bis(trimétylsilyl),
- le bitartrate de phénylpropanolamine,
- la bitartrate d'alcool nicotinylique,
- le 4,5-dicarboxylate de diméthyl-1,3,2-dioxathiolane,
- le 4,5-dicarboxylate de diéthyl-2-phényl-1,3-dioxolane,
- le 4,5-dicarboxylate de diméthyl-2-phényl-1,3-dioxolane,
- le 2,3-dihydroxysuccinate de dibenzyle,
- le 2,3-dihydroxysuccinate de di[2-oxo-2-(4-(trifluorométhyl)phényl)éthyl].

Lors de la réaction entre le support solide S-(B)ₘ-Y avec 1'anhydride cyclique de l'acide tartrique (ou d'un de ses dérivés) ou le composé de formule II, et lorsque le composé de formule II est un ester de l'acide tartrique (ou d'un de ses dérivés), on peut éventuellement soumettre à une réaction d'hydrolyse ou de saponification ledit anhydride cyclique ou ledit ester avant sa réaction avec ledit support solide, et ce afin de générer une fonction acide libre réactive avec le support solide fonctionnalisé.

Selon un autre mode de mise en oeuvre avantageux du procédé selon la présente invention, le composé de formule II est sélectionné dans le groupe constitué par l'acide mucique, l'acide saccharique, l'acide glucarique (auxquels cas, X' = -OH, n = 1, A = -CH(OH)-CH(OH)-, X = -COOH, P1 = P2 = H dans la formule II, les trois acides se disloquant par leurs stéréochimies respectives), leurs sels de potassium, de calcium) et leurs dérivés.

Selon encore un autre mode de mise en oeuvre avantageux du procédé selon la présente invention, ledit procédé consiste à faire réagir :
- un support solide fonctionnalisé de formule :

   H₂N-(CH₂)₁-CH(NH₂)-CO-NH-S,

   dans laquelle S est tel que défini précédemment,
- avec un tartrate de diméthyl-2,3-O-isopropylidène.

Selon un autre mode de mise en oeuvre avantageux du procédé selon la présente invention, ledit procédé consiste à faire réagir :
- un support solide fonctionnalisé de formule :

   H₂N- (CH₂) ₃-NH-CH₂-CO-NH-S,

   dans laquelle S est tel que défini précédemment,
- avec un tartrate de diméthyl-2,3-O-isopropylidène.

La présente invention a également pour objet un support de formule III suivante : 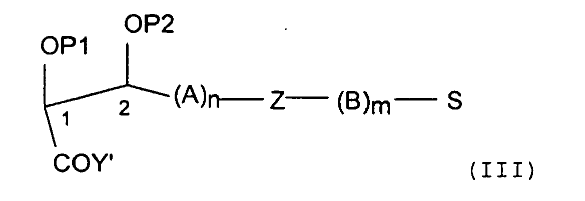 dans laquelle S, B, A, n, m, Z, P1 et P2 sont tels que définis ci-dessus et Y' représente un composé ou un enchaînement de plusieurs composés sélectionnés dans le groupe constitué par les lipides, les nucléotides, lesdits nucléotides étant substitués ou non, et les acides aminés, ces derniers comportant éventuellement des lipides. De manière générale, Y' représente donc un lipide, un oligonucléotide, un peptide, un pseudopeptide, etc. Y' peut comporter un ou plusieurs types des composés sus-mentionnés, Y' étant alors un polymère homogène ou hétérogène.

Le support de formule III peut être obtenu à partir du support de formule I après modification chimique ou dérivatisation du groupement -COX' à l'aide d'un réactif approprié, qui apportera une fonction permettant le greffage sur le support d'acides aminés, de lipides, de nucléotides, etc. On peut par exemple envisager le greffage d'une diamine, d'un dialcool ou d'un aminoalcool sur la fonction -COX', la fonction amine ou alcool libre ainsi introduite sur le support permettant la synthèse de peptides modifiés.

Le support de formule III décrit ci-dessus peut être obtenu à partir du support de formule I selon l'invention comme cela est décrit par exemple dans " *Solid phase organic reactions* ", Tetrahedron report number 394, Tetrahedron, 1996, 52, 4527-4554 et dans " *Solid phase organic reactions II ",* Tetrahedron report number 418, Tetrahedron, 1997, 53, 5643-5678.

Le support de formule III décrit ci-dessus peut ainsi être utilisé pour stocker le produit synthétisé, ledit produit étant ultérieurement séparé du support, au moment de son utilisation, avec formation simultanée de la fonction α-oxoaldéhyde.

La présente invention a également pour objet un procédé de synthèse de composés organiques comportant au moins une fonction α-oxoaldéhyde, caractérisé en ce que ledit procédé s'effectue en phase solide et comprend les étapes suivantes :
- préparation d'un support de formule I tel que décrit précédemment, ladite préparation s'effectuant comme cela a été décrit ci-dessus ;
- éventuellement, dérivatisation du groupe X' dudit support de formule I, de façon à introduire une fonction permettant l'élaboration sur le support du composé organique Y' tel que défini précédemment ;
- élaboration du composé Y' en phase solide ;
- si P1 et P2 sont différents d'un atome d'hydrogène, déprotection des deux fonctions hydroxyles en positions 1,2 protégées par P1 et P2 selon la formule III telle que décrite ci-dessus ;
- oxydation périodique en phase solide ; et
- isolement du produit formé, fonctionnalisé par au moins une fonction α-oxoaldéhyde.

De manière avantageuse, l'étape éventuelle de dérivatisation du groupe X' du support de formule I consiste à greffer une diamine, un dialcool ou un aminoalcool.

Quant à l'étape de déprotection éventuelle des deux fonctions hydroxyles en positions 1,2 protégées par P1 et P2, elle s'effectue dans des conditions bien connues de l'Homme de l'Art et décrites par exemple dans " *Protective groups in organic synthesis",* Theodora W. Greene et Peter G.M. Wuts, 1991, John Wiley and Sons Inc, ou dans " *Comprehensive organic synthesis",* B. M. Trost et I. Fleming Ed., Pergamon Press, 1991, volumes 1-9.

Selon un mode de mise en oeuvre avantageux, la déprotection du diol-1,2 s'effectue en milieu acide, de préférence en présence d'acide trifluoroacétique. Une déprotection en milieu acide convient, par exemple et de façon non limitative, dans le cas où P1 et P2 représentent des groupes acétals, cétals, -SiR' R'' R''', tertiobutyle, triphénylméthyle, tétrahydropyranyle ou méthoxyméthyle.

Lorsque le produit synthétisé à l'aide du support de formule I selon l'invention est un peptide ou une protéine, cette étape de déprotection permet également, de façon simultanée, la déprotection des chaînes latérales des acides aminés dudit peptide ou de ladite protéine.

Selon un autre mode de mise en oeuvre avantageux du procédé selon l'invention, la déprotection du diol-1,2 (fonctions hydroxyles en positions 1,2) s'effectue en milieu basique. Une déprotection en milieu basique convient, par exemple, lorsque P1 et P2 représentent des groupes esters -(CO)OR₁.

L'étape de déprotection donne ainsi le produit de formule IV Suivante :

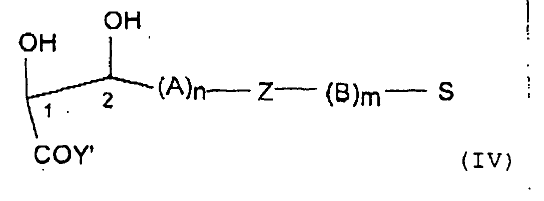

De manière préférée, l'oxydation périodique est réalisée à l'aide de periodate de sodium dans un solvant eau/acide acétique, tel que décrit par exemple par L. Zhang *et al.* dans Proc. Natl. Acad. Sci., 1998, 95, 9184-9189. Lorsque le produit synthétisé à l'aide du support de formule I selon l'invention est un peptide ou une protéine, un tel solvant permet la solubilisation desdits peptides. Un tel solvant eau/acide acétique explique également l'importance d'une bonne propriété de solvatation du support solide S choisi dans la formule I du support selon l'invention.

Toutefois, d'autres mélanges de solvants peuvent être utilisés pour réaliser l'étape d'oxydation périodique. Par exemple, dans le cas où le support de formule I selon l'invention est utilisé pour la synthèse de peptides contenant des méthionines, l'oxydation périodique peut être réalisée dans un mélange tampon citrate/méthanol/diméthylsulfure ; dans le cas où le support de formule I selon l'invention est utilisé pour la synthèse de peptides dérivatisés par une chaîne d'acide gras, l'oxydation périodique peut être réalisée dans un mélange tertiobutanol/acide acétique/eau.

L'oxydation périodique du produit de formule IV a pour conséquence la formation du produit Y' fonctionnalisé par une fonction α-oxoaldéhyde, ainsi que la formation du support solide de formule V suivante :

OHC-(A)ₙ-Z-(B)ₘ-S (V)

dans laquelle S, B, A, Z, n et m sont tels que définis précédemment.

De façon particulièrement avantageuse, le procédé selon l'invention, de par l'étape d'oxydation en phase solide décrite ci-dessus, permet simultanément la libération du produit du support et la formation de la fonction α-oxoaldéhyde au point d'ancrage du produit sur le support. Quand le produit synthétisé sur le support est un peptide, l'étape d'oxydation périodique en phase solide, qui s'effectue sur un peptide déprotégé, permet simultanément la libération du peptide du support, sans qu'il soit nécessaire d'effectuer une étape supplémentaire de déprotection en phase homogène, et la formation de la fonction α-oxoaldéhyde au point d'ancrage du peptide au support : il peut s'agir d'une extrémité dudit peptide ou d'une chaîne latérale de ce peptide.

Le produit fonctionnalisé par un α-oxoaldéhyde ainsi formé est ensuite isolé, par exemple par une étape de filtration suivie d'une purification par chromatographie liquide haute performance, au moyen de techniques connues en elles-mêmes.

Quant au support solide de formule V représentée ci-dessus, il peut avantageusement être recyclé ou régénéré en vue de sa réutilisation, c'est-à-dire réagir avec un réactif approprié de façon à produire un support de formule I selon l'invention tel que décrit précédemment, comme cela sera détaillé ci-après.

La présente invention a également pour objet une méthode de synthèse d'une banque de composés organiques, caractérisée en ce qu'elle comprend :
- la fourniture d'une pluralité de surfaces de support solide de formule S-(B)ₘ-Y, dans laquelle B, S, Y et m sont tels que décrits ci-dessus ;
- leur réaction avec un composé sélectionné dans le groupe constitué par les anhydrides cycliques de l'acide tartrique ou d'un de ses dérivés et les composés de formule II 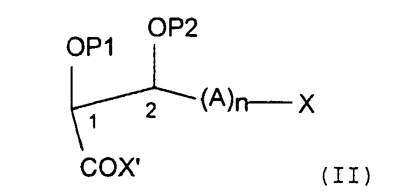
dans laquelle n, A, P1, P2, X' et X sont tels que définis ci-dessus, de façon à obtenir un support utilisable pour la synthèse de composés comportant une fonction α-oxoaldéhyde ;
- la réaction du support obtenu avec des mélanges d'acides aminés de nucléotides ou de lipides comme cela a été décrit précédemment dans le cadre de l'obtention d'un support de formule III à partir d'un support de formule I ; et
- l'obtention ainsi d'une banque de composés organiques.

La réaction desdites molécules organiques avec le support décrit ci-dessus s'effectue par exemple comme cela est décrit dans " *The combinatorial index* ", 1998, Academic Press, London.

La présente invention a également pour objet une banque de composés organiques, caractérisée en ce qu'elle est susceptible d'être obtenue selon le procédé décrit ci-dessus.

Les composés organiques de cette banque de composés pourront être séparés du support insoluble pour être utilisés ultérieurement, comme cela a été décrit ci-dessus dans le cadre du procédé de synthèse de composés organiques comportant une fonction α-oxoaldéhyde.

La présente invention a également pour objet l'utilisation d'un composé sélectionné dans le groupe constitué par les anhydrides cycliques de l'acide tartrique ou d'un de ses dérivés et les composés de formule II 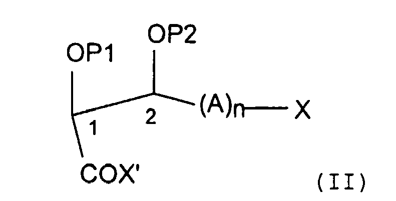 dans laquelle n, A, P1, P2 et X' et X sont tels que définis ci-dessus, pour la dérivatisation d'un support solide dont au moins la surface a la propriété d'être bien solvatée en milieu aqueux ou partiellement aqueux, le support résultant pouvant être utilisé pour la synthèse, en phase solide, de composés comportant au moins une fonction α-oxoaldéhyde.

De préférence, ledit support solide dont au moins la surface a la propriété d'être bien solvatée en milieu aqueux ou partiellement aqueux répond à la formule S-(B)ₘ-Y, S, B, m et Y étant tels que définis précédemment.

L'anhydride cyclique de l'acide tartrique ou d'un de ses dérivés est avantageusement l'anhydride di-O-benzoyl-tartrique ou l'anhydride diacétyl-tartrique.

Le composé de formule II est avantageusement tel que le groupe A comprend un groupe carbonyle en alpha du groupe -OP2 et représente l'acide tartrique ou un dérivé de l'acide tartrique.

En variante, le composé de formule II est sélectionné dans le groupe comprenant l'acide mucique, l'acide saccharique, l'acide glucarique, leurs sels et leurs dérivés.

La présente invention a, en outre, pour objet un procédé de préparation d'un réactif de diagnostic sur support solide, caractérisé en ce qu'il comporte les étapes suivantes :
- ligation de composés organiques comportant au moins une fonction α-oxoaldéhyde, obtenus par le procédé précédemment décrit, à une plaque de microtitration convenablement fonctionnalisée,
- obtention d'une plaque de microtitration sur laquelle sont fixés, de façon covalente, lesdits composés organiques.

Le réactif obtenu peut être utilisé pour la mise au point de réactifs immunologiques ou de tests pharmacologiques.

Lesdits composés organiques sont de préférence des peptides, des oligonucléotides ou des lipides.

Ladite plaque de microtitration est avantageusement fonctionnalisée de façon à permettre la ligation desdits composés organiques par liaison oxime, oxazolidine ou hydrazone.

La présente invention a également pour objet un procédé d'obtention d'une biopuce, caractérisé en ce qu'il comprend une étape de greffage d'oligonucléotides comportant au moins une fonction α-oxoaldéhyde, obtenus par le procédé précédemment décrit, à un support solide.

Dans les biopuces ainsi obtenues, les oligonucléotides sont fixés de façon covalente au support solide.

Ces biopuces permettent notamment le suivi de l'expression de gènes selon le concept présenté dans Science, 1995, 270, 467-470.

Selon un mode de mise en oeuvre avantageux du procédé selon l'invention, ledit support solide est une surface en verre ou en silicium.

Selon un autre mode de mise en oeuvre avantageux du procédé selon l'invention, ladite biopuce est une puce à ADN.

Outre les dispositions qui précèdent, l'invention comprend encore d'autres dispositions qui ressortiront de la description qui va suivre, qui se réfère à des exemples de supports selon l'invention et à des exemples de mise en oeuvre du procédé de synthèse d'α-oxoaldéhydes selon la présente invention, ainsi qu'aux figures annexées, dans lesquelles :
- les figures 1 et 2 représentent les étapes de la synthèse de supports de formule I selon l'invention ;
- la figure 3 représente les peptides 3-6 et 9 synthétisés à l'aide des supports selon l'invention, ainsi que les peptides 10, 12 et 14 synthétisés par des techniques classiques en phase solide ;
- la figure 4 représente le peptide 18, à savoir un peptide modifié par des acides gras et comportant une fonction α-oxoaldéhyde, ledit peptide étant synthétisé à l'aide d'un support selon l'invention ;
- la figure 5 représente le peptide 19, à savoir un peptide de type poly-lysine fonctionnalisé par 8 fonctions chloroacétyles et par une fonction α-oxoaldéhyde, ledit peptide étant synthétisé à l'aide d'un support selon l'invention ;
- la figure 6 représente le peptide 20, à savoir un peptide de type poly-lysine fonctionnalisé par 16 fonctions chloroacétyles et par une fonction α-oxoaldéhyde, ledit peptide étant synthétisé à l'aide d'un support selon l'invention ;
- les figures 7 et 8 représentent des réactions de ligation chimique de type hydrazone à l'aide de peptides selon l'invention ;
- la figure 9 représente une réaction de ligation chimique de type thiazolidine à l'aide d'un peptide selon l'invention ;
- la figure 10 représente une réaction de ligation chimique de type oxime à l'aide d'un peptide selon l'invention ; et
- la figure 11 représente le peptide 21, porteur d'une fonction α-oxoaldéhyde terminale et synthétisé à l'aide d'un support selon l'invention.

Il doit être bien entendu toutefois que ces exemples sont donnés uniquement à titre d'illustration de l'objet de l'invention, dont ils ne constituent en aucune manière une limitation.

### EXEMPLE 1 : Synthèse d'un support de formule I selon l'invention à partir d'une résine amino-PEGA^{®} et du (+)-diméthyl-2,3-O-isopropylidène-D-tartrate.

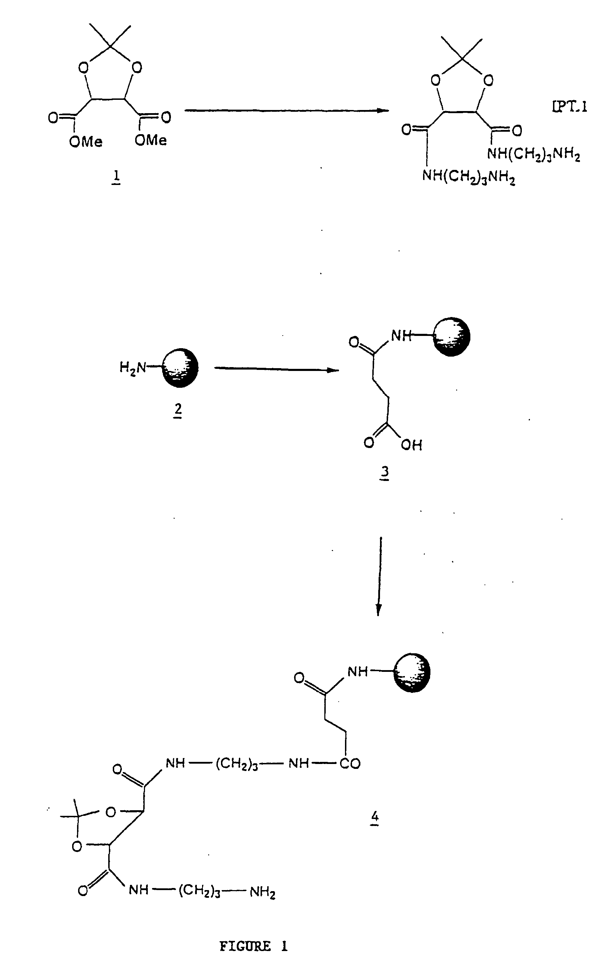

Les différentes étapes de cette synthèse sont résumées sur la figure 1.

2 ml (9,16 mmol) de (+)-diméthyl-2,3-O-isopropylidène-D-tartrate (fourni par ACROS) 1, ci-après dénommé IPT, sont ajoutés, pendant 45 minutes, à 10 ml (120 mmol) de 1,3-diaminopropane. Après 5 h 30 d'agitation à température ambiante, le 1,3-diaminopropane en excès est éliminé par évaporation sous pression réduite afin d'isoler le composé TPT-1, dont l'analyse par résonance magnétique nucléaire et par infra-rouge est la suivante

RMN ¹H 300 MHz (DMSO-d₆, TMS) : 8.19 (t, 2H, J = 5.5 Hz, CONH), 4.46 (s, 2H, COCH), 3.15 (m, 4H, CH₂NHCO), 2.50 (m, 4H, CH₂NH₂), 1.48 (q, 4H, J = 6.6 Hz, NHCH₂CH₂), 1.39 (s, 6H, (CH₃)₂C). RMN ¹³C 75 MHz (OMSO-d₆, TMS) : 171.5, 111.5, 77.6, 39.0, 36.4, 32.6, 26.2. IR (cm⁻¹) : 3356.8, 2940.3, 1660.7, 1537.6, 1084.6.

L' analyse élémentaire du composé IPT-1 est la suivante : 49,67 % en C, 9,14 % en H, 18,08 % en N, 22,84 % en O.

L'analyse du composé IPT-1 par spectrométrie de masse à électronébulisation (electrospray mass spectrometry) permet d'obtenir une valeur de 302,3, correspondant à la masse de ce composé.

Le protocole se poursuit de la façon suivante : 0,4 mmol de résine amino-PEGA^{®} 2 (0,4 mmol/g, Novabiochem) sont imbibés dans un volume minimum (9,6 ml) de diméthylformamide (DMF), puis 400, 3 mg (4,0 mmol) d'anhydride succinique sont ajoutés, en présence de 697 µl (4,0 mmol) de diisopropyléthylamine (DIEA) et de 2 ml de DMF. Le support résultant 3 est lavée par du DMF (2 fois 2 minutes), du dichlorométhane (2 fois 2 minutes) et de la NMP (2 fois 2 minutes).

1209 mg (40 mmol) du composé IPT-1 dissous dans 1 ml de NMP sont ajoutés au support 3 préalablement obtenu, imbibé d'un volume minimum de NMP. 265,4 mg (0,6 mmol) de benzotriazole-1-yl-oxy-tris(diméthylamino)-phosphonium-hexafluorophosphate (BOP) sont ensuite ajoutés en une fois et le mélange est agité pendant 1h. Le support résultant 4 est lavé par du DMF (4 fois 2 minutes), du dichlorométhane (2 fois 2 minutes) et de l'éther (2 fois 2 minutes), puis séchée sous vide.

On obtient ainsi un support de formule I selon l'invention, dans laquelle S est une résine amino-PEGA^{®}, m et n représentent 1, B est une chaîne -(CH₂)₂-CO-NH, Z est un groupement amide, A est une chaîne -CO-NH-(CH₂)₃-, P1 et P2 forment ensemble un groupe protecteur isopropylidène et X' représente une chaîne -NH-(CH₂)₃-NH₂.

Ce support est obtenu par réaction d'un support S-(B)ₘ-Y avec un composé de formule II tel que défini précédemment, avec Y = -COOH et X = -NH₂ (dans la formule II).

### EXEMPLE 2 : Synthèse d'un autre support de formule I selon l'invention à partir d'une résine amino-PEGA^{®} et du (+)-diméthyl-2,3-O-isopropylidène-D-tartrate.

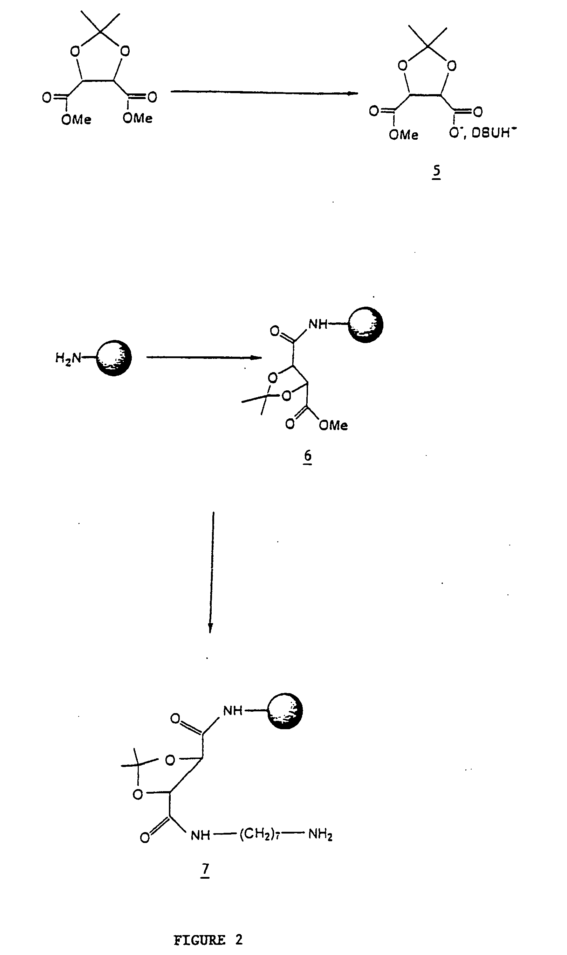

Les différentes étapes de cette synthèse sont résumées sur la figure 2.

7,2 µl (0,4 mmol) d'eau sont ajoutés à 873 µl de (+)-diméthyl-2,3-O-isopropylidène-D-tartrate (fourni par ACROS) à température ambiante. 59,8 µl (0,4 mmol) de 1,8-diaza-bicyclo[5.4.0]undecen-7-ène (DBU) sont ensuite ajoutés à cette solution et le mélange résultant est agité pendant 1 h. On obtient le mélange 5.

0,1 mmol de résine amino-PEGA^{®} (0,4 mmol/g, Novabiochem) sont lavés avec de la DIEA à 5 % dans du dichlorométhane et du DMF. La résine est imbibée d'un volume minimum de DMF, puis l'on y ajoute le mélange 5 ainsi que 177 mg (0,4 mmol) de réactif BOP (agent d'activation électrophile de la fonction carboxylate générée sur le composé 5) dissous dans 1 ml de DMF. Après 40 minutes d'agitation, le support résultant 6 est lavé par du DMF (4 fois 2 minutes), du dichlorométhane (2 fois 2 minutes).

On obtient ainsi un support de formule I selon l'invention, dans laquelle S est une résine amino-PEGA^{®}, m et n représentent 0, Z est un groupement amide, P1 et P2 forment ensemble un groupe protecteur isopropylidène et X' représente un groupe méthoxy. Il est obtenu par réaction d'un support S-(B)ₘ-Y avec un composé de formule II tel que défini précédemment, avec Y = -NH₂ et X = -COO⁻ transformé par un agent d'activation électrophile (BOP).

Une diamine peut avantageusement être greffée au niveau du groupement -COX' afin d'introduire sur le support une fonction amine libre permettant la synthèse de peptides modifiés.

Dans ce but, le support 6 est imbibé dans un volume minimum de DMF, et 1,0 g (7,7 mmol) de 1,7-diaminoheptane dissous dans 1 ml de DMF y sont ajoutés. Après 1 h d'agitation, le support résultant 7 est lavé par du DMF (5 fois 2 minutes), du dichlorométhane (2 fois 2 minutes) et de l'éther (2 fois 2 minutes), puis séchée sous vide.

### EXEMPLE 3 : Synthèse de deux autres supports de formule I selon l'invention à partir de résines aminométhyl-Novagel^{®} et Argogel®-NH₂ et du (+)-diméthyl-2,3-O-isopropylidène-D-tartrate.

Le protocole utilisé est similaire à celui décrit dans l'exemple 2.

Les résines aminométhyl-Novagel^{®} (0,76 nmol/g) et Argogel^{®}-NH₂ (0,41 mmol/g), commercialisées par Novabiochem pour la première résine et par Argonaut Technologies pour la seconde, sont neutralisées par une solution de DIEA 5 % dans CH₂Cl₂ puis gonflées dans un volume minimal de DMF.

4,8 mmol de DBU (717,8 µl) sont ajoutés en une fois à une solution contenant 48 mmol de (+)-dimethyl-2,3-O-isopropylidène-D-tartrate (10,47 ml) et 4,8 mmol d'eau (86,4 4 µl). Après une heure d'agitation, une moitié de cette solution est versée sur 0,6 mmol de résine aminométhyl-Novagel^{®} (0,76 mmol/g) et l'autre moitié sur 0,6 mmol de résine Argogel^{®}-NH₂ (0,41 mmol/g).

Pour chaque résine, 1,06 g (2,4 mmol) de BOP dissout dans 6 ml de DMF pour le premier couplage (45 minutes) et 12 ml pour le second (45 minutes) sont ajoutés en une fois. Les résines ont ensuite été lavées avec du DMF (4 fois 2 minutes) et du CH₂Cl₂ (2 fois 2 minutes), puis traitées par AC₂O/DIEA/CH₂Cl₂ (10% / 5% / 85 %) pendant 10 minutes.

On obtient ainsi des supports de formule I selon l'invention, dans lesquels S est une résine Novagel^{®} ou une résine Argogel^{®}, m et n représentent 0, Z est un groupement amide, P1 et P2 forment ensemble un groupe protecteur isopropylidène et X' représente un groupe méthoxy.

Comme cela a été décrit dans l'exemple 2, une diamine peut avantageusement être greffée au niveau du groupement -COX' du support. Dans ce but, une solution de 1,3-diaminopropane 7,70 M dans le DMF (3 ml) est ajoutée aux supports. Après 1 heure d'agitation, les supports sont lavés avec du DMF (5 fois 2 minutes), du CH₂Cl₂ (2 fois 2 minutes) puis de l'éther (2 fois 2 minutes) avant d'être séchés sous pression réduite. La charge des supports est déterminée en dosant, par spectrométrie UV, l'adduit fulvène-pipéridine libéré après couplage de la Fmoc-Gly-OH et déprotection par de la pipéridine 20 % dans le DMF. La charge est ainsi de 0,34 mmol/g pour le support selon l'invention à base de résine aminométhyl-Novagel® et de 0,23 mmol/g pour le support selon l'invention à base de résine Argogel®-NH₂.

### EXEMPLE 4 : Synthèse de peptides portant une fonction α-oxoaldéhyde à leur extrémité C-terminale à l'aide des supports selon l'invention.

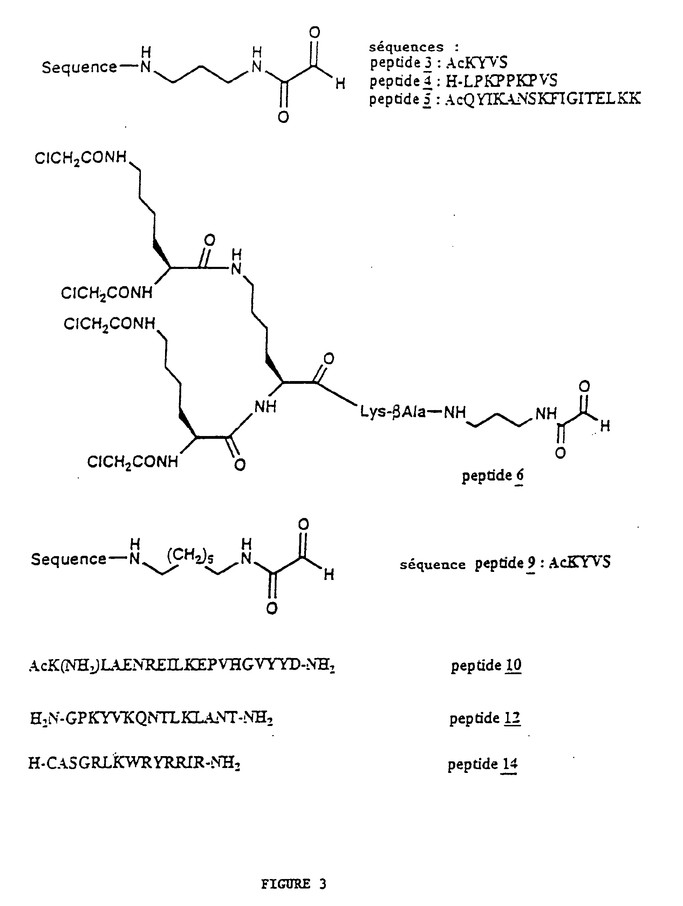

Les peptides 3-6 et 9 synthétisés à l'aide des supports selon l'invention sont représentés sur la figure 3.

### a) Synthèse peptidique en phase solide

Les peptides 3-5 et 9 ont été synthétisés respectivement à partir du support 4 décrit dans l'exemple 1 et du support 6 décrit dans l'exemple 2 (0,2 mmol/g).

La synthèse peptidique a été réalisée de façon automatisée grâce un synthétiseur 431A fourni par Applied Biosystem, selon la méthodologie décrite par Fields, G.B. *et al.* dans Int. J. Pept. Protein, 1990, 35, 161.

Les acides aminés protégés par des groupements Fmoc (9-fluorènylméthoxycarbonyle) ont été fournis par la Société Propeptide (Vert-Le-Petit, France). Les groupements protecteurs des chaînes latérales sont les suivants : Arg(2,2,5,7,8-pentaméthylchroman-6-sulfonyle), Asn (trityle), Asp (tertiobutyle), Gln (trityle), Lys (tertiobutyloxycarbonyle, aussi appelé Boc), Ser (tertiobutyle), Thr (tertiobutyle), Tyr (tertiobutyle).

Les synthèses ont été réalisées à partir de 0,1 mmole de support, 10 équivalents d'acide aminé activé ayant été utilisés pour chaque étape de couplage. Un couplage simple a été utilisé pour les peptides 3 et 9. Pour les autres peptides, les acides aminés ont été couplés deux fois.

A la suite de chaque couplage ou double couplage, une étape de blocage des fonctions amines résiduelles est réalisée pour prévenir la formation de peptides à délétion, avant de reprendre le cours de la synthèse. A cette fin, les fonctions amines sont bloquées par un groupe acétyle par réaction avec un mélange Ac₂0/DIEA/N-méthyl-pyrrolidone (NMP) 4,75/2,25/91,5 (en volume) contenant 7,5 mM de N-hydroxybenzotriazole (HOBt).

Lorsque la synthèse peptidique est terminée, si cela est nécessaire (peptides 3, 5 et 9, qui comportent des fonctions amines acétylées à leur extrémité), l'extrémité du peptide est acétylée par réaction avec le mélange décrit ci-dessus.

Le peptide 6 a été synthétisé de façon manuelle à partir de 0,1 mmole du support 4 décrit dans l'exemple 1 (0,2 mmol/g) en utilisant 10 équivalents d'acide aminé activé pour chaque étape de couplage. Les acides aminés ont été activés par un mélange, dans le DMF, de HBTU/HOBt/DIEA (10 eq./10 eq./30 eq.) (HBTU : N-oxyde d'hexafluorophosphate de N-[(1H-benzotriazol-1-yl) (diméthylamino) méthylène]-N-méthylméthanaminium ; HOBt : N-hydroxybenzotriazole ; DIEA : diisopropyléthyl-amine). Les fonctions amines n'ayant pas réagi lors de l'étape de couplage sont ensuite bloquées par un groupe acétyle (réaction avec un mélange Ac₂O/DIEA/CH₂Cl₂ 10/5/85 en volume). Des couplages simples (1 h) ont été réalisés pour le Fmoc-βAla-OH et le Fmoc-L-Lys(Boc)-OH. Le Fmoc-L-Lys(Fmoc)-OH a été couplé deux fois. De l'acide chloroacétique (2 équivalents) a été couplé deux fois en utilisant une activation au diisopropylcarbodiimide (2 équivalents) dans le DMF pendant 30 minutes.

### b) Déprotection et clivage du support

La déprotection des peptides synthétisés en phase solide, tels que décrits ci-dessus, est effectuée en phase solide en utilisant :
- pour les peptides 3, 4, 6 et 9 : 10 ml d'un mélange acide trifluoroacétique/eau/anisole (95/2,5/2,5 en volume) pendant 2 h à température ambiante ;
- pour le peptide 5 : 10 ml d'un mélange acide trifluoroacétique/eau/triisopropylsilane (95/2,5/2,5 en volume) pendant 3 h à température ambiante.

Les supports sont ensuite lavés avec du dichlorométhane (4 fois 2 minutes) et de l'éther (2 fois 2 minutes), puis séchés sous pression réduite.

Cette étape de déprotection permet à la fois la déprotection des chaînes latérales des acides aminés des peptides et celle des groupements hydroxyles voisins (en positions 1 et 2) du support de formule III selon l'invention, de façon à former le support de formule IV selon l'invention (élimination des groupements protecteurs P1 et P2).

### c) Oxydation périodique en phase solide

0,1 mmole du support sur lequel est attaché le peptide déprotégé, sous forme sèche (étape *b)* ci-dessus), sont imbibés de 5 ml d'un mélange eau/acide acétique (2/1 en volume) pendant 15 minutes. 128,3 mg (6 équivalents) de periodate de sodium dissous dans 2 ml d'un mélange eau/acide acétique (5/1 en volume) sont ensuite ajoutés en une fois. Cette étape permet simultanément la formation de la fonction α-oxoaldéhyde C-terminale et le clivage du peptide du support.

Après 2 minutes d'agitation, le support est filtré et lavé deux fois avec 6 ml d'eau, pendant 1 minute. Cette solution est alors versée dans 200 µl d'éthylèneglycol et injectée dans une colonne de chromatographie liquide haute performance en phase inverse (RP-HPLC) de type C18 Hyperprep, commercialisée par Interchim (Montluçon, France). Les peptides sont purifiés à l'aide d'un gradient linéaire d'un éluant eau / acétonitrile contenant 0,05 % d'acide trifluoroacétique.

Les peptides 3, 4, 6 et 9 ont été directement lyophilisés. Leurs rendements respectifs après purification par RP-HPLC sont de 38,0 %, 26,0 %, 30,1 % et 32,9 %.

Quant au peptide 5, on lui ajoute 500 mg de (+)-D-mannitol (fourni par Sigma) avant de le lyophiliser, ceci afin d'éviter des phénomènes d'agrégation du peptide.

La teneur en peptide 5 (3,3 %) a été déterminée par analyse quantitative des acides aminés en utilisant une détection à la ninhydrine après une hydrolyse acide totale à l'aide d'un mélange HCl 6N/phénol 10/1 à 110°C pendant 24 h. Le rendement en peptide 5 est de 6,1 %.

### EXEMPLE 5 : Synthèse d'un peptide fonctionnalisé en position C-terminale par un α-oxoaldéhyde, ledit perfide contenant des méthionines.

le peptide suivant :

AcKYML-NH-(CH₂)₃-NH-CO-CHO

a été élaboré en phase solide à l'aide du support 4 décrit dans l'exemple 1, selon le protocole décrit dans l'exemple 4. Les groupements protecteurs des chaînes latérales des acides aminés sont respectivement le groupe Boc pour la lysine et le groupe O-tcrtiobutyle pour la tyrosine.

L'étape de déprotection est effectuée en phase solide en utilisant 10 ml d'un mélange acide trifluoroacétique/eau / éthanedithiol (EDT) / triisopropyl-silane (94 / 2,5/ 2,5 / 1 en volume) pendant 2 h à température ambiante.

Le support est ensuite lavé par du dichlorométhane et de l'éther, comme décrit dans l'exemple 4, puis séché sous vide avant l'étape d'oxydation periodique.

L'oxydation periodique du peptide nécessite, de par la présence de méthionines, des conditions particulières, propres à éviter l'oxydation de cet acide aminé (risque de formation de méthionine sulfoxyde) : utilisation d'un pH proche de la neutralité et du diméthylsulfure, qui joue un rôle protecteur en étant oxydé préférentiellement à la méthionine.

Pour l'oxydation périodique et la coupure du peptide du support, on place donc 270 mg de support sec dans un réacteur. On y ajoute 5,4 ml de tampon citrate de sodium (pH 6), 5,4 ml de méthanol et 0,9 ml de Me₂S (diméthylsulfure).

15 minutes après, on ajoute en une fois 63,18 mg (6 équivalents) de periodate de sodium dans 2 ml de tampon citrate. Après 15 minutes d'agitation, on ajoute une nouvelle fois la même quantité de periodate de sodium. Après 15 minutes d'agitation, le surnageant est additionné rapidement à 0,36 ml d'éthylène glycol.

Le support est immédiatement reconditionné dans 5,4 ml de tampon citrate pH 6,0, 5,4 ml de méthanol et 0,9 ml de Me₂S pour être traité de la même manière par le periodate de sodium. Ce protocole est répété 10 fois.

Le peptide est isolé avec un rendement de 15,3 % après purification par RP-HPLC.

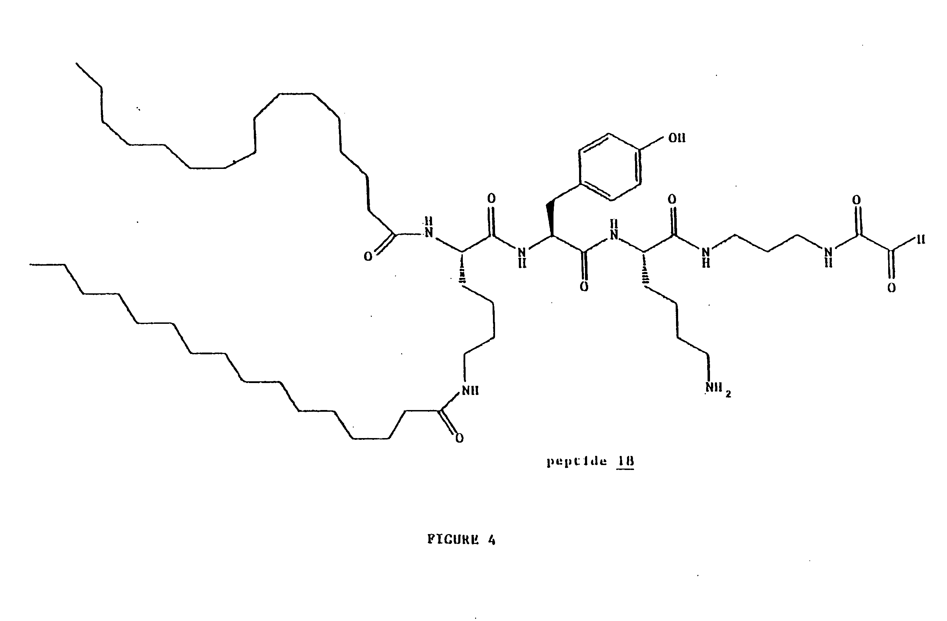

### EXEMPLE 6 : Synthèse du peptide 18 (figure 4) fonctionnalisé en position C-terminale par un α-oxoaldéhyde, ledit peptide étant modifié par des acides gras.

La synthèse peptidique est réalisée à l'aide du support de formule 4 selon l'invention, obtenu comme décrit dans l'exemple 1. On utilise 410 mg de support 4 (0,2 mmol/g, soit 0,082 mmol) gonflé dans le DMF par trois lavages de 2 minutes chacun. Sauf indication contraire, toutes les réactions ont lieu à température ambiante.
- Introduction d'une première lysine.
   76,8 mg de Fmoc-Lys(Boc)-OH (0,164 mmol, soit 2 équivalents), 25,1 mg d'HOBt (0,164 mmol, soit 2 équivalents) et 62,2 mg d'HBTU (0,164 mmol, soit 2 équivalents) sont introduits dans un tube et dissous dans 1 ml de DMF. On y ajoute 115 µl de DIEA (0,656 mmol, soit 8 équivalents). Après 1 minute d'agitation, la solution activée obtenue est déposée sur le support 4. Après 1 h d'agitation, le support est rincé 3 fois 2 minutes par du DMF, puis 3 fois 2 minutes par du dichlorométhane. La disparition de l'amine réactive est évaluée par le test de Kaiser (*Anal. Biochem.,* 1970, 34, 595) qualitatif (négatif) et le test du TNBS *(Anal. Biochem.,* 1976, 71, 260-264) (négatif).
   Une fois que l'amine réactive est éliminée, le support est rincé 3 fois 2 minutes par de la NMP. Le groupement Fmoc est déprotégé par addition d'un large excès de pipéridine à 20 % dans la NMP. Le support est ensuite rincé 3 fois 2 minutes par la NMP et 3 fois 2 minutes par le CH₂Cl₂. La qualité de la déprotection est évaluée par le test de Kaiser qualitatif (positif).
- Introduction d'une tyrosine.
   Le support est rincé 3 fois 2 minutes par le DMF. 75,4 mg de Fmoc-Tyr(tertiobutyle)-OH (0,164 mmol, soit 2 équivalents), 25,1 mg d'HOBt (0,164 mmol, soit 2 équivalents) et 62,2 mg d'HBTU (0,164 mmol, soit 2 équivalents) sont introduits dans un tube et dissous dans 1 ml de DMF. On procède ensuite de la même façon que décrit ci-dessus pour l'introduction d'une lysine.
- Introduction d'une autre lysine.
   Le support est rincé 3 fois 2 minutes par le DMF. 96,9 mg de Fmoc-Lys(Fmoc)-OH (0,164 mmol, soit 2 équivalents), 25,1 mg d'HOBt (0,164 mmol, soit 2 équivalents) et 62,2 mg d'HBTU (0,164 mmol, soit 2 équivalents) sont introduits dans un tube et dissous dans 1 ml de DMF. On procède ensuite de la même façon que décrit ci-dessus pour l'introduction de la première lysine.
- Modification du peptide par des acides gras.
   Le support sur lequel a été synthétisé le tripeptide Lys-Tyr-Lys est lavé 3 fois 2 minutes par un mélange DMF/CH₂Cl₂ 50/50 (en volume). 84,1 mg d'acide palmitique (0,328 mmol, soit 4 équivalents) sont introduits dans un tube et dissous dans 500 µl du mélange DMF/CH₂Cl₂ 50/50. 50,2 mg d'HOBt (0,328 mmol, soit 4 équivalents) et 124,4 mg d'HBTU (0,328 mmol, soit 4 équivalents) sont introduits dans un même tube et dissous dans 1 ml de DMF/CH₂Cl₂ 50/50. Le contenu des deux tubes est mélangé, agité pendant 1 minute, puis on y ajoute 230 µl de DIEA (1,312 mmol, soit 16 équivalents). Après 1 minute d'agitation, la solution activée obtenue est déposée sur le support. Après 1 h d'agitation, le support est lavé 3 fois 2 minutes par le mélange DMF/CH₂Cl₂ 50/50.
   Dans un même tube, on introduit de nouveau 84,1 mg d'acide palmitique (0,328 mmol, soit 4 équivalents) et 146 mg de PyBrop (hexafluorophosphate de bromo-trispyrrolidino-phosphonium) (0,328 mmol, soit 4 équivalents) dissous dans 1 ml de DMF/CH₂Cl₂ 50/50. 173,5 µl de DIEA (0,984 mmol, soit 12 équivalents) sont solubilisés dans 1 ml de DFM/CH₂Cl₂ 50/50. Les deux solutions sont mélangées pendant 1 minute. La solution résultante est déposée sur le support. Après 1 h d'agitation, le support est lavé 3 fois 2 minutes par le mélange DFM/CH₂Cl₂ 50/50, puis 3 fois 2 minutes par le CH₂Cl₂. La disparition de l'amine réactive est évaluée par le test du TNBS (négatif).
- Obtention du peptide 18.

Le support résultant des opérations précédemment décrites est séché sous vide pendant 1 nuit. 20 ml d'un mélange d'acide trifluoroacétique, d'eau et de triisopropylsilane 95/2,5/2,5 (en volume) sont déposés sur le support. On agite pendant 2 minutes, puis on élimine le mélange acide trifluoroacétique / eau /triisopropylsilane et on redépose sur le support 20 ml de ce mélange. Après 20 minutes d'agitation, le support est lavé 3 fois 2 minutes par le CH₂Cl₂. Le support est séché sous vide pendant une nuit.

112 mg de support sec (0,224 mmol) sont lavés 2 fois 2 minutes par du tertiobutanol. 28,9 mg de periodate de sodium (0,135 mmol, soit 6 équivalents) sont dissous dans 200 µl d'eau. On y ajoute 200 µl d'une solution d'acide acétique à 33 % dans l'eau et 200 µl de tertiobutanol. La solution résultante est déposée sur le support.

Après 15 minutes d'agitation, la solution ayant été déposée sur le support est introduite dans un tube contenant 80 µl d'éthylène glycol. Le support est lavé par 600 µl d'un mélange eau/acide acétique à 33 % dans l'cau/tertiobutanol 1/1/1 (en volume). La solution résultante est également introduite dans le tube contenant l'éthylène glycol. Le peptide 18 (figure 4) précipite dans le tube. Sa précipitation est favorisée en placant le tube à -20°C. Le précipité est lavé par 200 µl d'un mélange eau / acide acétique à 33 % dans l'eau/tertiobutanol 1/1/1 (en volume). La solution résultante est replacée à -20°C pour faire précipiter le produit, puis centrifugée. Le culot ainsi récupéré constitue 50 % du produit total formé sur le support. Le reste du produit est extrait après 6 lavages du support par 4 ml d'un mélange tertiobutanol/méthanol à 50°C suivis d'une précipitation à froid dans le mélange d'extraction. Le rendement total de l'extraction est de 79%. En utilisant 112 mg de support (0,224 mmol), 20,1 mg de produit pur 18 ont été isolés. [M+Na]⁺ calculé : 1048 ; trouve : 1048.

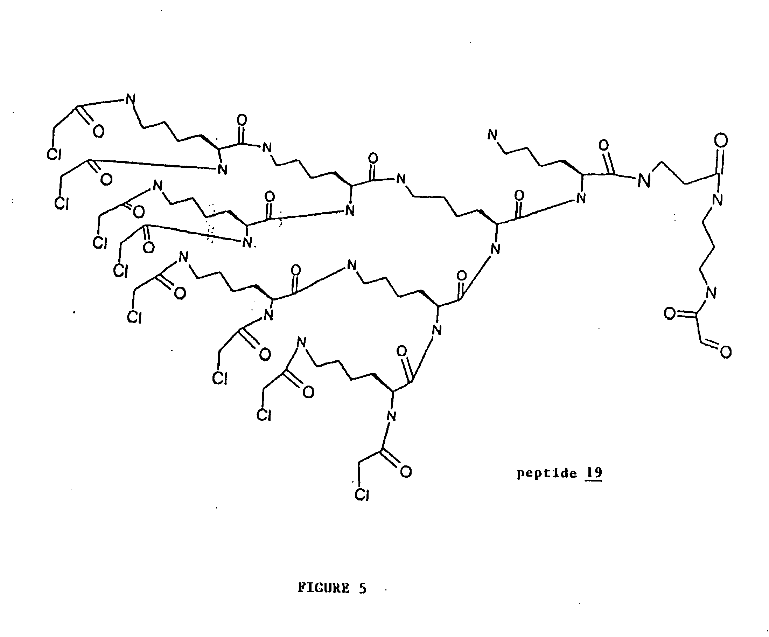

### EXEMPLE 7 : Synthèse du peptide 19 de valence 8 (figure 5), à savoir un peptide de type poly-lysine fonctionnalisé par 8 fonctions chloroacétyles et par une fonction α-oxoaldéhyde, ledit peptide étant synthétisé à l'aide d'un support selon l'invention.

Le peptide 19 est synthétisé manuellement à partir de 0,1 mmol de support 4 (0,2 mmol/g) obtenu selon l'exemple 1. Tous les acides aminés (10 équivalents par fonction -NH₂ libre du support) sont activés avec HBTU/HOBt/DIEA (10 équivalents / 10 équivalents / 30 équivalents) dans le DMF. Les peptides son acétylées par un mélange AC₂O/DIEA/CH₂Cl₂ (dichlorométhane) : 10/5/85 (en volume) après chaque étape de couplage. Des couplages simples (1 h) sont réalisés pour la Fmoc-βA1a-OH et la Fmoc-L-Lys(Boc)-OH. Les Fmoc-L-Lys(Fmoc)-OH sont toutes couplées 2 fois (1 h). L'acide chloroacétique (64 équivalents) est couplé 2 fois avec une activation par le diisopropylcarbodiimide (32 équivalents).

Le peptide fonctionnalisé est déprotégé en phase solide en utilisant 10 ml de TFA/H₂O/anisole (95/2,5/2,5 en volume) pendant 2 heures à température ambiante. Le support est ensuite lavé par du CH₂Cl₂ (4 fois 2 minutes) et de l'éther (2 fois 2 minutes) et séché sous pression réduite.

L'oxydation périodique est réalisée après gonflement du support (0,1 mmol) avec 5 ml d'eau/acide acétique 2/1 (en volume) pendant 15 minutes, en utilisant 128, 33 mg de periodate de sodium dissous dans 2 ml d'eau/acide acétique 5/1 (en volume) et ajoutés en une fois sur le support. La suspension est agitée pendant 2 minutes, puis le support est filtré et lavé par 6 ml d'eau (2 x 1 minute). Les filtrats sont rassemblés et 200 µl d'éthylène glycol sont ajoutés afin de stopper la réaction d'oxydation.

La purification du produit est réalisée par RP-HPLC sur colonne Hyperprep C18 (15 x 300 mm). Le produit est purifié avec un gradient linéaire 0-50% en éluant B pendant 100 minutes, un débit de 3 ml/minute et une mesure de l'absorbance à 215 nm.

Eluant A : eau contenant 0,05 % en volume de TFA.

Eluant B : acétonitrile/eau 4/1 (en volume) contenant 0,05 % de TFA.

Les fractions pures sont directement collectées et lyophilisées. On obtient 45 mg de peptide 19 purifié, soit un rendement de 23.5%.

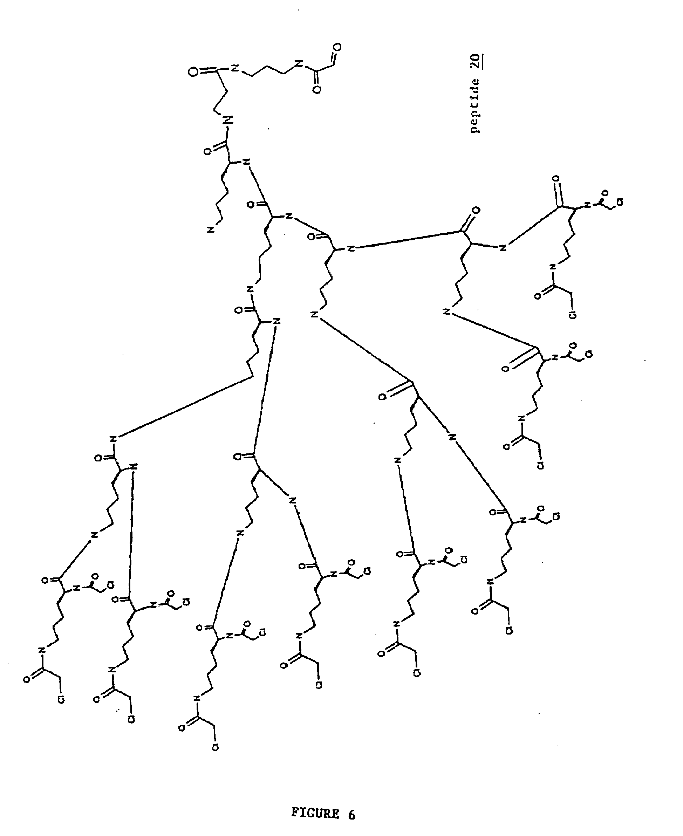

### EXEMPLE 8 : Synthèse du peptide 20 de valence 16 (figure 6), à savoir un peptide de type poly-lysine fonctionnalisé par 16 fonctions chloroacétyles et par une fonction α-oxoaldéhyde, ledit peptide étant synthétisé à l'aide d'un support selon l'invention.

Le peptide 20 est synthétisé à partir de 0,1 mmol de support 6 (0,2 mmol/g) obtenu selon l'exemple 2 et fonctionnalisé par une diamine selon le protocole décrit dans l'exemple 2, la diamine utilisée étant le 1,3-diaminopropane.

Les réactifs d'activation utilisés lors des couplages des acides aminés sont HBTU, HOBt, DIEA dont le nombre d'équivalents utilisé par fonction -NH₂ lors de chaque couplage sera rappelé dans ce qui suit.

La durée d'activation est d'une minute avant introduction sur le support. La déprotection des groupements Fmoc est réalisée par de la pipéridine à 20 % dans la NMP (1 fois 5 minutes et 1 fois 20 minutes). Un test de Kaiser et un test TNBS sont effectués après chaque étape de couplage afin de vérifier l'absence de fonction -NH₂ réactives.

Le premier couplage est effectué avec 10 équivalents de Fmoc-β-Ala-OH/HBTU/HOBt (soit 312 mg/136 mg/379,5 mg)et 12 équivalents de DIEA dans la NMP (soit 210 µl). Après 50 minutes de réaction et lavage du support pour éliminer l'excès de réactif, un test de Kaiser est réalisé pour contrôler l'absence de fonction -NH₂ libre.

Deuxième couplage :
Fmoc-Lys-Boc-OH (4 équivalents, soit 187,4 mg).
Nombre d'équivalents HOBt/HBTU/DIEA :
4/4/12 (54,4 mg/151, 4 mg/210 µl).
Temps de réaction : 50 minutes.

Troisième couplage :
Fmoc-Lys-Fmoc-OH (4 équivalents, soit 236,3 mg).
Nombre d'équivalents HOBt/HBTU/DIEA :
4/4/12 (54,4 mg/151,4 mg/210 µl).
Temps de réaction : 50 minutes.

Quatrième couplage :
Fmoc-Lys-Fmoc-OH (4 équivalents , soit 473 mg).
Nombre d'équivalents HOBt/HBTU/DIEA :
4/4/12 (109 mg/303,6 mg/420 µl).
Temps de réaction : 50 minutes.

Cinquième couplage :
Fmoc-Lys-Fmoc-OH (2,5 équivalents, soit 590 mg).
Nombre d'équivalents HOBt/HBTU/DIEA :
2,5/2,5/7,5 (136 mg/379,5 mg/579 µl)
Temps de réaction : 50 minutes.

Sixième couplage :
Fmoc-Lys-Fmoc-OH (2,5 équivalents, soit 1,18 g)
Nombre d'équivalents HOBt/HBTU/DIEA :
2,5/2,5/7,5 (272 mg/759 mg/1158 µl).
Temps de réaction : 50 minutes.

Le couplage de l'acide chloroacétique (8 équivalents par fonction -NH₂) est réalisé 2 fois en utilisant le diisopropylcarbodiimide comme agent d'activation.

La déprotection du support et des acides aminés est réalisée en utilisant 10 ml de TFA/H₂O/anisole (95/2,5/2,5 en volume) pendant 2 heures à température ambiante. Le support est ensuite lavé par du dichlorométhane et de l'éther avant d'être séché sous pression réduite.

L'oxydation périodique est réalisée après gonflement du support avec 5 ml d'eau/acide acétique 2/1 (en volume) pendant 15 minutes, en utilisant 128 mg de periodate de sodium dissous dans 2 ml d'eau/acide acétique 5/1 (en volume). Après 2 minutes de réaction, le support est filtré puis lavé à l'eau (2 fois 6 ml). Les filtrats sont rassemblés et 200 µl d'éthylène glycol sont ajoutés afin de stopper la réaction d'oxydation.

La purification du produit est réalisée par HPLC sur colonne Hyperprep C18 (15 x 200 mm) en utilisant un gradient linéaire (0-50% d'éluant B pendant 100 minutes), à un débit de 3 ml/min, la mesure de l'absorbance se faisant à 215 nm.

Eluant A : eau contenant 0,05 % en volume de TFA.

Eluant B : eau/acétonitrile (1/4 en volume) contenant 0,05 % de TFA.

Les fractions pures sont collectées et lyophylisées. On obtient 71 mg de peptide 20 purifié, soit un rendement de 20 %.

### EXEMPLE 9 : Ligations chimiques mettant en oeuvre les peptides selon l'invention, fonctionnalisés en position C-terminale par un α-oxoaldéhyde.

### 1) Synthèse des peptides 10, 12 et 14

Ces peptides sont représentés sur la figure 3.

### • Synthèse des peptides 10 et 14

Les peptides 10 et 14 ont été élaborés sur une résine 4-méthyl-benzhydrylamine polystyrène (0,57 mmole de fonctions amines réactives par gramme de résine ; Applied Biosystem, Foster City, USA) selon le protocole décrit par R.B. Merrifield dans J. Am. Chem. Soc., 1963, 85, 2149 et dans Science, 1986, 232, 341 (stratégie Boc/benzyle). Le synthétiseur peptidique automatique est un appareil Applied Biosystem (Foster City, USA) 431A mettant en oeuvre le protocole décrit dans Int. J. Pept. Protein Res., 1992, 40, 180.

Les acides aminés protégés par un groupement Boc ont été fournis par Propeptide (Vert-Le-Petit, France). Les groupements protecteurs des chaînes latérales sont les suivants : Arg (tosyle), Asp (O-cyclohexyle), Asn (trityle), Lys (2-chlorobenzylo-xycarbonyle), Gln (trityle), Tyr (2-bromobenzyloxy-carbonyle), Glu (O-cyclohexyle), Asn (trityle), His (dinitrophényle), Ser (O-benzyle), Cys (para-méthylbenzyle), Trp (formyle).

Pour le peptide 10, la lysine à modifier avec le réactif de N-amination N-Boc-3-(4-cyanophényl) oxaziridine (BCPO) a été introduite sous la forme Boc-L-Lys(Fmoc)-OH, fournie par France Biochem (Meudon, France). Le groupe Fmoc est éliminé par un mélange de pipéridine à 20 % dans le DMF après l'assemblage des acides aminés. Le protocole de N-amination est celui qui a été décrit en rapport avec le synthétiseur automatique 431A Applied Biosystem (Tetrahedron Letters, 1996, 37, 7259-7262 et J. Peptide Res., 1998, 52, 180-184).

Le peptide 10 a été déprotégé et séparé du support en utilisant de l'acide fluorhydrique (HF) liquide, selon les proportions suivantes : support sec / HF / paracrésol / parathiocrésol 1 g/10 ml/0,75 g/0,25 g, pendant 1 h 30 et à 0°C. L'acide fluorhydrique est ensuite éliminé sous pression réduite à 0°C, puis le peptide brut est précipité dans 200 ml d'éther froid. Le précipité est centrifugé, dissous dans un mélange eau/acide acétique, puis lyophilisé.

Le peptide brut est purifié par RP-HPLC sur une colonne Hyperprep, à l'aide d'un gradient linéaire d'un éluant eau/acétonitrile contenant 0,05 % d'acide trifluoroacétique. Le rendement en peptide 10 est de 16 %. Sa masse, obtenue par spectrométrie de masse à électronébulisation, est de 2330,6.

En ce qui concerne le peptide 14, les groupes formyle situés au niveau des tryptophanes ont été enlevés, en phase solide, par un lavage du support avec un mélange pipéridine/DMF (1/10 en volume) pendant 3 h. Le support, sur lequel est greffé le peptide 14, est ensuite lavé avec du DMF, puis de l'éther, et séché sous vide.

La déprotection finale et le clivage du peptide du support sont effectués à l'aide d'un mélange support sec/HF/para-crésol/parathiocrésol (1,5 g/10 ml/0,75 g/0,25 g) pendant 1 h 30 et à 0°C. Le peptide brut est précipité dans l'éther, centrifugé puis dissous dans un mélange eau/acide acétique (3/1 en volume), et enfin lyophilisé. La purification par RP-HPLC est réalisée à l'aide d'une colonne C18 nucléosil de 15 x 300 mm, fournie par Macherey nagel (Paris, France), de façon similaire au peptide 10. Le rendement en peptide 14 est de 44,6 %. Sa masse, obtenue par spectrométrie de masse à désorption de plasma, est de 1823,3.

### • Synthèse du groupement BocNHNHCH₂COOH

31,36 g (0, 144 mmol) de Boc₂O sont ajoutés à 15,80 ml (0,131 mmol) de N-méthylmorpholine et 20 g (0,131 mmol) de chlorhydrate d'éthylhydrazinoacétate dissous dans 130 ml d'un mélange eau/éthanol (1/1 en volume). Le mélange réactionnel est agité pendant 24 h à température ambiante, puis 11,5 g (0,288 mmol) de soude sont ajoutés durant 15 minutes. Après 3 h 45 d(='agitation, le mélange est dilué dans 100 ml d'eau et saturé de chlorure de sodium. Le pH est ajusté à 3,0 à l'aide d'acide citrique solide.

La solution aqueuse résultante est extraite à l'éther (8 fois 50 ml). Les extraits successifs sont réunis, lavés à l'eau (2 fois 50 ml), séchés avec du sulfate de sodium et concentrés sous pression réduite.

Le produit brut obtenu est recristallisé dans un mélange tertiobutylméthyléther/méthanol 10/1 (en volume) pour donner 12,2 g d'un solide blanc (rendement : 43 %) dont l'analyse élémentaire obtenue est de 44,52 % en C, 7,69 % en H, 15,02 % en N et 33,57 % en O. L'analyse RMN ¹H de ce produit est la suivante (300 MHz, MeOH-d₄) : 1,44 (tertiobutyle), 4,06 (CH₂CO).

### • Synthèse du peptide 12

Le peptide 12 a été élaboré à l'aide de 0,25 mmol d'une résine Rink Amide (0,47 mmol/g, France Biochem, Meudon, France), c'est-à-dire une résine 4-(2',4'-diméthoxyphényl-Fmoc-aminométhyl)-phénoxy, de façon similaire aux peptides 3-5 et 9 (exemple 4).

4 équivalents de BocNHNHCH₂COOH préalablement synthétisés ont été greffés à l'extrémité N-terminale du peptide en utilisant un mélange BOP/DIEA (4 éq./12 éq.) dans le DMF.

Le peptide 12 est séparé du support et déprotégé à l'aide de 10 ml d'un mélange acide trifluoro-acétique/thioanisol/eau (90/5/5 en volume) pendant 2 h 30. Le mélange contenant le peptide déprotégé est ajouté goutte-à-goutte à 200 ml d'éther froid. Le précipité est centrifugé, repris dans un mélange eau/acide acétique (9/1 en volume) puis lyophilisé.

Le peptide brut est purifié par RP-HPLC sur une colonne Hyperprep C18 de 15 x 300 mm. On utilise un gradient linéaire de 10 à 40 % en éluant B pendant 100 minutes (éluant B : eau/CH₃CN 1/4 en volume, contenant 0,05 % de TFA ; éluant A : eau contenant 0,05 % de TFA en volume), à un débit de 3 ml/min, l'absorbance étant mesurée à 215 nm.

Le pH des fractions contenant le peptide 12 est ajusté à 6,8 avec du tampon Tris 0,1 M, avant la lyophilisation.

Le solide obtenu (537,9 mg) contient 20,9 % de peptide 12, déterminé comme pour le peptide 5. D'où un rendement de 28,5 % en peptide 12.

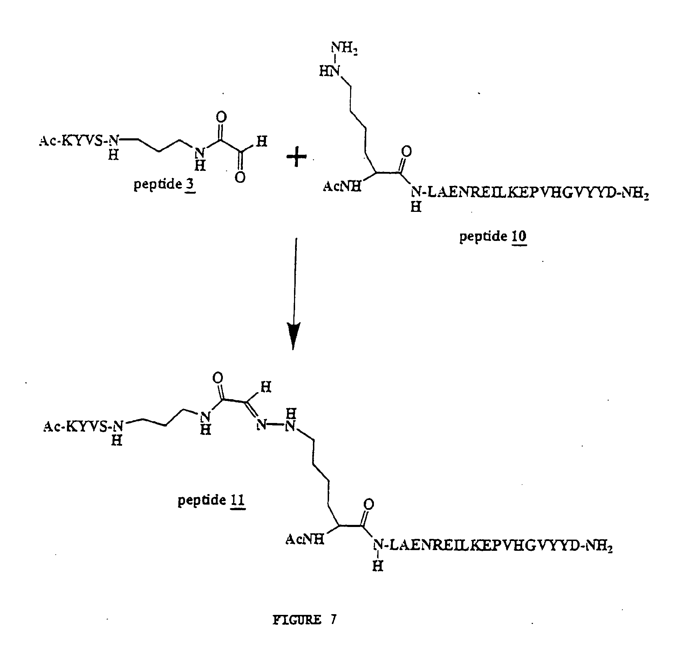

### 2) Ligation de type hydrazone entre le peptide 10 et le peptide 3 (figure 7)

11,2 mg (3,7 µmol) de peptide 10 et 3,2 mg (4,2 µmol) de peptide 3, obtenu selon l'exemple 4, sont dissous dans 2,25 ml de tampon citrate/phosphate (pH 6,0) et dans 560 µl de diméthylsulfoxyde, à température ambiante. Après 48 h, le mélange est purifié par RP-HPLC sur une colonne Hyperprep C18 de 15 x 300 mm. Après lyophilisation, on obtient 6,6 mg de peptide 11, soit un rendement de 48,8 %.

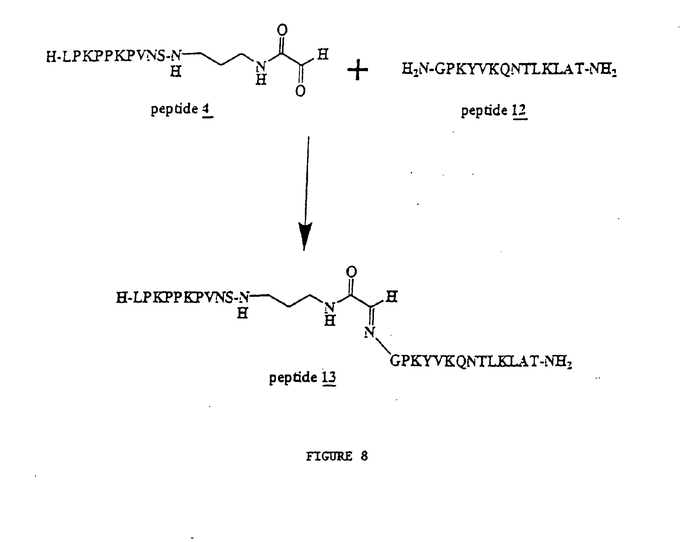

### 3) Ligation de type hydrazone entre le peptide 12 et le peptide 4 (figure 8)

15,65 mg (2,08 µmol) de peptide 12 et 5,87 mg (4,15 µmol) de peptide 4, obtenu selon l'exemple 4, sont dissous dans 1,5 ml de tampon citrate/phosphate (pH 6,0), à température ambiante. Après 48 h, le mélange est purifié par RP-HPLC comme décrit en *2)*. Après lyophilisation, on obtient 6,2 mg de peptide 13, soit un rendement de 47,0 %.

Il ressort des figures 7 et 8 qu'une ligation chimique de type hydrazone entre deux fragments peptidiques peut être réalisée avec de bons rendements, l'un des fragments (peptide 10 de la figure 7 et peptide 12 de la figure 8) portant, au niveau de son extrémité N-terminale, une fonction hydrazine, alors que l'autre fragment peptidique (peptide 3 de la figure 7 et peptide 4 de la figure 8) porte, à son extrémité C-terminale, une fonction α-oxoaldéhyde, cette fonction ayant été introduite grâce à la synthèse peptidique sur un support conforme à l'invention.

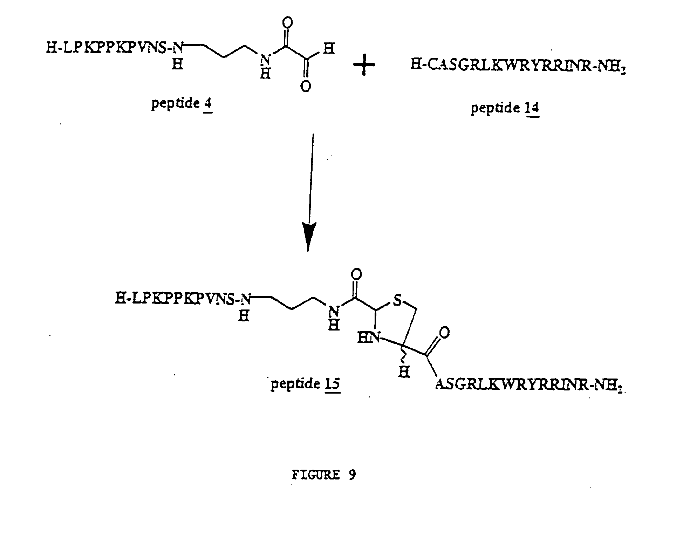

### 4) Ligation de type thiazolidine entre le peptide 14 et le peptide 4 (figure 9)

La réaction a été réalisée sous atmosphère d'azote. 6 mg (2,29 µmol) de peptide 14 sont dissous dans 680 µl d'un mélange 1/1 (en volume) de NMP et de tampon citrate/phosphate (pH 5,1). On ajoute alors 328 µg (1,15 µmol) de chlorhydrate de tris(2-carboxyéthyl)-phosphine dissous dans 48,3 µl du tampon susmentionné, puis 6,49 mg (5,58 µmol) de peptide 4 dissous dans 680 µl de tampon/NMP (1/1 en volume). Le mélange est agité pendant 1 h à température ambiante, puis laissé à 37°C pendant 20 h.

Le mélange est alors purifié par RP-HPLC comme décrit en 2). Après lyophilisation, on obtient 4,8 mg de peptide 15, soit un rendement de 53,7 %.

Il ressort ainsi de la figure 9 qu'un peptide portant une fonction α-oxoaldéhyde au niveau de son extrémité C-terminale, obtenu grâce à une synthèse peptidique réalisée sur un support conforme à l'invention, peut se lier chimiquement, avec un bon rendement, à un peptide portant un résidu cystéine (fonction β-amino-thiol) en position N-terminale, donnant lieu à la formation d'une liaison thiazolidine entre les deux fragments peptidiques.

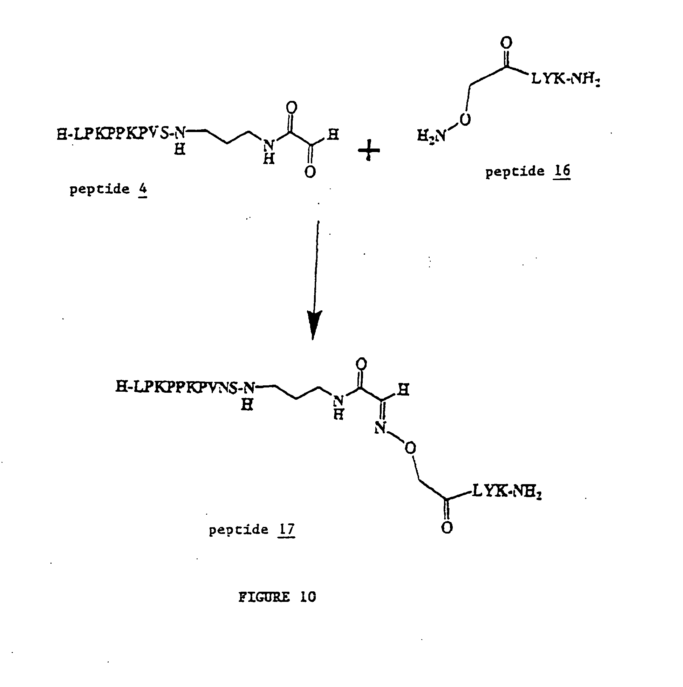

### 5) Ligation de type oxime entre le peptide 16 et le peptide 4 (figure 10)

### • Synthèse du peptide 16

Ce tripeptide a été synthétisé en stratégie Boc/benzyle à partir de 0,5 mmole de résine tBoc-Leu-OCH₂-PAM (667 mg, charge 0,75 mmol/g). Après déprotection de la leucine par un mélange TFA/CH₂Cl₂ (1/1 en volume, pendant 30 minutes), les acides aminés Boc-L-Tyr(2-bromobenzyloxy-carbonyle)-OH (988,4 mg, 4 équivalents, simple couplage) et Boc-Lys(2-chlorobenzyloxycarbonyle)-OH (428,3 mg, 4 équivalents, double couplage) ont été activés pendant 1 minute avec HBTU/DIEA (756 mg, soit 4 équivalents / 1044 µL, soit 12 équivalents) avant d'être ajoutés à la résine solvatée par un volume minimal de NMP. Ensuite, Boc-HN-O-CH₂-CO₂H (191,2 mg, soit 2 équivalents) et BOP (440 mg, soit 2 équivalents) ont été ajoutés sur la résine solvatée par un volume minimal de NMP. La DIEA (500 µl, soit 6 équivalents) est ajoutée en une fois et la suspension agitée 45 minutes à température ambiante.

La déprotection et le clivage du peptide du support (sur 0,8 g) ont été effectués par HF/paracrésol/parathiocrésol (10 ml/0,2 g/0,2 g) pendant 1 h 30 à 0°C. Après évaporation de l'acide fluorhydrique, le résidu est mis en suspension dans du TFA, filtré et le filtrat est additionné goutte à goutte dans de l'éther froid. Le précipité est centrifugé, et purifié par RP-HPLC sur une colonne C18 Hyperprep (20 x 300 mm). Eluant A : TFA 0,05% dans H₂O et éluant B : TFA 0,05% dans isopropanol / H₂O : 3/2. Gradient 0-10% d'éluant B en 150 minutes, débit 1 ml/min, détection à 215 nm.

### • Ligation chimique

3,53 mg du peptide 4, obtenu selon l'exemple 4, sont dissous dans 200 µl d'eau. On y ajoute 3,25 mg de peptide 16 dissous dans 1,2 ml de tampon acétate de sodium/acide acétique pH 4,6, préparé en mélangeant 510 µl d'acide acétique 0,2 M à 492 µl d'acétate de sodium 0,2 M et en complétant à 2 ml avec de l'eau.

Après 42 h de réaction, le peptide 17 est purifié par RP-HPLC comme décrit en *2)*. Après lyophilisation, on obtient 4,0 mg de peptide 17, soit un rendement de 78,13 %.

Il ressort ainsi de la figure 10 qu'un peptide portant une fonction α-oxoaldéhyde au niveau de son extrémité C-terminale, obtenu grâce à une synthèse peptidique réalisée sur un support conforme à l'invention, peut se lier chimiquement, avec un très bon rendement, à un peptide portant une fonction hydroxylamine en position N-terminale, donnant lieu à la formation d'une liaison oxime entre les deux fragments peptidiques.

### EXEMPLE 10 : Recyclage d'un support selon l'invention après son utilisation dans un procédé de synthèse de composés organiques comportant au moins une fonction α-oxoaldéhyde.

L'oxydation périodique réalisée au cours du procédé selon l'invention, pour la synthèse de composés organiques comportant au moins une fonction α-oxoaldéhyde, génère également une fonction aldéhyde sur le support solide, c'est-à-dire le produit de formule V :

OHC-(A)ₙ-Z-(B)ₘ-S (V)

Dans cet exemple, la régénération d'un support portant une fonction amine à son extrémité est décrite, dans le cas où le groupement -(A)ₙ-Z-(B)ₘ- est un groupe amide (-CO-NH-), c'est-à-dire dans le cas où le support solide, après l'étape d'oxydation périodique, répond à la formule VI :

OHC-CO-NH-S (VI)

Une ou plusieurs fonctions amines peuvent être générées à partir de l'α-oxoaldéhyde par amination réductrice.

On peut par exemple faire réagir le produit de formule VI avec NH₄Br/NaCNBH₃ dans le méthanol, ce qui donnera le produit de formule VII suivante :

H₂N-CH₂-CO-NH-S (VII)

On peut également faire réagir le produit de formule V avec un mélange NaCNBH₃/1,3-diaminopropane (ou une autre diamine de formule H₂N-(CH₂)ₐ-NH₂) dans le méthanol, ce qui donnera le produit de formule VIII suivante :

H₂N- (CH₂)ₐ-NH-CH₂-CO-NH-S (VIII)

Dans ce dernier cas, l'utilisation d'une diamine primaire permet de créer, sur le support régénéré, une fonction amine primaire et une fonction amine secondaire, ce qui multiplie la charge du support par deux. L'utilisation d'une diamine secondaire aurait permis de créer sur le support une amine tertiaire et une amine secondaire, la charge initiale du support étant ainsi conservée.

Les réactions d'aminations réductrices décrites ci-dessus sont bien connues de l'Homme de l'Art, comme en témoigne l'ouvrage " *Reduction by the alumino- and borohydrides in organic synthesis ",* J. Seyden-Penne, 1991, VCH Publishers Inc./Lavoisier, New-York.

Une fois l'amination réductrice effectuée, il est ensuite possible de régénérer un support de formule I selon l'invention, en faisant réagir le produit obtenu (produit VII ou VIII)avec un composé de formule II tel que décrit précédemment.

Par exemple, si l'amination réductrice est effectuée avec NH₄Br/NaCNBH₃ ou avec une diamine secondaire, la réaction du produit de réduction, par exemple le composé de formule VII, avec le composé de formule II donne le support suivant :

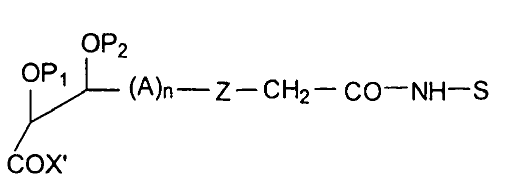

Si l'amination réductrice est effectuée avec une diamine primaire, par exemple avec une diamine de formule H₂N-(CH₂)₃-NH₂, la réaction du produit de réduction, par exemple le composé de formule VIII, dans lequel a = 3, avec le composé de formule II donne le support suivant :

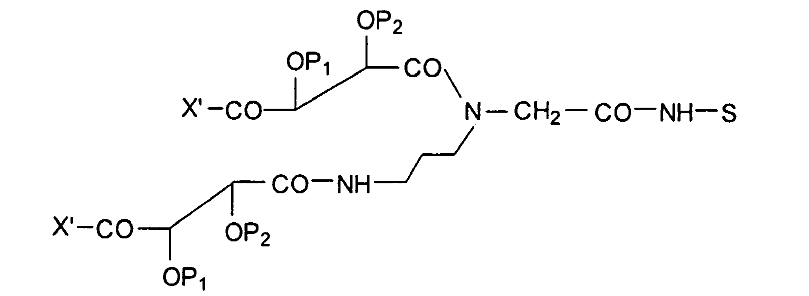

Ces exemples démontrent ainsi l'utilité des peptides selon l'invention, qui portent une fonction α-oxoaldéhyde au niveau de leur extrémité C-terminale et qui sont obtenus à l'aide d'un support selon la présente invention, dans le cadre de la ligation chimique.

### EXEMPLE 11 : Synthèse d'un oligonucléotide fonctionnalisé par un α-oxoaldéhyde à l'aide d'un support selon l'invention.

Le support utilisé répond à la formule générale I décrite précédemment, dans laquelle n = m = 0, P1 et P2 représentent des groupes acétyles CH₃CO-, X' représente un groupe -OH, Z représente une liaison amide et S est un verre borosilicaté. Un tel support est obtenu en faisant réagir un dérivé de l'acide tartrique, dont les fonctions hydroxyles en positions 1,2 sont protégées par des groupes acétyles, avec un verre borosilicaté fonctionnalisé par une fonction amine, la fonctionnalisation de ce verre étant réalisée par des techniques bien connues de l'Homme de l'Art, décrites par exemple dans " *Oligonucleotides synthesis : a practical approach* ", 1984, M. J. GAIT Ed., IRL Press, Washington D.C. Le dérivé de l'acide tartrique utilisé est par exemple l'anhydride diacétyl-tartrique (anhydride cyclique), ou l'acide tartrique dont les fonctions hydroxyles en positions 1,2 sont protégées par des groupes acétyles et qui est activé par le BOP.

Le support obtenu est fonctionnalisé par le 1,3-diaminopropane, en utilisant encore le BOP comme réactif d'activation. L'accrochage d'oligonucléotides sur le support résultant est effectué conformément aux références suivantes : *" Oligonucleotides synthesis : a practical approach",* 1984, M. J. GAIT Ed., IRL Press, Washington D.C., " *Current Protocols in molecular biology ",* 1994, John Wiley & Sons Ed., sections 2.11.1-2.11.25.

L'accrochage d'une première base désoxynucléotidique se fait par exemple *via* une liaison carbamate : réaction du support obtenu ci-dessus avec du phosgène (création d'une fonction isocyanate sur le support), puis réaction du désoxynucléotide dont la fonction hydroxyle en position 5' du sucre est protégée par un groupe diméthoxytrityle, le désoxynucléotide se fixant au support par sa fonction hydroxyle en position 3'. La base du désoxynucléotide est protégée par des groupement acyles, par exemple un benzoyle ou un méthyl-2-propanoyle.

De proche en proche, l'accrochage successif de plusieurs bases désoxynucléotidiques permet la synthèse en phase solide d'ADN. De façon similaire, une synthèse d'ARN selon ce même protocole pourrait être réalisée, l'hydroxyle en position 2' du sucre étant alors convenablement protégé, par exemple par un groupe tertiobutylediméthylsilyle.

En fin de synthèse, les groupes P1 et P2 du support selon l'invention sont éliminés par un traitement en milieu basique, par exemple par l'ammoniaque. Ce traitement permet simultanément la déprotection des bases de l'oligonucléotide. Une étape d'oxydation périodique en phase solide, telle que décrite dans l'exemple 4, permet ensuite d'obtenir l'oligonucléotide (ADN ou ARN) fonctionnalisé par un α-oxoaldéhyde. Dans le cas de la synthèse d'ARN, le traitement en milieu basique sus-mentionné est précédé d'une étape de déprotection du groupe hydroxyle en position 2' du sucre, par exemple par le Bu₄N⁺, F⁻.

### EXEMPLE 12 : Synthèse d'un peptide fonctionnalisé par un α-oxoaldéhyde à l'aide d'un support selon l'invention et utilisation de ce peptide en tant que réactif de diagnostic.

### 1) Préparation d'un support selon l'invention.

On utilise une résine amino PEGA^{®} de charge sèche égale à 0,3 mmol/g et de charge humide (dans le méthanol) de 0,06 mmol/g. La quantité de résine utilisée est de 25,1 g, soit 1,5 mmol. La résine est lavée trois fois par du dichlorométhane, trois fois par de la DIEA à 5% dans le dichlorométhane, puis trois fois par du DMF. La résine est ensuite imbibée avec un volume minimum de DMF.

Dans 108 µl d'eau (6 mmol), on ajoute 13,11 ml (69,45 µmol) de (+)-diméthyl-2,3-O-isopropylidène-D-tartrate, puis 897 µl de DBU. Le mélange est agité pendant 1 heure à température ambiante, puis ajouté à la résine préparée ci-dessus. Après 30 secondes d'agitation, on ajoute 3122 mg (6 mmol) de PyBOP (benzotriazole-1-yl-oxy-tris-pyrrolidino-phosphonium hexafluorophosphate). Le milieu réactionnel est laissé 40 minutes à température ambiante et sous agitation. On effectue ensuite 4 lavages de la résine à l'aide de DMF, puis 4 lavages à l'aide de dichlorométhane. On obtient un support de formule I selon l'invention identique au support 6 préparé conformément à l'exemple 2.

Du 4,7,10-trioxa-1,13-tridecanediamine (TTD) est greffé à l'extrémité du support afin d'introduire une fonction amine libre à la surface de ce dernier, permettant la synthèse ultérieure de peptides. Dans ce but, le support est imbibé avec un volume minimum de DMF, puis on ajoute 6,61 ml de TTD (30 mmol, soit 20 fois plus que la quantité initiale de résine amino PEGA^{®}). Après 1 heure d'agitation, le support est lavé 5 fois par du DMF, 2 fois par du dichlorométhane et 2 fois par de l'éther, puis séché sous vide. Le support obtenu présente une charge de 0,18 mmol/g, déterminée après couplage de la Fmoc-Gly-OH sur une quantité connue de support et dosage de l'adduit pipéridine/dibenzofulvène par spectrométrie UV.

### 2) Synthèse d'un peptide fonctionnalisé par un α-oxoaldéhyde.

Le peptide de séquence suivante :
AVDTGSGGGGQPHDTAPRGARKKQ
est synthétisé sur le support solide préparé ci-dessus en utilisant la stratégie Fmoc/tert-butyl dans un synthétiseur de peptides de la marque Pionner (Perseptive Biosystems). Les groupes protecteurs des chaînes latérales sont les suivants (^{t}Bu : tertiobutyle ; Trt : trityle ; Boc : tertio-butyleoxycarbonyle ; Pbf : 2,2,4,6,7-pentaméthyldihydrobenzofuran-5-sulfonyle) : Arg (Pbf), Asn (Trt), Asp (O^{t}Bu), Cys (Trt), Gln (Trt), Glu (O^{t}Bu), His (Trt), Ser (^{t}Bu), Thr (^{t}Bu), Trp (Boc), Lys (Boc), Tyr (^{t}Bu).

La synthèse peptidique est effectuée avec 0,555 g de support. Les acides aminés sont utilisés en excès de 10 et activés par HOBt/HBTU (0,5 M) dans le DMF. On effectue pour chaque aminé un double couplage, suivi d'un capping.

Une fois la synthèse peptidique effectuée, le peptide, porteur d'une fonction amine primaire terminale, est déprotégé sur le support avec un mélange TFA/eau/anisole (95/2,5/2,5). Le support est lavé par du dichlorométhane (3 x 2 minutes), de l'éther éthylique (2 x 2 minutes), puis séché sous vide.

La coupure periodique générant la fonction α-oxoaldéhyde terminale est réalisée par les étapes suivantes : suspension du support dans 5 ml d'acide acétique, ajout de 6 équivalents de NaIO₄ agitation pendant 5 minutes, ajout de 100 µl d'éthanolamine et agitation pendant 1 minute.

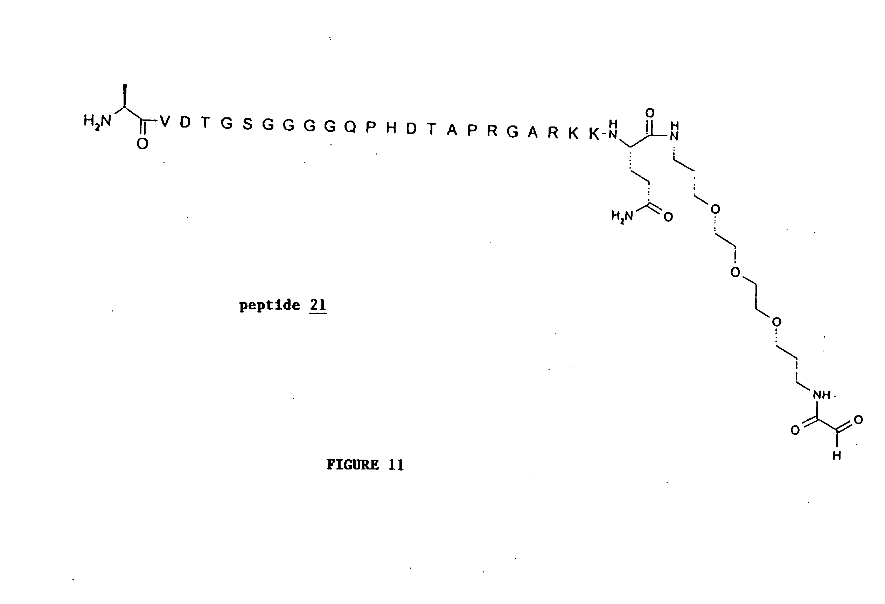

La solution récupérée, qui contient le peptide 21 (figure 11) porteur d'une fonction α-oxoaldéhyde terminale, est purifié par dessalage sur colonne Sephadex^{®} G10 (60 cm x 2,5 cm). Les conditions de chromatographie sont les suivantes :
- solvant : acide acétique 5%,
- débit du solvant : 0,7 ml/minute,
- vitesse de la pompe : 30 tours/minute,
- longueur d'onde pour la détection du peptide : 240 nm.

La fraction chromatographique correspondant au peptide est lyophilisée, puis le peptide est purifié par RP-HPLC préparative sur colonne C18. Les conditions de chromatographie sont les suivantes :
- phase stationnaire : C18 Nucleosil^{®},
- phase mobile : eau contenant 0,05% de TFA, avec un gradient linéaire de 0 à 80% d'acétonotrille,
- température : 60°C,
- débit de la phase mobile : 3 ml/minute,
- longueur d'onde pour la détection du peptide: 215 nm.

La fraction chromatographique correspondant au peptide est lyophilisée, puis caractérisée. L'homogénéité du peptide est confirmée par HPLC analytique sur colonne C18-VYDAC^{®} éluée avec le même solvant que ci-dessus. L'identité du peptide est confirmée en déterminant la composition en acides aminés, par une hydrolyse acide totale pendant 24 heures avec un mélange HCl 6N/phénol (10/1) et par spectrométrie de masse enregistrée sur un spectromètre Bio Ion 20 plasma fourni par la société Bio Ion AB, Upsala, Suède (masse calculée : 2605 g/mol ; trouvée : 2605 g/mol).

### 3) Réactif de diagnostic de type ELISA préparé à l'aide de ce peptide.

Des puits de plaques de microtitration (Carbo-bind, New York, USA) sont recouverts du peptide synthétisé ci-dessus (0,1 ml de peptide de concentration 0,5 µg/ml dans un tampon acétate de sodium 0,1 M, pH 5,5), pendant 1 heure et en atmosphère humidifiée.

Chaque puits subit ensuite 3 lavages dans un laveur automatique avec du tampon phosphate 0,01 M comprenant 1,8% de NaCl, pH 7,4 (PBS) et les sites de liaison en excès sont bloqués par du lait entier en poudre (addition de 0,2 ml de lait en poudre à 2,5% dans du PBS), l'incubation étant réalisée à 37°C pendant 60 minutes.

Après 3 lavages effectués à l'aide de PBS comprenant 0,05% de Tween 20 (tampon PBS-T, commercialisé par Sigma), les sérums humains à tester sont dilués au 1/50^{ème} dans du PBS comprenant 2,5% de lait entier et 0,5% de Tween 20. 0,1 ml de ces sérums dilués sont incubés dans des puits contenant le peptide 21 synthétisé ci-dessus, pendant 120 minutes à 37°C.

Après 4 lavages à l'aide de PBS-T, 0,1 ml de conjugués peroxydase-anticorps de chèvre-anti-IgG-A-M humaines (Diagnostic Pasteur), dilués au 1/10000^{ème} dans du tampon PBS comprenant 0,5% de Tween 20 et 2,5% de lait entier, sont incubés pendant 60 minutes à 37°C.

Après 4 lavages en PBS-T, l'activité peroxydase de l'anticorps conjugué, qui se lie aux Ig fixées sur le support, est mesurée en utilisant comme substrat 0,1 ml de dihydrochlorure d'o-phénylènediamine et de l'H₂O₂, dans un tampon citrate 0,05 M, pH 5,5, pendant 30 minutes à l'obscurité et à température ambiante.

La réaction est bloquée par l'addition d'H₂SO₄ 2N (25 µl). L'absorbance est enregistrée contre un blanc à 492 nm avec un lecteur automatique multicanaux (Mr 5000, Dynatech).

La moyenne A₄₉₂ + 3 écarts type des échantillons EBV (Epstein-Barr Virus)-négatifs est utilisée comme valeur seuil dans les tests ELISA. Une seconde manière d'exprimer les résultats est de calculer le rapport R : [A₄₉₂ sérum positif]/[(moyenne A₄₉₂ + 3 écarts type) sérums négatifs], les sérums considérés positifs devant avoir un rapport R supérieur à 1.

Les résultats obtenus pour les tests ELISAS réalisés sur les plaques Carbo-Bind sont présentés dans le tableau I. Les mêmes tests ont été effectués à partir d'une plaque classique non-covalente (Nunc, Maxisorp, Rocksilde, Danemark). La comparaison des rapports R des deux tests montre que les plaques Carbo-Bind, sur lesquelles les peptides sont liés de façon covalente, permettent d'orienter le peptide, d'améliorer le signal des sérums positifs et de réduire le signal et la dispersion des sérums négatifs, ainsi que de diminuer le bruit de fond.

**Tableau I**

| | **Plaque Carbo-Bind** | | **Plaque classique** | |
|---|---|---|---|---|
| Sérums positifs | Absorbance | Rapport R | Absorbance | Rapport R |
| 1 | 0,58 | 4,14 | 1,56 | 1,71 |
| 2 | 0,27 | 1,93 | 0,61 | 0,67 |
| 3 | 0,33 | 2,36 | 1,62 | 1,78 |
| 4 | 0,17 | 1,21 | 1,04 | 1,14 |
| 5 | 0,57 | 4,07 | 1,40 | 1,54 |
| 6 | 1,40 | 10,00 | 1,52 | 1,67 |
| 7 | 0,04 | 0,29 | 0,85 | 0,93 |
| 8 | 0,14 | 1,00 | 0,41 | 0,45 |
| 9 | 0,05 | 0,36 | 0,80 | 0,88 |
| 10 | 0,43 | 3,07 | 1,00 | 1,10 |
| 11 | 0,55 | 3,93 | 1,55 | 1,70 |
| 12 | 0,08 | 0,57 | 0,64 | 0,70 |
| 13 | 1,62 | 11,57 | 0,93 | 1,02 |
| 14 | 1,05 | 7,50 | 1,80 | 1,98 |
| 15 | 0,07 | 0,50 | 0,52 | 0,57 |
| 16 | 0,73 | 5,21 | 0,75 | 0,82 |
| 17 | 1,56 | 11,14 | 1,83 | 2,01 |
| 18 | 1,72 | 12,29 | 1,50 | 1,65 |
| 19 | 1,33 | 9,50 | 1,56 | 1,71 |
| 20 | 1,61 | 11,50 | 2,03 | 2,23 |
| 21 | 0,23 | 1,64 | 0,42 | 0,46 |
| 22 | 0,36 | 2,57 | 0,87 | 0,96 |
| 23 | 1,67 | 11,93 | 1,48 | 1,63 |
| Sérums négatifs | Absorbance | | Absorbance | |
| 1 | 0,05 | | 0,26 | |
| 2 | 0,03 | | 0,22 | |
| 3 | 0,03 | | 0,21 | |
| 4 | 0,02 | | 0,05 | |
| 5 | 0,07 | | 0,25 | |
| 6 | 0,02 | | 0,17 | |
| 7 | 0,03 | | 0,11 | |
| 8 | 0,07 | | 0,34 | |
| 9 | 0,09 | | 0,47 | |
| 10 | 0,04 | | 0,14 | |
| 11 | 0,03 | | 0,10 | |
| 12 | 0,09 | | 0,97 | |
| 13 | 0,06 | | 0,21 | |
| 14 | 0,04 | | 0,17 | |
| 15 | 0,06 | | 0,38 | |
| 16 | 0,04 | | 0,09 | |
| 1.7 | 0,03 | | 0,23 | |
| 18 | 0,03 | | 0,09 | |
| 19 | 0,07 | | 0,14 | |
| 20 | 0,17 | | 0,74 | |
| 21 | 0,04 | | 0,11 | |
| 22 | 0,04 | | 0,13 | |
| 23 | 0,07 | | 0,20 | |
| **Moyenne** | 0,05 | | 0,25 | |
| **Ecart type** | 0,03 | | 0,22 | |
| **Seuil de positivité** | 0,14 | | 0,91 | |

## Revendications

1. Support solide fonctionnalisé pour la synthèse de composés comportant au moins une fonction α-oxoaldéhyde, lequel support esc **caractérisé en ce q**u'il répond à la formule I suivante : 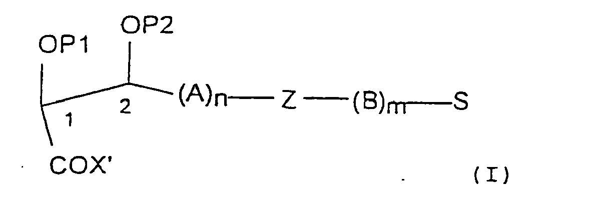 dans laquelle :
- S représente un support solide dont au moins la surface a la propriété d'être bien solvatée en milieu aqueux ou partiellement aqueux ;
- n et m, qui peuvent être identiques ou différents, représentent 0 ou 1 ;
- Z représente un groupement sélectionné dans le groupe constitué par les groupements éther, thioéther, ester, amine, amide, sulfonamide, hydroxylamine, hydrazine, hydrazone, thiazolidine, oxime, carbonate, carbamate, thiocarbamate, urée, thiourée et leurs dérivés lorsque n est égal à 1 ;
- Z représente un groupement sélectionné dans le groupe constitué par les groupements ester, amide, N-acylhydroxylamine, hydrazide, hydrazone, oxime, thiazolidine et leurs dérivés lorsque n est égal à 0 ;
- P1 et P2, qui peuvent être identiques ou différents ou former ensemble un cycle avec les atomes d'oxygène auxquels ils sont attachés, représentent des atomes d'hydrogène ou des groupements protecteurs des fonctions hydroxyles en positions 1,2, P1 et P2 étant dans ce cas sélectionnés dans le groupe constitué par les groupements -R₁, -(CO)R₁, -(CO)OR₁, -(SO₂)OR₁, -SiR'R''R''', acétals et cétals, R₁, R', R'' et R''' représentant des groupes alkyles comprenant de 1 à 18 atomes de carbone, linéaires, ramifiés ou cycliques, saturés ou insaturés, ou bien des groupes aryles ou hétéroaryles, lesdits groupes alkyles, aryles ou hétéroaryles étant éventuellement partiellement ou intégralement substitués par un ou plusieurs atomes d'halogènes, un ou plusieurs groupes amino, hydroxy, alcoxy, aryloxy, alkylthio ou arylthio ;
- A et B, qui peuvent être identiques ou différents, représentent une chaîne carbonée linéaire, ramifiée ou cyclique, saturée ou insaturée, comportant de 1 à 18 atomes de carbone et éventuellement de 1 à 7 groupements sélectionnés dans le groupe constitué par les groupements carbonyles, les cycles carbonés, les hétérocycles, les aryles et les hétéroaryles, A et/ou B comportant éventuellement en outre de 1 à 16 hétéroatomes, de préférence de 1 à 16 atomes d'azote ou d'oxygène, et pouvant être substitués par 1 à 16 groupements hydroxyles ou amino éventuellement convenablement protégés,
l'un au moins des carbones de A et/ou de B étant éventuellement substitué par un groupement : 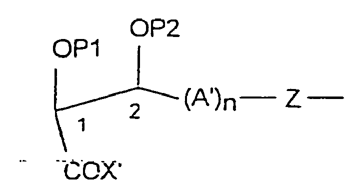 dans lequel A' est identique ou différent de A ; dans ce dernier cas, A' est sélectionné parmi les autres groupements A définis ci-dessus ; et
- X' est sélectionné dans le groupe constitué par les groupes -OR₁, -NR'R'' et 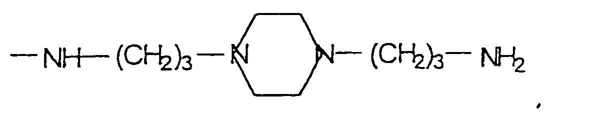
R₁, R' et R'' représentant un atome d'hydrogène ou étant tels que définis ci-dessus.

2. Support selon la revendication 1, **caractérisé en ce que** ledit support solide S est sélectionné dans le groupe constitué par :
- les particules en verre borosilicaté poreux,
- les surfaces en verre ou en silicium,
- les résines sélectionnées dans le groupe constitué par les résines polyacrylamides, les résines polyacrylamide-polyéthylèneglycol, les résines polyéthylèneglycol-polystyrène, les résines réticulées éthoxylate-acrylate et les résines de type polyéthylène, polystyrène ou polypropylène greffé par des molécules hydrophiles.

3. Support de formule I selon la revendication 1 ou la revendication 2, **caractérisé en ce que** :
- S est une résine de type polyacrylamide-polyéthylèneglycol,
- m et n représentent 1,
- B est une chaîne -(CH₂)₂-CO-NH,
- Z est un groupement amide,
- A est une chaîne -CO-NH-(CH₂)₃-,
- P1 et P2 forment ensemble un groupement protecteur isopropylidène et
- X' représente une chaîne -NH-(CH₂)₃-NH₂.

4. Support de formule I selon la revendication 1 ou la revendication 2, **caractérisé en ce que** :
- S est une résine de type polyacrylamide-polyéthylèneglycol ou polyéthylèneglycol-polystyrène,
- m et n représentent 0,
- Z est un groupement amide,
- P1 et P2 forment ensemble un groupe protecteur isopropylidène et
- X' représente un groupe méthoxy.

5. Support de formule I selon la revendication 1 ou la revendication 2, **caractérisé en ce que** :
- S est tel défini dans la revendication 1 ou la revendication 2,
- m représente 1,
- n représente 0,
- Z est un groupement amide,
- P1 et P2 forment: ensemble un groupe protecteur isopropylidène,
- X' représente un groupe méthoxy et
- B représente le groupe suivant :

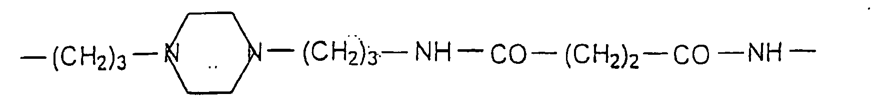

6. Support de formule I selon la revendication 1 ou la revendication 2, **caractérisé en ce que** :
- S est tel défini dans la revendication 1 ou la revendication 2,
- m et n représentent 1,
- Z est un groupe amide,
- P1 et P2 forment ensemble un groupe protecteur isopropylidène,
- B est un groupe -(CH₂)₂-CO-NH-,
- A représente le groupe suivant : 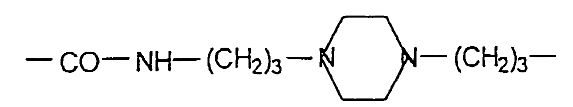 et
- x' représente le groupe suivant :

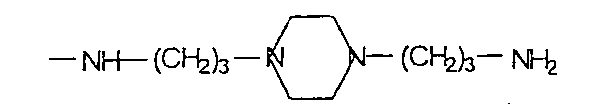

7. Support de formule I selon la revendication 1 ou la revendication 2, **caractérisé en ce que** :
- S est tel défini dans la revendication 1 ou la revendication 2,
- m représente 0,
- n représente 1,
- Z est un groupe carbamate,
- P1 et P2 forment ensemble un groupe protecteur isopropylidène,
- X' représente un groupe méthoxy et
- A représente le groupe suivant :

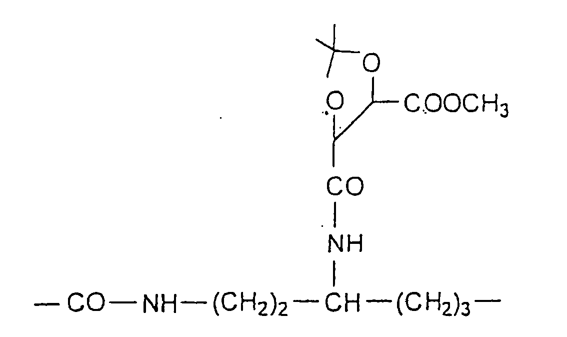

8. Support de formule I selon la revendication 1 ou la revendication 2, **caractérisé en ce qu'**il répond à la formule suivante :

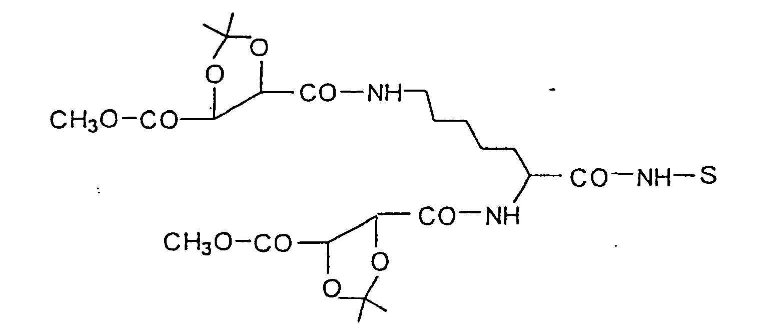

9. Support de formule I selon la revendication 1 ou la revendication 2, **caractérisé en ce qu'**il répond à la formule suivante :

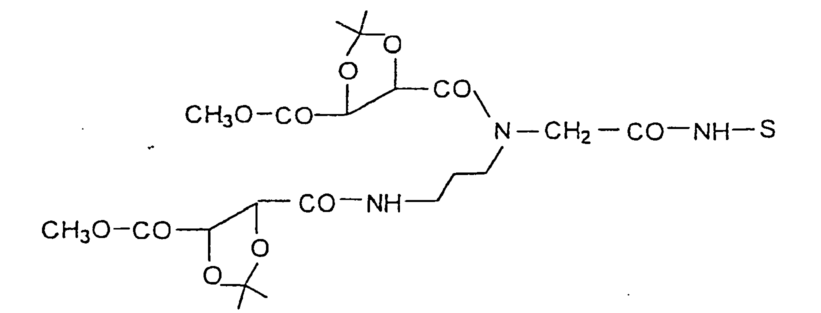

10. Procédé de préparation du support de formule I selon l'une quelconque des revendication précédentes, **caractérisé en ce qu'**il consiste à faire réagir :
- un support solide fonctionnalisé de formule S-(B)ₘ-Y, dans laquelle B, S et m sont tels que décrits dans l'une quelconque des revendications précédentes et Y représente un groupe nucléophile, B pouvant être substitué par au moins un autre groupement Y, identique ou différent de celui représenté dans la formule S-(B)ₘ-Y, en plus de celui représenté dans la formule S-(B)ₘ-Y, auquel cas ledit support solide est polyfonctionnalisé,
- avec un composé sélectionné dans le groupe constitué par les anhydrides cycliques de l'acide tartrique ou d'un de ses dérivés et les composés de formule II : 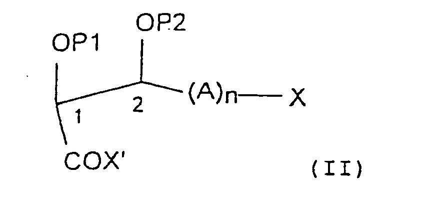
dans laquelle n, A, P1, P2 et X' sont tels que définis dans l'une quelconque des revendications précédentes et X représente un groupe électrophile.

11. Procédé de préparation du support de formule I selon l'une quelconque des revendications 1 à 9 **caractérisé en ce qu'**il consiste à faire réagir :
- un support solide fonctionnalisé de formule S-(B)ₘ-Y, dans laquelle B, S et m sont tels que décrits dans l'une quelconque des revendications précédentes et Y représente un groupe électrophile, B pouvant être substitué par au moins un autre groupement Y, identique ou différent de celui représenté dans la formule S-(B)ₘ-Y, en plus de celui représenté dans la formule S-(B)ₘ-Y, auquel cas ledit support solide est polyfonctionnalisé,
- avec un composé sélectionné dans le groupe constitué par les anhydrides cycliques de l'acide tartrique ou d'un de ses dérivés et les composés de formule II : 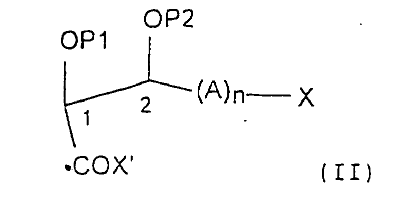
dans laquelle n = 1 et A, P1, P2 et X' sont tels que définis dans l'une quelconque des revendications précédentes et X représente un groupe nucléophile.

12. Procédé selon la revendication 10 ou la revendication 11, **caractérisé en ce qu'**un groupe électrophile convenable est sélectionné dans le groupe constitué par les groupements suivants :
- lorsque n = 1 : -CR₂R₃Cl, -CR₂R₃Br, -CR₂R₃I, -CR₂R₃OSO₂R₄, -CHO, -COOR₂, -COF, -COCl, -COBr, -SO₂OR₂, -SOOCl, ester succinimidylique, ester sulfosuccinimidylique, -COO⁻ transformé par un agent d'activation électrophile, -N=C=O (isocyanate), -N=C=S (isothiocyanate), -O-CO-OR₂, -O-CO-Im, -O-COCl, -NR₂-COCl et -NR₂-CO-Im,
où Im représente un groupe imidazolyle éventuellement substitué de formule : 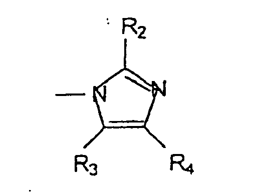 et où R₂, R₃ et R₄, qui peuvent être identiques ou différents, représentent des atomes d'hydrogène ou des groupes alkyles comprenant de 1 à 18 atomes de carbone, linéaires, ramifiés ou cycliques, saturés ou insaturés, ou bien des groupes aryles ou hétéroaryles, lesdits groupes alkyles, aryles et hétéroaryles étant éventuellement partiellement ou intégralement substitués par un ou plusieurs atomes d'halogènes, un ou plusieurs groupes amino, hydroxy, alcoxy, aryloxy, alkylthio ou arylthio ;
- lorsque n = 0 : -CHO, -COOR₂, -COF, -COCl, -COBr, -COO⁻ transformé par un agent d'activation électrophile, R₂ étant tel que décrit ci-dessus pour n = 1.

13. Procédé selon la revendication 10 ou la revendication 11, **caractérisé en ce qu'**un groupe nucléophile convenable est sélectionné dans le groupe constitué par les groupements suivants, lorsque n = 0 ou lorsque n = 1 :
-OH, -SH, -COO⁻, -NHR₅, -CONR₅NH₂, -N[R₅(CO)]NH₂, -N[R₅O(CO)]NH₂, -N[R₅NH(CO)]NH₂, -SO₂NH₂, -SO₂NR₅NH₂, -ONH₂, -(CO)ONH₂ et -C(NHR₅) -CR₅R₆-SH, R₅ et R₆ ayant la même signification que les groupes R₂ à R₄ définis dans la revendication 12.

14. Procédé selon la revendication 10 ou 11,
**caractérisé en ce que**, lorsque n = 0 et X = -COOR₂, avec R₂ = H, ledit groupement X = -COOR₂ peut jouer aussi bien le rôle de nucléophile que d'électrophile.

15. Procédé selon l'une quelconque des revendications 10 à 14, **caractérisé en ce que**, lorsque P1 et P2 dans le composé de formule II représentent des atomes d'hydrogène, des groupes P1 et P2 autres que des atomes d'hydrogène, à savoir des groupements protecteurs des fonctions hydroxyles en positions 1,2, peuvent éventuellement être introduits après la réaction du support solide de formule S-(B)ₘ-Y avec le composé de formule II et avant que le support de formule I selon l'une quelconque des revendications 1 à 9 ne soit utilisé pour la synthèse de composas comportant au moins une fonction α-oxoaldéhyde.

16. Procédé selon l'une quelconque des revendications 10 à 15, **caractérisé en ce que** le composé de formule II représente l'acide tartrique ou un dérivé de l'acide tartrique.

17. Procédé selon l'une quelconque des revendications 10 à 15, **caractérisé en ce que** le composé de formule II est sélectionné dans le groupe constitué par l'acide mucique, l'acide saccharique, l'acide glucarique, leurs sels et leurs dérivés.

18. Procédé selon l'une quelconque des revendications 10 à 16, **caractérisé en ce qu'**il consiste à faire réagir :
- un support solide fonctionnalise de formule :
H₂N-(CH₂)₄-CN(NH₂)-CO-NH-S,
dans laquelle S est tel que défini dans la revendication 10,
- avec un tartrate de diméthyl-2,3-O-isopropylidène.

19. Procédé selon l'une quelconque des revendications 10 à 16, **caractérisé en ce qu'**il consiste à faire réagir :
- un support solide fonctionnalisé de formule :
H₂N-(CH₂)₃-NH-CH₂-CO-NH-S,
dans laquelle S est tel que défini dans la revendication 10,
- avec un tartrate de diméthyl-2,3-O-isopropylidène.

20. Support de formule III suivante : 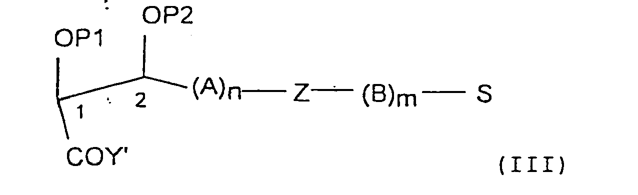 dans laquelle S, B, A, n, m, Z, P1 et P2 sont tels que définis dans l'une quelconque des revendications 1 à 9 et Y' représente un composé ou un enchaînement de plusieurs composés sélectionnés dans le groupe constitué par les lipides, les nucléotides, lesdits nucléotides étant substitués ou non, et les acides aminés, ces derniers comportant éventuellement des lipides.

21. Support de formule III selon la revendication-20, **caractérisé** én ce qu'il est obtenu à partir du support de formule I selon l'une quelconque des revendications 1 à 9 après modification chimique du groupement -COX' à l'aide d'un réactif approprié, qui apporte une fonction permettant l'élaboration sur le support du composé Y'.

22. Support selon la revendication 21, **caractérisé en ce que** ledit réactif approprié est une diamine, un dialcool ou un aminoalcool.

23. Procédé de synthèse de composés organiques comportant au moins une fonction α-oxoaldéhyde, **caractérisé en ce que** Ledit procédé s'effectue en phase solide et comprend les étapes suivantes :
- préparation d'un support de formule I tel que défini dans l'une quelconque des revendications 1 à 9, ladite préparation s'effectuant selon l'une quelconque des revendications 10 à 19. ;
- éventuellement, dérivatisation du groupe X' dudit support de formule I, de façon à introduire une fonction permettant l'élaboration sur le support du composé Y' tel que défini dans la revendication 20 ou la revendication 21 ;
- élaboration du composé Y' en phase solide ;
- si P1 et P2 sont différents d'un atome d'hydrogène, déprotection des deux fonctions hydroxyles en positions 1,2 protégées par P1 et P2 selon la formule III telle que décrite dans la revendication 20 ;
- oxydation périodique en phase solide ; et
- isolement du produit formé, fonctionnalisé par au moins une fonction α-oxoaldéhyde.

24. Procédé selon la revendication 23, **caractérisé en ce que** ladite dérivatisation du groupe X' du support de formule I consiste à greffer une diamine, un dialcool ou un aminoalcool.

25. Procédé selon la revendication 23 ou la revendication 24, **caractérisé en ce que** la déprotection des deux fonctions hydroxyles en positions 1,2 s'effectue en milieu acide, de préférence en présence d'acide trifluoroacétique.

26. Procédé selon la revendication 23 ou la revendication 24, **caractérisé en ce que** la déprotection des deux fonctions hydroxyles en positions 1,2 s'effectue en milieu basique.

27. Procédé selon l'une quelconque des revendications 23 à 26, **caractérisé en ce que** l'oxydation périodique s'effectue en présence de periodate de sodium.

28. Méthode de synthèse d'une banque de composés organiques, **caractérisée en ce qu'**elle comprend :
- la fourniture d'une pluralité de surfaces de support solide de formule S-(B)ₘ-Y, dans laquelle B, S, Y et m sont tels que décrits dans l'une quelconque des revendications 10 à 19 ;
- leur réaction avec un composé sélectionné dans le groupe constitué par les anhydrides cycliques de l'acide tartrique ou d'un de ses dérivés et les composés formule II 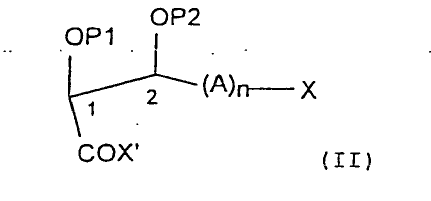
dans laquelle n, A, P1, P2, X' et X sont tels que définis dans l'une quelconque des revendications 10 à 19, de façon à obtenir un support utilisable pour la synthèse de composés comportant une fonction α-oxoaldéhyde ;
- la réaction du support obtenu avec des mélanges d'acides animés, de nucléotides ou de lipides ;
- et l'obtention ainsi d'une banque de composés organiques.

29. Banque de composés organiques, **caractérisée en ce qu'**elle est susceptible d'être obtenue par le procédé décrit dans la revendication 28.

30. Utilisation d'un composé sélectionné dans le groupe constitué par les anhydrides cycliques de l'acide tartrique ou d'un de ses dérivés et les composés de formule II 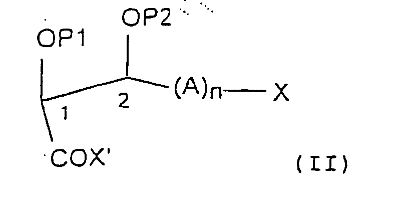 dans laquelle n, A, P1, P2 et X' et X sont tels que définis dans l'une quelconque des revendications 10 à 19, pour la dérivatisation d'un support solide dont au moins la surface a la propriété d'être bien solvatée en milieu aqueux ou partiellement aqueux, le support résultant pouvant être utilisé pour la synthèse, en phase solide, de composés comportant au moins une fonction α-oxoaldéhyde.

31. Utilisation selon la revendication 30 **caractérisée en ce que** ledit support solide dont au moins la surface a la propriété d'être bien solvatée en milieu aqueux ou partiellement aqueux répond à la formule S-(B)ₘ-Y, S, B, m et Y étant tels que définis dans l'une quelconque des revendications 10 à 19.

32. Utilisation selon la revendication 30 ou la revendication 31 **caractérisée en ce que** ledit anhydride cyclique de l'acide tartrique ou d'un de ses dérivés est l'anhydride di-O-benzoyl-tartrique ou l'anhydride diacétyl-tartrique.

33. Utilisation selon la revendication 30 ou la revendication 31 **caractérisée en ce que** le composé de formule II représente l'acide tartrique ou un dérivé de l'acide tartrique.

34. Utilisation selon la revendication 30 ou la revendication 31 **caractérisée en ce que** le composé de formule II est sélectionné dans le groupe constitué par l'acide mucique, l'acide saccharique, l'acide glucarique, leurs sels et leurs dérivés.

35. Procédé de préparation d'un réactif de diagnostic sur support solide, **caractérisé en ce qu'**il comprend les étapes suivantes :
- préparation d'un support de formule I tel que défini dans l'une quelconque des revendications 1 à 9, ladite préparation s'effectuant selon l'une quelconque des revendications 10 à 19 ;
- éventuellement, dérivatisation du groupe X' dudit support de formule I, de façon à introduire une fonction permettant l'élaboration sur le support du composé Y' tel que défini dans la revendication 20 ou la revendication 21 ;
- élaboration du composé Y' en phase solide ;
- si P1 et P2 sont différents d'un atome d'hydrogène, déprotection des deux fonctions hydroxyles en positions 1,2 protégées par P1 et P2 selon la formule III telle que décrite dans la revendication 20 ;
- oxydation périodique en phase solide ;
- isolement du produit formé, fonctionnalisé par au moins une fonction α-oxoaldéhyde ;
- ligation dudit produit formé comportant au moins une fonction α-oxoaldehyde, à une plaque de microtitration convenablement fonctionnalisée,
- obtention d'une plaque de microtitration sur laquelle sont fixés; de façon covalente, lesdits produits.

36. Procédé selon la revendication 35, **caractérisé en ce que** lesdits produits sont des peptides, des oligonucléotides ou des lipides.

37. Procédé selon la revendication 35 ou la revendication 36 **caractérisé en ce que** ladite ligation desdits produits s'effectue par liaison oxime, oxazolidine ou hydrazone.

38. Procédé d'obtention d'une biopucë, **caractérisé en ce qu'**il comprend les étapes suivantes :
- préparation d'un support de formule I tel que défini dans l'une quelconque des revendications 1 à 9, ladite préparation s'effectuant selon l'une quelconque des revendications 10 à 19 ;
- éventuellement, dérivatisation du groupe X' dudit support de formule I, de façon à introduire une fonction permettant l'élaboration sur le support du composé Y', nucléotide ou enchaînement de nucléotides tel que défini dans la revendication 20 ou la revendication 21 ;
- élaboration d'un oligonucléotide en phase solide ;
- si P1 et P2 sont différents d'un atome d'hydrogène, déprotection des deux fonctions hydroxyles en positions 1, 2 protégées par P1 et P2 selon la formule III telle que décrite dans la revendication 20 ;
- oxydation périodique en phase solide ;
- isolement des oligonucléotides formés, fonctionnalisés par au moins une fonction α-oxoaldéhyde ;
- greffage desdits oligonucléotides à un support solide.

39. Procédé selon la revendication 38 , **caractérisé en ce que** ledit support solide est une surface en verre ou en silicium.

40. Procédé selon la revendication 38 ou la revendication 39, **caractérisé en ce que** ladite biopuce est une puce à ADN.

## Claims

1. A functionalized solid support for the synthesis of compounds containing at least one α-oxoaldehyde function, which support is **characterized in that** it corresponds to formula I below: 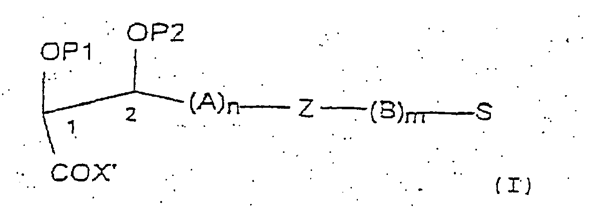 in which:
- S represents a solid support, at least the surface of which has the property of being well solvated in an aqueous or partially aqueous medium;
- n and m, which may be identical or different, represent 0 or 1;
- Z represents a group selected from the group consisting of ether, thioether, ester, amine, amide, sulfonamide, hydroxylamine, hydrazine, hydrazone, thiazolidine, oxime, carbonate, carbamate, thiocarbamate, urea and the thiourea groups, and derivatives thereof, when n is equal to 1;
- Z represents a group selected from the group consisting of ester, amide, N-acylhydroxylamine, hydrazide, hydrazone, oxime and thiazolidine groups, and derivatives thereof, when n is equal to 0;
- P1 and P2, which may be identical or different or may together form a ring with the oxygen atoms to which they are attached, represent hydrogen atoms or protective groups for the hydroxyl functions in the 1-, 2-positions, P1 and P2 being, in this case, selected from the group consisting of the groups -R₁, -(CO)R₁, - (CO)OR₁, -(SO₂)OR₁, -SiR' R'' R''', acetals and cetals, R₁, R' , R'' and R''' representing saturated or unsaturated, linear, branched or cyclic alkyl groups containing from 1 to 18 carbon atoms, or else aryl or heteroaryl groups, said alkyl, aryl or heteroaryl groups being optionally partially or completely substituted with one or more halogen atoms, or one or more amino, hydroxyl, alkoxy, aryloxy, alkylthio or arylthio groups;
- A and B, which may be identical or different, represent a saturated or unsaturated, linear, branched or cyclic carbon-based chain containing from 1 to 18 carbon atoms and, optionally, from 1 to 7 groups selected from the group consisting of carbonyl groups, carbon-based rings, heterocycles, aryls and hetero-aryls, A and/or B optionally also containing from 1 to 16 heteroatoms, preferably from 1 to 16 nitrogen or oxygen atoms, and possibly being substituted with 1 to 16 hydroxyl or amino groups which are optionally suitably protected,
at least one of the carbons of A and/or of B being optionally substituted with a group: 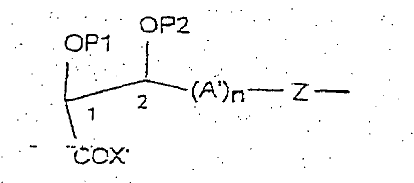 in which A' is identical to or different from A; in the latter case, A' is selected from the other groups A defined above; and
- X' is selected from the group consisting of the groups -OR₁, -NR'R" and 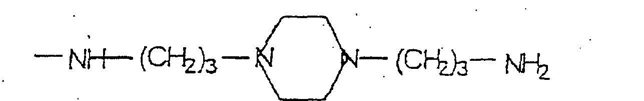 R₁, R' and R'' representing a hydrogen atom or being as defined above.

2. A support according to claim 1, **characterized in that** said solid support S is selected from the group consisting of:
- porous borosilicate glass particles,
- glass or silicon surfaces,
- resins selected from the group consisting of polyacrylamide resins, polyacrylamide/polyethylene glycol resins, polyethylene glycol/polystyrene resins, crosslinked ethoxylate/acrylate resins and resins of the type polyethylene, polystyrene or polypropylene grafted with hydrophilic molecules.

3. A support of formula I according to claim 1 or claim 2, **characterized in that**:
- S is a resin of the polyacrylamide/polyethylene glycol type,
- m and n represent 1,
- B is a -(CH₂)₂-CO-NH chain,
- Z is an amide group,
- A is a -CO-NH-(CH₂)₃- chain,
- P1 and P2 together form an isopropylidene protective group, and
- X' represents an -NH-(CH₂)₃-NH₂ chain.

4. A support of formula I according to claim 1 or claim 2, **characterized in that**:
- S is a resin of the polyacrylamide/polyethylene glycol or polyethylene glycol/polystyrene type,
- m and n represent 0,
- Z is an amide group,
- P1 and P2 together form an isopropylidene protective group, and
- X' represents a methoxy group.

5. A support of formula I according to claim 1 or claim 2, **characterized in that**:
- S is as defined in claim 1 or claim 2,
- m represents 1,
- n represents 0,
- Z is an amide group,
- P1 and P2 together form an isopropylidene protective group,
- X' represents a methoxy group, and
- B represents the following group:

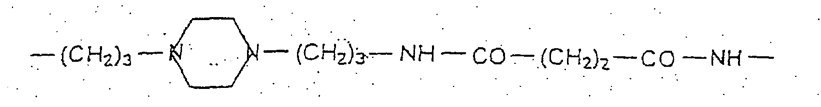

6. A support of formula I according to claim 1 or claim 2, **characterized in that**:
- S is as defined in claim 1 or claim 2,
- m and n represent 1,
- Z is an amide group,
- P1 and P2 together form an isopropylidene protective group,
- B is a group -(CH₂)₂-CO-NH-,
- A represents the following group: 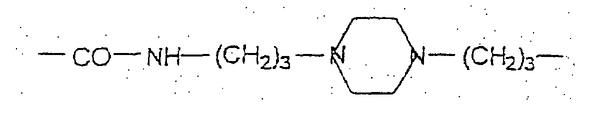 and
- x' represents the following group:

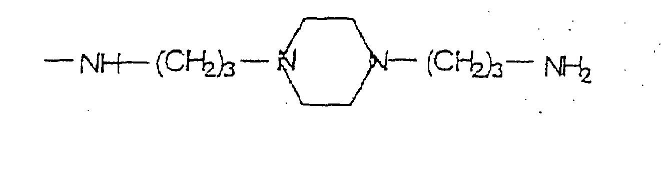

7. A support of formula I according to claim 1 or claim 2, **characterized in that**:
- S is as defined in claim 1 or claim 2,
- m represents 0,
- n represents 1,
- Z is a carbamate group,
- P1 and P2 together form an isopropylidene protective group,
- X' represents a methoxy group, and
- A represents the following group:

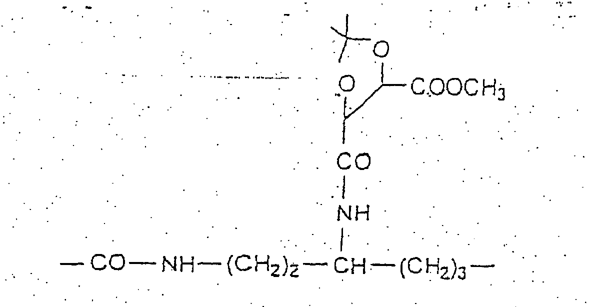

8. A support of formula I according to claim 1 or claim 2, **characterized in that** it corresponds to the following formula:

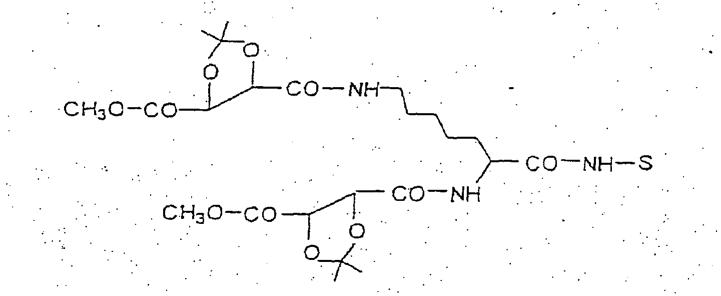

9. A support of formula I according to claim 1 or claim 2, **characterized in that** it corresponds to the following formula:

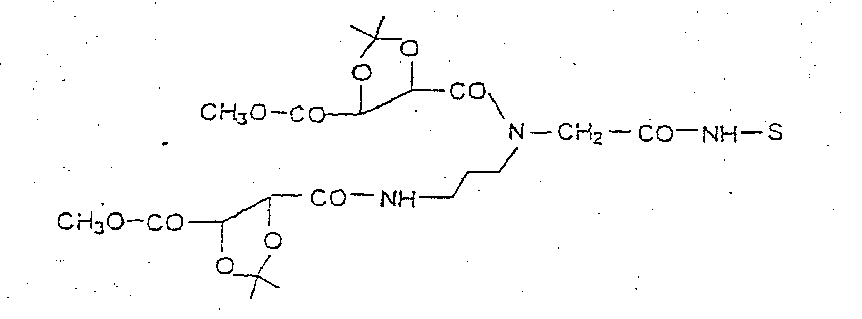

10. A process for preparing the support of formula I according to any one of the preceding claims, **characterized in that** it consists in reacting:
- a functionalized solid support of formula S-(B)ₘ-Y, in which B, S and m are as described in any one of the preceding claims and Y represents a nucleophilic group, it being possible for B to be substituted with at least one other group Y, which is identical to or different from that represented in the formula S-(B)ₘ-Y, in addition to that represented in the formula S-(B)ₘ-Y, in which case said solid support is polyfunctionalized,
- with a compound selected from the group consisting of the cyclic anhydrides of tartaric acid or one of its derivatives and the compounds of formula II: 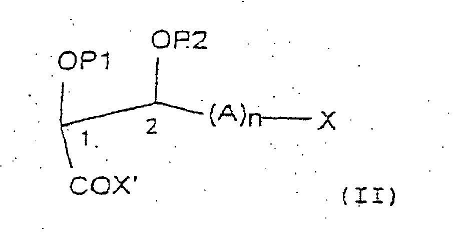
in which n, A, P1, P2 and X' are as defined in any one of the preceding claims, and X represents an electrophilic group.

11. A process for preparing the support of formula I according to any one of claims 1 to 9, **characterized in that** it consists in reacting:
- a functionalized solid support of formula S-(B)ₘ-Y, in which B, S and m are as described in any one of the preceding claims and Y represents an electrophilic group, it being possible for B to be substituted with at least one other group Y, which may be identical to or different from that represented in the formula S-(B)ₘ-Y, in addition to that represented in the formula S-(B)ₘ-Y, in which case said solid support is polyfunctionalized,
- with a compound selected from the group consisting of the cyclic anhydrides of tartaric acid or one of its derivatives and the compounds of formula II: 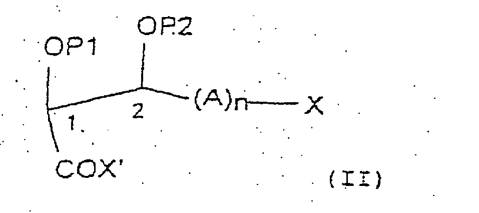
in which n = 1 and A, P1, P2 and X' are as defined in any one of the preceding claims and X represents a nucleophilic group.

12. A process according to claim 10 or claim 11, **characterized in that** a suitable electrophilic group is selected from the group consisting of the following groups:
- when n = 1: -CR₂R₃Cl, -CR₂R₃Br, -CR₂R₃I, -CR₂R₃OSO₂R₄, -CHO, -COOR₂, -COF, -COCl, -COBr, -SO₂OR₂, -SOOC1, succinimidyl ester, sulfosuccinimidyl ester, -COO⁻ converted with an electrophilic activation agent, -N=C=O (isocyanate), -N=C=S (isothiocyanate), -O-CO-OR₂, -O-CO-Im, -O-COCl, -NR₂-COCl and -NR₂-CO-Im,
where Im represents an optionally substituted imidazolyl group of formula: 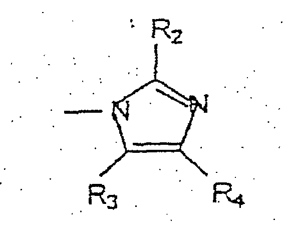 and where R₂, R₃ and R₄, which may be identical or different, represent hydrogen atoms or saturated or unsaturated, linear, branched or cyclic alkyl groups containing from 1 to 18 carbon atoms, or else aryl or heteroaryl groups, said alkyl, aryl and heteroaryl groups being optionally partially or completely substituted with one or more halogen atoms, or one or more amino, hydroxyl, alkoxy, aryloxy, alkylthio or arylthio groups;
- when n = 0: -CHO, -COOR₂, -COF, -COCl, -COBr and -COO⁻ converted with an electrophilic activation agent, R₂ being as described above for n = 1.

13. A process according to claim 10 or claim 11, **characterized in that** a suitable nucleophilic group is selected from the group consisting of the following groups, when n = 0 or when n = 1:
-OH, -SH, -COO⁻, -NHR₅, -CONR₅NH₂, -N[R₅(CO)]NH₂, -N[R₅O(CO)]NH₂, -N (R₅NH (CO)]NH₂, -SO₂NH₂, -SO₂NR₅NH₂, -ONH₂, -(CO)ONH₂ and -C(NHR₅)-CR₅R₆-SH, R₅ and R₆ having the same meaning as the groups R₂ to R₄ defined in claim 12.

14. A process according to claim 10 or 11, **characterized in that**, when n = 0 and X = -COOR₂, with R₂ = H, said group X = -COOR₂ can play the role of both a nucleophile and an electrophile.

15. A process according to any one of claims 10 to 14, **characterized in that**, when P1 and P2 in the compound of formula II represent hydrogen atoms, groups P1 and P2 other than hydrogen atoms, i.e. protective groups for the hydroxyl functions in the 1-, 2-positions, can optionally be introduced after the reaction of the solid support of formula S-(B)ₘ-Y with the compound of formula II and before the support of formula I according to any one of claims 1 to 9 is used for the synthesis of compounds containing at least one α-oxo-aldehyde function.

16. A process according to any one of claims 10 to 15, **characterized in that** the compound of formula II represents tartaric acid or a tartaric acid derivative.

17. A process according to any one of claims 10 to 15, **characterized in that** the compound of formula II is selected from the group consisting of mucic acid, saccharic acid and glucaric acid, salts thereof and derivatives thereof.

18. A process according to any one of claims 10 to 16, **characterized in that** it consists in reacting:
- a functionalized solid support of formula:
H₂N- (CH₂)₄-CH(NH₂) -CO-NH-S,
in which S is as defined in claim 10,
- with dimethyl-2,3-0-isopropylidene tartrate.

19. A process according to any one of claims 10 to 16, **characterized in that** it consists in reacting:
- a functionalized solid support of formula:
H₂N-(CH₂)₃-NH-CH₂-CO-NH-S,
in which S is as defined in claim 10,
- with dimethyl-2,3-O-isopropylidene tartrate.

20. A support of formula III below: 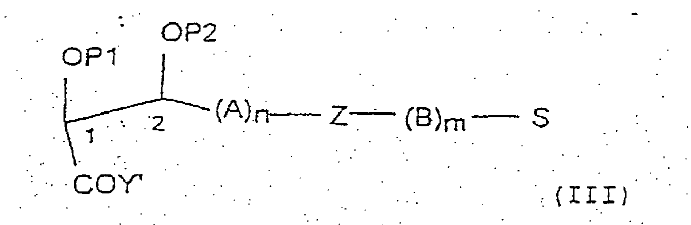 in which S, B, A, n, m, Z, P1 and P2 are as defined in any one of claims 1 to 9 and Y' represents a compound or a series of several compounds selected from the group consisting of lipids, nucleotides, said nucleotides being substituted or unsubstituted, and amino acids, the latter optionally comprising lipids.

21. A support of formula III according to claim 20, **characterized in that** it is obtained from the support of formula I according to any one of claims 1 to 9, after chemical modification of the group -COX' using an appropriate reagent, which provides a function that makes it possible to produce the compound Y' on the support.

22. A support according to claim 21, **characterized in that** said suitable reagent is a diamine, a dialcohol or an aminoalcohol.

23. A process for synthesizing organic compounds containing at least one α-oxoaldehyde function, **characterized in that** said process is carried out in solid phase and comprises the following steps:
- preparation of a support of formula I as defined in any one of claims 1 to 9, said preparation being carried out according to any one of claims 10 to 19;
- optionally, derivatization of the group X' of said support of formula I, so as to introduce a function that allows the production on the support of the compound Y' as defined in claim 20 or claim 21;
- solid-phase production of the compound Y';
- if P1 and P2 are different from a hydrogen atom, deprotection of the two hydroxyl functions in the 1-, 2-positions protected by P1 and P2 according to formula III as described in claim 20;
- solid-phase periodic oxidation; and
- isolation of the product formed, functionalized with at least one α-oxoaldehyde function.

24. A process according to claim 23, **characterized in that** said derivatization of the group X' of the support of formula I consists in grafting a diamine, a dialcohol or an amino alcohol.

25. A process according to claim 23 or 24, **characterized in that** the deprotection of the two hydroxyl functions in the 1-, 2-positions is carried out in an acidic medium, preferably in the presence of trifluoroacetic acid.

26. A process according to claim 23 or claim 24, **characterized in that** the deprotection of the two hydroxyl functions in the 1-, 2-positions is carried out in a basic medium.

27. A process according to any one of claims 23 to 26, **characterized in that** the periodic oxidation is carried out in the presence of sodium periodate.

28. A method for synthesizing a library of organic compounds, **characterized in that** it comprises:
- the provision of a plurality of surfaces of a solid support of formula S-(B)ₘ-Y, in which B, S, Y and m are as described in any one of claims 10 to 19;
- reaction thereof with a compound selected from the group consisting of the cyclic anhydrides of tartaric acid or of one of its derivatives and the compounds of formula II 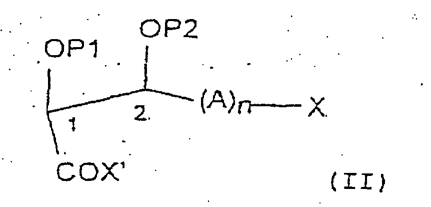
in which n, A, P1, P2, X' and X are as defined in any one of claims 10 to 19, so as to obtain a support that can be used for the synthesis of compounds containing an α-oxoaldehyde function;
- reaction of the support obtained with mixtures of amino acids, of nucleotides or of lipids;
- and thus the production of a library of organic compounds.

29. A library of organic compounds, **characterized in that** it can be obtained by means of the process described in claim 28.

30. Use of a compound selected from the group consisting of the cyclic anhydrides of tartaric acid or of one of its derivatives and the compounds of formula II 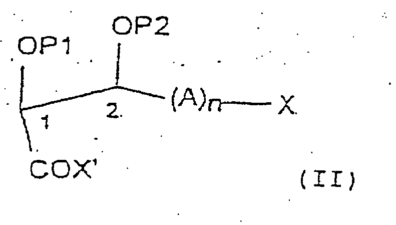 in which n, A, P1, P2, X' and X are as defined in any one of claims 10 to 19, for the derivatization of a solid support, at least the surface of which has the property of being well solvated in an aqueous or partially aqueous medium, it being possible for the resulting support to be used for the solid-phase synthesis of compounds containing at least one α-oxoaldehyde function.

31. Use according to claim 30, **characterized in that** said solid support, at least the surface of which has the property of being well solvated in an aqueous or partially aqueous medium, corresponds to the formula S-(B)ₘ-Y, S, B, m and Y being as defined in any one of claims 10 to 19.

32. Use according to claim 30 or claim 31, **characterized in that** said cyclic anhydride of tartaric acid or of one of its derivatives is di-O-benzoyltartaric anhydride or diacetyltartaric anhydride.

33. Use according to claim 30 or claim 31, **characterized in that** the compound of formula II represents tartaric acid or a tartaric acid derivative.

34. Use according to claim 30 or claim 31, **characterized in that** the compound of formula II is selected from the group consisting of mucic acid, saccharic acid and glucaric acid, salts thereof and derivatives thereof.

35. A process for preparing a diagnostic reagent on a solid support, **characterized in that** it comprises the following steps:
- preparation of a support of formula I as defined in any one of claims 1 to 9, said preparation being carried out according to any one of claims 10 to 19;
- optionally, derivatization of the group X' of said support of formula I, so as to introduce a function that allows the production on the support of the compound Y' as defined in claim 20 or claim 21;
- solid-phase production of the compound Y';
- if P1 and P2 are different from a hydrogen atom, deprotection of the two hydroxyl functions in the 1-, 2-positions protected by P1 according to P2 according to formula III as described in claim 20;
- solid-phase periodic oxidation;
- isolation of the product formed, functionalized with at least one α-oxoaldehyde function;
- ligation of said product formed containing at least one α-oxoaldehyde function, to a suitably functionalized microtitration plate;
- production of a microtitration plate on which said products are covalently attached.

36. A process according to claim 35, **characterized in that** said products are peptides, oligonucleotides or lipids.

37. A process according to claim 35 or claim 36, **characterized in that** said ligation of said products is carried out via an oxime, oxazolidine or hydrazone bond.

38. A process for obtaining a biochip, **characterized in that** it comprises the following steps:
- preparation of a support of formula I as defined in any one of claims 1 to 9, said preparation being carried out according to any one of claims 10 to 19;
- optionally, derivatization of the group X' of said support of formula I, so as to introduce a function that allows the production on the support of the compound Y', a nucleotide or series of nucleotides as defined in claim 20 or claim 21;
- solid-phase production of an oligonucleotide;
- if P1 and P2 are different from a hydrogen atom, deprotection of the two hydroxyl functions in the 1-, 2-positions protected by P1 and P2 according to formula III as described in claim 20;
- solid-phase periodic oxidation;
- isolation of the oligonucleotides formed, functionalized with at least one α-oxoaldehyde function;
- grafting of said oligonucleotides to a solid support.

39. A process according to claim 38, **characterized in that** said solid support is a glass or silicon surface.

40. A process according to claim 38 or claim 39, **characterized in that** said biochip is a DNA chip.

## Patentansprüche

1. Funktionalisierter fester Träger für die Synthese von Verbindungen, die mindestens eine α-Oxoaldehyd-Funktion aufweisen, wobei der Träger **dadurch gekennzeichnet ist, dass** er der folgenden Formel (I) entspricht: 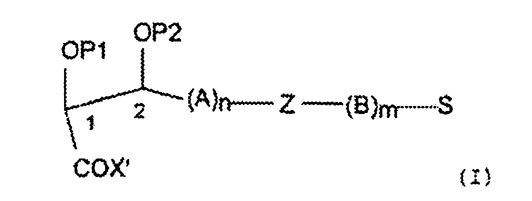 worin:
- S steht für einen festen Träger, bei dem mindestens die Oberfläche die Eigenschaft hat, in einem wässrigen Medium oder in einem partiell wässrigen Medium gut solvatisiert zu sein;
- n und m, die gleich oder verschieden sein können, für die Zahl 0 oder 1 stehen;
- Z steht für eine Gruppe, ausgewählt aus der Gruppe, die besteht aus Ether-, Thioether-, Ester-, Amin-, Amid-, Sulfonamid-, Hydroxylamin-, Hydrazin-, Hydrazon-, Thiazolidin-, Oxim-, Carbonat-, Carbamat-, Thiocarbamat-, Harnstoff-, Thioharnstoff-Gruppen und ihren Derivaten, wenn n für die Zahl 1 steht; oder
- Z steht für eine Gruppe, ausgewählt aus der Gruppe, die besteht aus Ester-, Amid-, N-Acylhydroxylamin-, Hydrazid-, Hydrazon-, Oxim-, Thiazolidin-Gruppen und ihren Derivaten, wenn n für die Zahl 0 steht;
- P1 und P2, die gleich oder verschieden sein können oder gemeinsam zusammen mit den Sauerstoffatomen, an die sie gebunden sind, einen Ring bilden können, Wasserstoffatome oder Schutzgruppen für Hydroxyl-Funktionen in den Positionen 1, 2, darstellen, wobei P1 und P2 in diesem Fall ausgewählt sind aus der Gruppe, die besteht aus -R₁-, -(CO)R₁-, -(CO)OR₁-, -(SO₂)OR₁-, -SiR'R"R"'-, Acetal- und Ketal-Gruppen, worin R₁, R', R" und R''' gesättigte oder ungesättigte, lineare, verzweigte oder cyclische Alkylgruppen mit 1 bis 18 Kohlenstoffatomen darstellen, oder Arylgruppen oder Heteroarylgruppen darstellen, wobei die genannten Alkyl-, Aryl- oder Heteroaryl-Gruppen gegebenenfalls teilweise oder vollständig substituiert sind durch ein oder mehr Halogenatome, eine oder mehr Amino-, Hydroxy-, Alkoxy-, Aryloxy-, Alkylthio- oder Arylthio-Gruppen;
- A und B, die gleich oder verschieden sein können, stehen für eine gesättigte oder ungesättigte lineare, verzweigte oder cyclische Kohlenstoffkette, die 1 bis 18 Kohlenstoffatome und gegebenenfalls 1 bis 7 Gruppen, ausgewählt aus der Gruppe, die besteht aus Carbonyl-, Kohlenstoffring-, Heterocyclen-, Aryl- und Heteroaryl-Gruppen aufweisen, wobei A und/oder B gegebenenfalls außerdem 1 bis 16 Heteroatome, vorzugsweise 1 bis 16 Stickstoff- oder Sauerstoffatome, aufweisen und durch 1 bis 16 Hydroxylgruppen oder gegebenenfalls in geeigneter Weise geschützte Aminogruppen substituiert sein können,
wobei mindestens eines der Kohlenstoffatome von A und/oder B gegebenenfalls substituiert ist durch eine Gruppe: 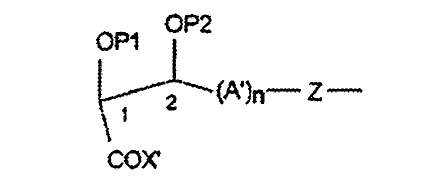 in der A' mit A identisch oder von A verschieden ist, wobei im letztgenannten Fall A' ausgewählt ist aus den oben genannten anderen Gruppen A; und
- X' ausgewählt ist aus der Gruppe, die besteht aus -OR₁-, -NR'R"-Gruppen und 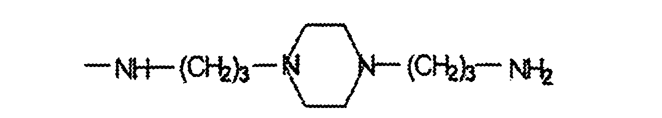
worin R₁, R' und R" für ein Wasserstoffatom stehen oder wie oben definiert sind.

2. Träger nach Anspruch 1, **dadurch gekennzeichnet, dass** der feste Träger S ausgewählt ist aus der Gruppe, die besteht aus:
- Teilchen aus porösem Borsilicatglas,
- Oberflächen aus Glas oder Silicium,
- Harzen, ausgewählt aus der Gruppe, die besteht aus Polyacrylamid-, Polyacrylamid-Polyethylenglycol-, Polyethylenglycol-Polystyrol-, vernetzten Ethoxylat-Acrylat-Harzen und Harzen vom Polyethylen-, Polystyrol- oder Polypropylen-Typ, auf die hydrophile Moleküle aufgepfropft sind.

3. Träger der Formel (I) nach Anspruch 1 oder 2, **dadurch gekennzeichnet, dass**
- S steht für ein Harz vom Polyacrylamid-Polyethylenglycol-Typ,
- m und n stehen für die Zahl 1,
- B steht für eine -(CH₂)₂-CO-NH-Kette,
- Z steht für eine Amidgruppe,
- A steht für eine -CO-NH-(CH₂)₃-Kette,
- P1 und P2 gemeinsam eine Isopropyliden-Schutzgruppe bilden und
- X' steht für eine -NH-(CH₂)₃-NH₂-Kette.

4. Träger der Formel (I) nach Anspruch 1 oder 2, **dadurch gekennzeichnet, dass**
- S steht für ein Harz vom Polyacrylamid-Polyethylenglycol- oder Polyethylenglycol-Polystyrol-Typ,
- m und n stehen für die Zahl 0,
- Z steht für eine Amidgruppe,
- P1 und P2 gemeinsam eine Isopropyliden-Schutzgruppe bilden und
- X' steht für eine Methoxygruppe.

5. Träger der Formel (I) nach Anspruch 1 oder 2, **dadurch gekennzeichnet, dass**
- S wie in Anspruch 1 oder in Anspruch 2 definiert ist,
- m steht für die Zahl 1,
- n steht für die Zahl 0,
- Z steht für eine Amidgruppe,
- P1 und P2 gemeinsam eine lsopropyliden-Schutzgruppe bilden,
- X' steht für eine Methoxygruppe und
- B steht für die folgende Gruppe:

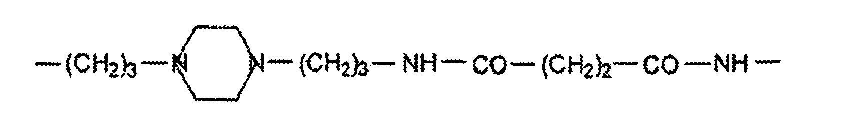

6. Träger der Formel (I) nach Anspruch 1 oder 2, **dadurch gekennzeichnet, dass**
- S wie in Anspruch 1 oder in Anspruch 2 definiert ist,
- m und n stehen für die Zahl 1,
- Z steht für eine Amidgruppe,
- P1 und P2 gemeinsam eine Isopropyliden-Schutzgruppe bilden,
- B steht für eine -(CH₂)₂-CO-NH-Gruppe,
- A steht für die folgende Gruppe: 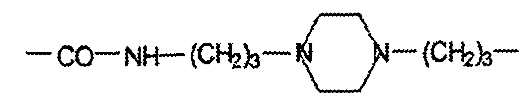 und
- X' steht für die folgende Gruppe

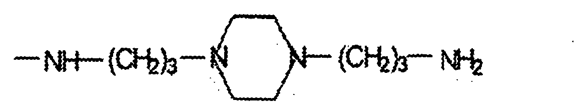

7. Träger der Formel (I) nach Anspruch 1 oder 2, **dadurch gekennzeichnet, dass**
- S wie in Anspruch 1 oder in Anspruch 2 definiert ist,
- m steht für die Zahl 0,
- n steht für die Zahl 1,
- Z steht für eine Carbamat-Gruppe,
- P1 und P2 gemeinsam eine lsopropyliden-Schutzgruppe bilden,
- X' steht für eine Methoxygruppe und
- A steht für die folgende Gruppe:

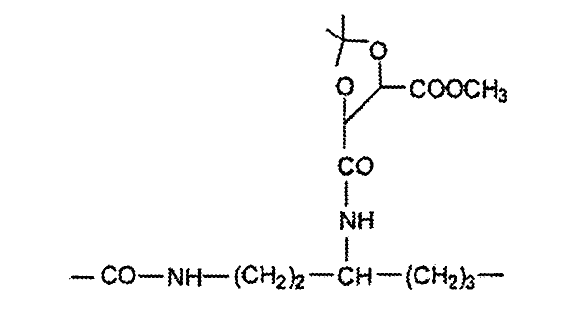

8. Träger der Formel (I) nach Anspruch 1 oder 2, **dadurch gekennzeichnet, dass** er der folgenden Formel entspricht:

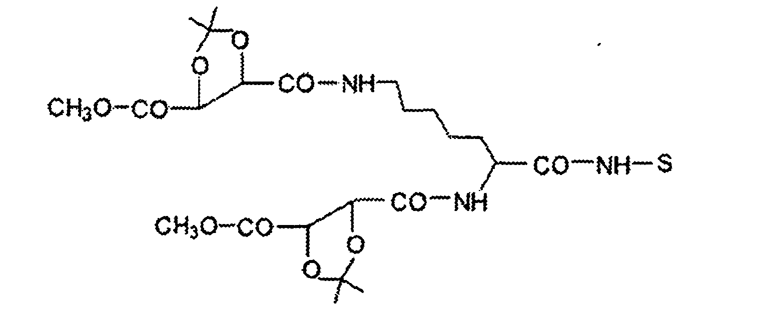

9. Träger der Formel (I) nach Anspruch 1 oder 2, **dadurch gekennzeichnet, dass** er der folgenden Formel entspricht:

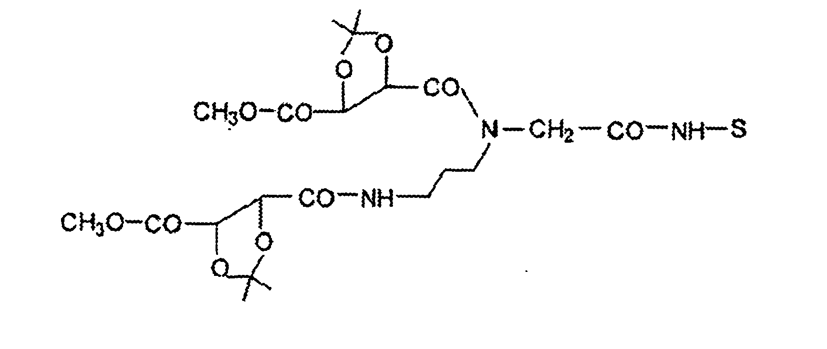

10. Verfahren zur Herstellung des Trägers der Formel (I) nach einem der vorhergehenden Ansprüche, **dadurch gekennzeichnet, dass** es darin besteht, dass man
- einen funktionalisierten festen Träger der Formel S-(B)ₘ-Y, in der B, S und m die Bedeutungen haben, wie sie in einem der vorhergehenden Ansprüche angegeben sind, und Y steht für eine nucleophile Gruppe, wobei B durch mindestens eine andere Gruppe Y substituiert sein kann, die gleich oder verschieden ist von derjenigen, wie sie für die Formel S-(B)ₘ-Y angegeben ist, zusätzlich zu derjenigen, wie sie für die Formel S-(B)ₘ-Y angegeben ist, wobei in diesem Fall der genannte feste Träger polyfunktionalisiert ist,
- reagieren lässt mit einer Verbindung, ausgewählt aus der Gruppe, die besteht aus cyclischen Anhydriden der Weinsäure oder einem ihrer Derivate und den Verbindungen der Formel (II): 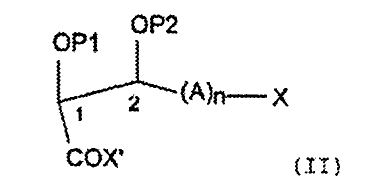
in der n, A, P1, P2 und X' wie in einem der vorhergehenden Ansprüche definiert sind und X steht für eine elektrophile Gruppe.

11. Verfahren zur Herstellung des Trägers der Formel (I) nach einem der Ansprüche 1 bis 9, **dadurch gekennzeichnet, dass** es darin besteht, dass man
- einen funktionalisierten festen Träger der Formel S-(B)ₘ-Y, in dem B, S und m die in einem der vorhergehenden Ansprüche angegebenen Bedeutungen haben und Y eine elektrophile Gruppe darstellt, wobei B durch mindestens eine andere Gruppe Y substituiert sein kann, die gleich oder verschieden ist von derjenigen, wie sie für die Formel S-(B)ₘ-Y angegeben ist, zusätzlich zu derjenigen, wie sie für die Formel S-(B)ₘ-Y angegeben ist, wobei in diesem Fall der feste Träger polyfunktionalisiert ist,
- reagieren lässt mit einer Verbindung, ausgewählt aus der Gruppe, die besteht aus cyclischen Anhydriden der Weinsäure oder einem ihrer Derivate und den Verbindungen der Formel (II): 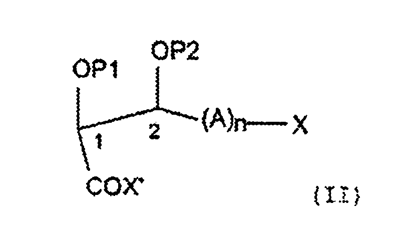 in der n = 1 und A, P1, P2 und X' wie in einem der vorhergehenden Ansprüche definiert sind und X für eine nucleophile Gruppe steht.

12. Verfahren nach Anspruch 10 oder 11, **dadurch gekennzeichnet, dass** eine geeignete elektrophile Gruppe ausgewählt ist aus der Gruppe, die besteht aus den folgenden Gruppen:
- wenn
n = 1 : -CR₂R₃Cl, -CR₂R₃Br, -CR₂R₃I,
-CR₂R₃OSO₂R₄, -CHO, -COOR₂, -COF, -COCl, -COBr, -SO₂OR₂, -SOOCl, einem Succinimidylester, einem Sulfosuccinimidylester, -COO⁻, das durch ein elektrophiles Aktivierungsmittel umgewandelt worden ist, -N=C=O (Isocyanat), -N=C=S (Isothiocyanat), -O-CO-OR₂, -O-CO-Im, -O-COCI, -NR₂-COCl und -NR₂-CO-Im, worin Im eine gegebenenfalls substituierte Imidazolyl-Gruppe der Formel darstellt: 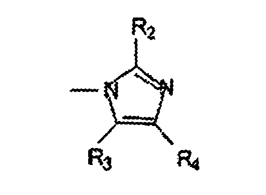 und worin R₂, R₃ und R₄, die gleich oder verschieden sein können, Wasserstoffatome oder gesättigte oder ungesättigte lineare, verzweigte oder cyclische Alkylgruppen mit 1 bis 18 Kohlenstoffatomen oder Aryl- oder Heteroaryl-Gruppen darstellen, wobei die genannten Alkyl-, Aryl- und Heteroaryl-Gruppen gegebenenfalls teilweise oder vollständig substituiert sind durch ein oder mehr Halogenatome, eine oder mehr
Amino-, Hydroxy-, Alkoxy-, Aryloxy-, Alkylthio- oder Arylthio-Gruppen;
- wenn n = 0: -CHO, -COOR₂, -COF, -COCl, -COBr, COO⁻, das durch ein elektrophiles Aktivierungsmittel umgewandelt worden ist, wobei R₂ so definiert ist, wie es oben für n = 1 angegeben ist.

13. Verfahren nach Anspruch 10 oder 11, **dadurch gekennzeichnet, dass** eine geeignete elektrophile Gruppe ausgewählt wird aus der Gruppe, die besteht aus den folgenden Gruppen, wenn n = 0 oder n = 1;
-OH, -SH, -COO⁻, -NHR₅, -CONR₃NH₂, -N[R₅(CO)]NH₂, -N[R₅O(CO)]NH₂, -N[R₅NH(CO)]NH₂, -SO₂NH₂, -SO₂NR₅NH₂, -ONH₂, -(CO)ONH₂ und -C(NHR₅)-CR₅R₆-SH,
worin R₅ und R₆ die gleiche Bedeutung haben wie die Gruppen R₂ bis R₄, die in Anspruch 12 definiert sind.

14. Verfahren nach Anspruch 10 oder 11, **dadurch gekennzeichnet, dass** dann, wenn n = 0 und X = -COOR₂, wobei R₂ = H, die genannte Gruppe X = -COOR₂ sowohl die Rolle eines Nucleophils als auch eines Elektrophils spielen kann.

15. Verfahren nach einem der Ansprüche 10 bis 14, **dadurch gekennzeichnet, dass** dann, wenn P1 und P2 in der Verbindung der Formel (II) Wasserstoffatome darstellen, Gruppen P1 und P2, die von Wasserstoffatomen verschieden sind, d.h. Schutzgruppen für Hydroxyl-Funktionen gegebenenfalls in die Positionen 1 und 2 eingeführt werden können nach der Reaktion des festen Trägers der Formel S-(B)ₘ-Y mit der Verbindung der Formel (II) und bevor der Träger der Formel (I) nach einem der Ansprüche 1 bis 9 verwendet wird für die Synthese von Verbindungen, die mindestens eine α-Oxoaldehyd-Funktion aufweisen.

16. Verfahren nach einem der Ansprüche 10 bis 15, **dadurch gekennzeichnet, dass** die Verbindung der Formel (II) Weinsäure oder ein Derivat der Weinsäure darstellt.

17. Verfahren nach einem der Ansprüche 10 bis 15, **dadurch gekennzeichnet, dass** die Verbindung der Formel (II) ausgewählt wird aus der Gruppe, die besteht aus Mucinsäure, Zuckersäure, Glucarsäure, deren Salzen und Derivaten.

18. Verfahren nach einem der Ansprüche 10 bis 16, **dadurch gekennzeichnet, dass** es darin besteht, dass man
- einen funktionalisierten festen Träger der Formel:
H₂N-(CH₂)₄-CH(NH₂)-CO-NH-S.
in der S wie in Anspruch 10 definiert ist,
- mit einem Dimethyl-2,3-O-isopropyliden-tartrat reagieren lässt.

19. Verfahren nach einem der Ansprüche 10 bis 16, **dadurch gekennzeichnet, dass** es darin besteht, dass man
- einen funktionalisierten festen Träger der Formel:
H₂N- (CH₂)₃-NH-CH₃-CO-NH-S,
in der S wie in Anspruch 10 definiert ist,
- mit einem Dimethyl-2,3-O-isopropyliden-tartrat reagieren lässt.

20. Träger der folgenden Formel (III): 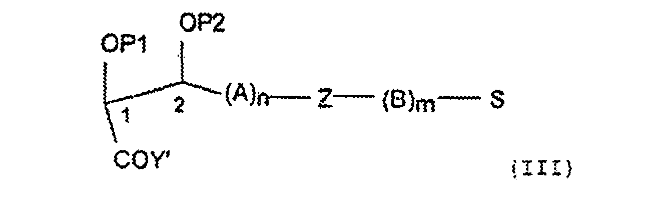 in der S, B, A, n, m, Z, P1 und P2 wie in einem der Ansprüchen 1 bis 9 definiert sind und Y' steht für eine Verbindung oder eine (Ketten)Verknüpfung von mehreren Verbindungen, ausgewählt aus der Gruppe, die besteht aus Lipiden, Nucleotiden, wobei die Nucleotide substituiert oder unsubstituiert sein können, und Aminosäuren, wobei letztere gegebenenfalls Lipide aufweisen können.

21. Träger der Formel (III) nach Anspruch 20, **dadurch gekennzeichnet, dass** er erhältlich ist aus einem Träger der Formel (I) nach einem der Ansprüche 1 bis 9 nach der chemischen Modifizierung der Gruppe -COX' mit Hilfe eines geeigneten Reagens, das eine Funktion zuführt, welche die Herstellung der Verbindung Y' auf dem Träger erlaubt.

22. Träger nach Anspruch 21, **dadurch gekennzeichnet, dass** das geeignete Reagens ein Diamin, ein Dialkohol oder ein Aminoalkohol ist.

23. Verfahren zur Synthese von organischen Verbindungen, die mindestens eine α-Oxoaldehyd-Funktion aufweisen, **dadurch gekennzeichnet, dass** das Verfahren in fester Phase durchgeführt wird und die folgenden Stufen umfasst:
- Herstellung eines Trägers der Formel (I), wie er in einem der Ansprüche 1 bis 9 definiert ist, wobei die genannte Herstellung nach einem der Ansprüche 10 bis 19 durchgeführt wird;
- gegebenenfalls Derivatisierung der Gruppe X' des Trägers der Formel (I) um auf diese Weise eine Funktion einzuführen, welche die Herstellung der Verbindung X' auf dem Träger erlaubt, wie in Anspruch 20 oder 21 definiert;
- Herstellung der Verbindung Y' in fester Phase;
- Entfernung der Schutzgruppen von den beiden Hydroxyl-Funktionen in den Positionen 1, 2, die durch P1 und P2 nach der Formel (III), wie in Anspruch 20 angegeben, geschützt worden sind, wenn P1 und P2 von einem Wasserstoffatom verschieden sind;
- Period-Oxidation in fester Phase; und
- Isolierung des gebildeten Produkts, das durch mindestens eine α-Oxoaldehyd-Funktion funktionalisiert ist.

24. Verfahren nach Anspruch 23, **dadurch gekennzeichnet, dass** die Derivatisierung der Gruppe X' des Trägers der Formel (I) darin besteht, dass man ein Diamin, einen Dialkohol oder einen Aminoalkohol aufpfropft.

25. Verfahren nach Anspruch 23 oder 24, **dadurch gekennzeichnet, dass** die Entfernung der Schutzgruppe von den beiden Hydroxyl-Funktionen in den Positionen 1, 2 in einem sauren bzw. Säuremedium, vorzugsweise in Gegenwart von Trifluoressigsäure, durchgeführt wird.

26. Verfahren nach Anspruch 23 oder 24, **dadurch gekennzeichnet, dass** die Entfernung der Schutzgruppen von den beiden Hydroxyl-Funktionen in den Positionen 1, 2 in einem basischen Medium durchgeführt wird.

27. Verfahren nach einem der Ansprüche 23 bis 26, **dadurch gekennzeichnet, dass** die Period-Oxidation durchgeführt wird in Gegenwart von Natriumperiodat.

28. Verfahren zur Synthese einer Bank (Gruppe) von organischen Verbindungen, **dadurch gekennzeichnet, dass** sie umfasst:
- die Bereitstellung einer Vielzahl von Oberflächen eines festen Trägers der Formel S-(B)ₘ-Y, worin B, S, Y und m wie in einem der Ansprüche 10 bis 19 definiert sind;
- ihre Reaktion mit einer Verbindung, ausgewählt aus der Gruppe, die besteht aus cyclischen Anhydriden der Weinsäure oder einem ihrer Derivate und Verbindungen der Formel (II) 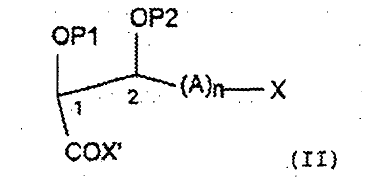 in der n, A, P1, P2, X' und X wie in einem der Ansprüche 10 bis 19 definiert sind, sodass man einen Träger erhält, der verwendbar ist für die Synthese von Verbindungen, die eine α-Oxoaldehyd-Funktion aufweisen;
- Umsetzung des erhaltenen Trägers mit Mischungen von Aminosäuren, Nucleotiden oder Lipiden; und
- Bildung auf diese Weise einer Bank (Gruppe) von organischen Verbindungen.

29. Bank (Gruppe) von organischen Verbindungen; **dadurch gekennzeichnet, dass** sie erhältlich ist nach dem Verfahren, wie es in Anspruch 28 beschrieben ist.

30. Verwendung einer Verbindung, ausgewählt aus der Gruppe, die besteht aus cyclischen Anhydriden der Weinsäure oder einem ihrer Derivate und Verbindungen der Formel (II) 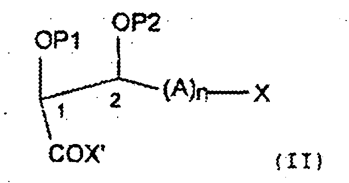 in der n, A, P1, P2 und X' und X wie in einem der Ansprüche 10 bis 19 definiert sind, für die Derivatisierung eines festen Trägers, von dem mindestens die Oberfläche die Eigenschaft hat, in einem wässrigen oder partiell wässrigen Medium gut solvatisiert zu sein, wobei der resultierende Träger für die Synthese von Verbindungen, die mindestens eine α-Oxoaldehyd-Funktion aufweisen, in fester Phase verwendet werden kann.

31. Verwendung nach Anspruch 30, **dadurch gekennzeichnet, dass** der genannte feste Träger, von dem mindestens die Oberfläche die Eigenschaft hat, in einem wässrigen oder partiell wässrigen Medium solvatisiert zu sein, der Formel S-(B)ₘ-Y entspricht, worin S, B, m und Y wie in einem der Ansprüche 10 bis 19 definiert sind.

32. Verwendung nach Anspruch 30 oder 31, **dadurch gekennzeichnet, dass** das genannte cyclische Anhydrid der Weinsäure oder eines ihrer Derivate das Di-O-benzyol-weinsäureanhydrid oder das Diacetyl-weinsäureanhydrid ist.

33. Verwendung nach Anspruch 30 oder 31, **dadurch gekennzeichnet, dass** die Verbindung der Formel (II) die Weinsäure oder ein Derivat der Weinsäure darstellt.

34. Verwendung nach Anspruch 30 oder 31, **dadurch gekennzeichnet, dass** die Verbindung der Formel (II) ausgewählt ist aus der Gruppe, die besteht aus Mucinsäure, Zuckersäure, Glucarsäure, ihren Salzen und Derivaten.

35. Verfahren zur Herstellung eines diagnostischen Reagens auf einem festen Träger, **dadurch gekennzeichnet, dass** es die folgenden Stufen umfasst:
- Herstellung eines Trägers der Formel (I), wie sie in einem der Ansprüche 1 bis 9 definiert ist, wobei die genannte Herstellung nach einem der Ansprüche 10 bis 19 durchgeführt wird;
- gegebenenfalls Derivatisierung der Gruppe X' des genannten Trägers der Formel (I), um auf diese Weise eine Funktion einzuführen, welche die Herstellung der Verbindung Y', wie in Anspruch 20 oder 21 definiert, auf dem Träger erlaubt;
- Herstellung der Verbindung Y' in fester Phase;
- Entfernung der Schutzgruppen von den beiden Hydroxyl-Funktionen in den Positionen 1, 2, die durch P1 und P2 nach der Formel (III), wie in Anspruch 20 angegeben, geschützt worden sind, wenn P1 und P2 von einem Wasserstoffatom verschieden sind;
- Period-Oxidation in fester Phase;
- Ligieren des gebildeten Produkts, das durch mindestens eine α-Oxoaldehyd-Funktion funktionalisiert ist;
- Verbinden des gebildeten Produkts, das mindestens eine α-Oxoaldehyd-Funktion aufweist, mit einer in geeigneter Weise funktionalisierten Mikrotitrier-Platte, und
- Bildung einer Mikrotitrier-Platte, auf der auf kovalente Weise die genannten Produkte fixiert sind.

36. Verfahren nach Anspruch 35, **dadurch gekennzeichnet, dass** die genannten Produkte Peptide, Oligonucleotide oder Lipide sind.

37. Verfahren nach Anspruch 35 oder 36, **dadurch gekennzeichnet, dass** das Ligieren der Produkte durchgeführt wird durch eine Oxim-, Oxazolidin- oder Hydrazon-Bindung.

38. Verfahren zur Herstellung eines Biochips, **dadurch gekennzeichnet, dass** es die folgenden Stufen umfasst:
- Herstellung eines Trägers der Formel (I), wie er in einem der Ansprüche 1 bis 9 definiert ist, wobei die genannte Herstellung nach einem der Ansprüche 10 bis 19 durchgeführt wird;
- gegebenenfalls Derivatisierung der Gruppe X' des genannten Trägers der Formel (I), um auf diese Weise eine Funktion einzuführen, welche die Herstellung der Verbindung Y', eines Nucleotids oder einer (Ketten)Verknüpfung von Nucleotiden, wie in Anspruch 20 oder 21 definiert, erlaubt;
- Herstellung eines Oligonucleotids in fester Phase;
- Entfernung der Schutzgruppen von den beiden Hydroxyl-Gruppen in den Positionen 1, 2, die durch P1 und P2 nach Formel (III), wie in Anspruch 20 angegeben, geschützt worden sind, wenn P1 und P2 von einem Wasserstoffatom verschieden sind;
- Period-Oxidation in fester Phase;
- Isolierung der gebildeten Oligonucleotide, die durch mindestens eine α-Oxoaldehyd-Funktion funktionalisiert sind; und
- Aufpfropfung der genannten Oligonucleotide auf einen festen Träger.

39. Verfahren nach Anspruch 38, **dadurch gekennzeichnet, dass** der feste Träger eine Oberfläche aus Glas oder aus Silicium ist.

40. Verfahren nach Anspruch 38 oder 39, **dadurch gekennzeichnet, dass** der genannte Biochip ein DNA-Chip ist.
